(19) Europäisches Patentamt
European Patent Office
Office européen des brevets

(11) Publication number: **0 226 304 B1**

(12) **EUROPEAN PATENT SPECIFICATION**

(45) Date of publication of patent specification: **28.08.91**

(51) Int. Cl.5: **A61K 31/40**, A61K 31/43, //(A61K31/40,31:195), (A61K31/43,31:195)

(21) Application number: **86308321.8**

(22) Date of filing: **24.10.86**

(54) **Composition containing a penem or carbapenem antibiotic.**

(30) Priority: **24.10.85 JP 238283/85**
**10.04.86 JP 81218/86**

(43) Date of publication of application:
**24.06.87 Bulletin 87/26**

(45) Publication of the grant of the patent:
**28.08.91 Bulletin 91/35**

(84) Designated Contracting States:
**AT BE CH DE ES FR GB IT LI LU NL SE**

(56) References cited:
**EP-A- 0 048 301**
**EP-A- 0 091 594**
**EP-A- 0 178 911**

(73) Proprietor: **SANKYO COMPANY LIMITED**
**No. 1-6, 3-chome Nihonbashi Honcho Chuo-ku**
**Tokyo(JP)**

(72) Inventor: **Shiokari, Takashi c/o Product Development Labs.**
**Sankyo Co. Ltd. 2-58, 1-chome**
**Hiromachi Shinagawa-ku Tokyo(JP)**
Inventor: **Ueda, Seigo c/o Product Development Labs.**
**Sankyo Co. Ltd. 2-58, 1-chome**
**Hiromachi Shinagawa-ku Tokyo(JP)**
Inventor: **Iwata, Masayuki c/o Product Development Labs.**
**Sankyo Co. Ltd. 2-58, 1-chome**
**Hiromachi Shinagawa-ku Tokyo(JP)**
Inventor: **Kawahara, Yukinori c/o Product Development Labs.**
**Sankyo Co. Ltd. 2-58, 1-chome**
**Hiromachi Shinagawa-ku Tokyo(JP)**

(74) Representative: **Gibson, Christian John Robert et al**
**MARKS & CLERK 57/60 Lincoln's Inn Fields**
**London WC2A 3LS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to a novel composition comprising a penem or carbapenem antibiotic in association with an amino acid derivative.

The class of compounds known as "penem and carbapenem antibiotics" is, of course, very well known and is potentially of great value for the treatment of bacterial infections. Although, as a group, these penem and carbapenem antibiotics exhibit excellent anti-bacterial activity and a variety of other properties which render them highly suitable for pharmaceutical use, they do have a number of disadvantages. One of the problems of these antibiotics is that, in general, they exhibit a degree of renal toxicity, and some degree of kidney damage is a frequent side effect of their use; accordingly, such penem and carbapenem antibiotics should not be used for the treatment of patients with actual or suspected impaired renal function. As a result, the penem and carbapenem antibiotics cannot be used for many patients for whom otherwise they would be the antibiotic of choice. The problem of renal toxicity is particularly acute when the antibiotics are administered by intravenous or intramuscular injection in a high dose.

We have now surprisingly discovered that the concurrent, or effectively concurrent, administration, with the penem or carbapenem antibiotic, of one or more of a certain class of acylated amino acid derivatives significantly reduces this renal toxicity.

EP-A-0 00 7614 discloses the use of a dipeptidase inhibitor in associated with antibiotics similar to those to which the present invention relates. However, the dipeptidase inhibitors employed are structurally different from the amino acid derivatives of the present invention and are employed for a totally different purpose. The amino acid derivatives employed in the present invention possess little or no dipeptidase inhibitory activity.

Accordingly, in one aspect, the present invention provides a composition comprising:

(a) a penem or carbapenem antibiotic; and

(b) a pharmaceutically acceptable N-acylated derivative of an amino acid wherein the amino group and the carboxylic acid group are attached to a saturated aliphatic carbon chain or carbon atom, or a salt thereof, provided that, where the composition comprises only one amino acid derivative, the amino acid is not ornithine, lysine, phenylalanine or phenylglycine.

EP-A-0 178 911 discloses such a composition where the amino acid is ornithine, lysine, phenylalanine or phenylglycine.

There is no particular limitation on the nature of the penem or carbapenem antibiotic to which the present invention can be applied and it is believed that the beneficial effects of the concurrent administration of the N-acylated amino acid derivative will be achieved regardless of the particular antibiotic chosen. However, the penem and carbapenem antibiotics which are currently of most actual or potential interest may be represented by the general formula I:

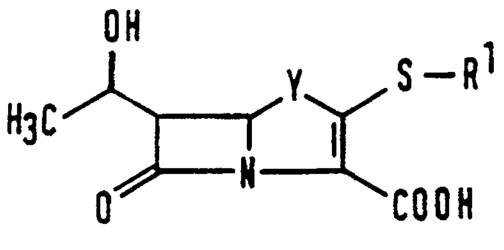

(I)

in which:

Y represents a sulphur atom, a methylene group or a methylene group having 1 or 2 methyl and/or methoxy substituents; and

$R^1$ represents a $C_1$-$C_6$ alkyl group, a $C_1$-$C_6$ alkyl group having at least one of substituents (i) or a heterocyclic group having from 4 to 14 ring atoms of which from 1 to 5 are nitrogen and/or oxygen and/or sulphur hetero-atoms where said heterocyclic group is unsubstituted or has at least one of substituents (ii);

substituents (i):

halogen atoms, amino groups, amino groups having at least one of substituents (iii), $C_1$-$C_4$ alkylideneamino groups, $C_1$-$C_4$ aminoalkylideneamino groups, amidino groups, amidino groups having from 1 to 3 of substituents (iii), heterocyclic groups having from 4 to 14 ring atoms of which from 1 to 5 are nitrogen and/or oxygen and/or sulphur hetero-atoms wherein said heterocyclic group is unsubstituted

or has at least one of substituents (ii), imino groups, cyano groups, carbamoyl groups and carbamoyl groups having at least one $C_1$-$C_4$ alkyl and/or $C_1$-$C_4$ alkoxy substituent;

substituents (ii):

$C_1$-$C_6$ alkanimidoyl groups, $C_1$-$C_6$ alkyl groups, alkoxyalkyl groups where the alkoxy and alkyl parts are each $C_1$-$C_4$, carbamoyl groups, carbamoyl groups having at least one $C_1$-$C_4$ alkyl and/or $C_1$-$C_4$ alkoxy substituent, $C_1$-$C_4$ haloalkyl groups, heterocyclic acylimidoyl groups where the heterocyclic part has from 5 to 9 ring atoms of which from 1 to 3 are nitrogen and/or oxygen and/or sulphur hetero-atoms, amidino groups, amidino groups having from 1 to 3 of substituents (iii), imino groups, oxygen atoms, $C_1$-$C_6$ alkanoyl groups, $C_1$-$C_6$ alkanesulphonyl groups, $C_1$-$C_6$ alkanesulphinyl groups, hydroximino groups, $C_1$-$C_4$ alkoximino groups, carbamoyloxy groups, carbamoyloxy groups having at least one $C_1$-$C_4$ alkyl and/or $C_1$-$C_4$ alkoxy substituent, carbamoyloxyalkyl groups where the alkyl part is $C_1$-$C_4$ and the carbamoyl part is unsubstituted or has at least one $C_1$-$C_4$ alkyl and/or $C_1$-$C_4$ alkoxy substituent and $C_1$-$C_4$ iminoalkyl groups;

substituents (iii):

$C_1$-$C_6$ alkyl groups, $C_2$-$C_6$ alkenyl groups, $C_2$-$C_6$ alkynyl groups, and said alkyl, alkenyl and alkynyl groups having at least one substituent selected from halogen atoms, carbamoyloxy groups and carbamoyloxy groups having at least one $C_1$-$C_4$ alkyl and/or $C_1$-$C_4$ alkoxy substituent.

Preferably Y represents a sulphur atom, a methylene group, or the group $CH_3$-CH<, $CH_3O$-CH< or $(CH_3)_2C$<.

Preferred examples of groups which may be represented by $R^1$ include the ethyl, 2-fluoroethyl, 2-(aminomethyleneamino(ethyl, $N^1$,$N^1$-dimethylamidinomethyl, $N^1$,$N^1$,$N^2$-trimethylamidinomethyl, 3-pyrrolidinyl, 1-formimidoyl-3-pyrrolidinyl, 1-acetimidoyl-3-pyrrolidinyl, 1-propionimidoyl-3-pyrrolidinyl, 2-methyl-1,4,5,6-tetrahydro-5-pyrimidinyl, 2-methoxymethyl-1,4,5,6-tetrahydro-5-pyrimidinyl, 3-azetidinyl, 1-acetimidoyl-3-azetidinyl, $N^1$-methyl-$N^1$-(2-propynyl)amidinomethyl, $N^1$-(2-fluoroethyl)-$N^1$-methylamidinomethyl, $N^1$-(3-fluoropropyl)-$N^1$-methylamidinomethyl, $N^1$-methyl-$N^1$-(2,2,2-trifluoroethyl)amidinomethyl, 1-(3-azetidinyl)ethyl, 1-(1-acetimidoyl-3-azetidinyl)ethyl, 1,4,5,6-tetrahydro-2-pyrimidinylmethyl, 1-(4,5-dihydro-2-thiazolyl)ethyl, 5-carbamoyl-3-pyrrolidinyl, 1-acetimidoyl-5-carbamoyl-3-pyrrolidinyl,2-chloromethyl-1,4,5,6-tetrahydro-5-pyrimidinyl, 1-butyrimidoyl-3-pyrrolidinyl, 1-nicotinimidoyl-3-pyrrolidinyl, $N^1$,$N^1$-diallylamidinomethyl, $N^1$-methyl-$N^1$-(2-propynyl)amidino, $N^1$-(2-fluoroethyl)-$N^1$-methylamidino, $N^1$-(3-fluoropropyl)-$N^1$-methylamidino, $N^1$-methyl-$N^1$-(2,2,2-trifluoroethyl)amidino, $N^1$-allyl-$N^1$-methylamidinomethyl, cyanomethyl, 2-cyanoethyl, 1-cyanoethyl, 2-cyano-1-methylethyl, 2-aminoethyl, 1-carbamoylethyl, 2-(1-aminoethylideneamino)ethyl, 1-amidino-3-pyrrolidinyl, 2-methyl-1,3-diazabicyclo[3.3.0]oct-2-en-7-yl, 2-methoxymethyl-1,3-diazabicyclo-[3.3.0]oct-2-en-7-yl, 5-imino-2-pyrrolidinyl, 2-imino-5-piperidinyl, 1-acetimidoyl-5-methylcarbamoyl-3-pyrrolidinyl, 1-acetimidoyl-5-methoxycarbamoyl-3-pyrrolidinyl, 2-imino-2-(S-oxothiomorpholino)ethyl, 2-imino-2-(1,1-dioxo-1,3-thiazolidin-3-yl)ethyl, 2-imino-2-(S,S-dioxothiomorpholino)ethyl, 2-imino-2-(3,5-dioxo-1-piperazinyl)ethyl, 2-imino-2-(4-methyl-3,5-dioxo-1-piperazinyl)ethyl, 2-imino-2-(3-oxo-1-piperazinyl)ethyl, 2-imino-2-(4-methyl-3-oxo-1-piperazinyl)ethyl, 2-imino-2-(4-acetyl-3-oxo-1-piperazinyl)ethyl, 2-imino-2-(4-methanesulphonyl-3-oxo-1-piperazinyl)ethyl, $N^1$-(2-carbamoyloxyethyl)-$N^1$-methylamidinomethyl, 2-(3-hydroximino-1-pyrrolidinyl)-2-iminoethyl, 2-imino-2-(3-methoximino-1-pyrrolidinyl)ethyl, 2-(4-hydroximinopiperidino)-2-iminoethyl, 2-imino-2-(4-methoximinopiperidino)ethyl, 2-(3-carbamoyloxy-1-pyrrolidinyl)-2-iminoethyl, 2-imino-2-(3-oxo-1-piperazinyl)ethyl, 2-(3-carbamoylpiperidino)-2-iminoethyl, 2-(3-carbamoyloxypiperidino)-2-iminoethyl, 2-(2-carbamoyloxy-1-pyrrolidinyl)-2-iminoethyl, 2-(2-carbamoyloxymethyl-1-pyrrolidinyl)-2-iminoethyl, 2-(4-carbamoyloxypiperidino)-2-iminoethyl, 2-(4-formyl-1-piperazinyl)-2-iminoethyl, 2-(4-acetyl-1-piperazinyl)-2-iminoethyl, 1-formyl-3-azetidinyl, 1-iminomethyl-3-azetidinyl, 1-methyl-4-piperidyl, 1-acetimidoyl-4-piperidyl and 1-acetyl-3-pyrrolidinyl groups.

The invention may also be applied to pharmaceutically acceptable salts and esters of such antibiotics, such as are well known in the art.

Specific examples of compounds of general formula I which may be employed in the present invention are those in which $R^1$ and Y are as defined below:

| Comp. No. | $R^1$ | Y |
|---|---|---|
| 1. | 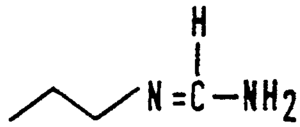 | $CH_2$ |
| 2. | 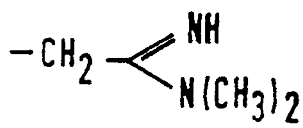 | $CH_2$ |
| 3. | 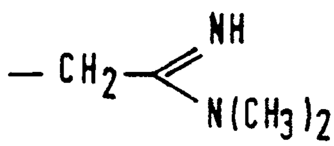 | 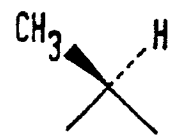 |
| 4. | 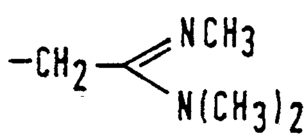 | $CH_2$ |
| 5. | 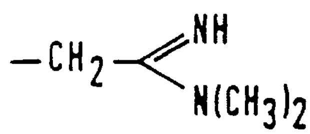 | S |

| Comp. No. | $R^1$ | Y |
|---|---|---|
| 6. | 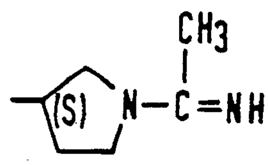 | $CH_2$ |
| 7. | 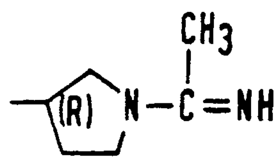 | $CH_2$ |
| 8. | 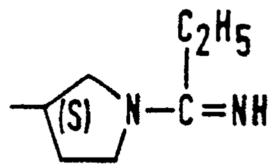 | $CH_2$ |
| 9 | 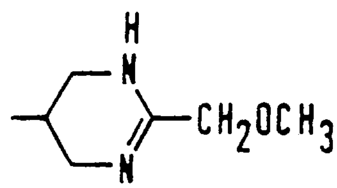 | $CH_2$ |
| 10. | 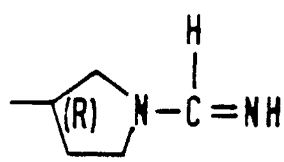 | $CH_2$ |
| 11. | 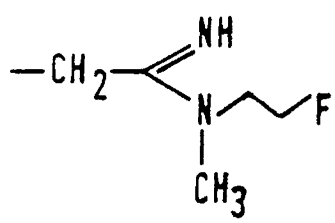 | $CH_2$ |

| Comp. No. | $R^1$ | Y |
|---|---|---|
| 12. | 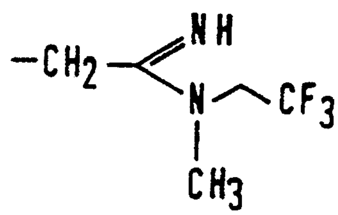 | $CH_2$ |
| 13. | 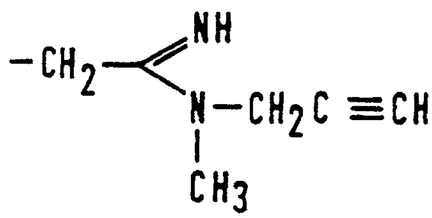 | $CH_2$ |
| 14. | 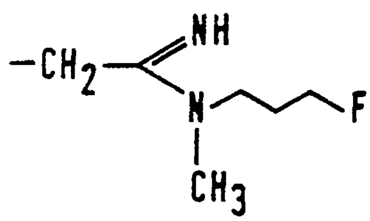 | $CH_2$ |
| 15. | 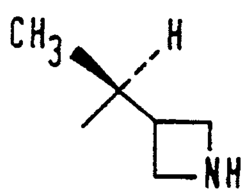 | $CH_2$ |
| 16. | 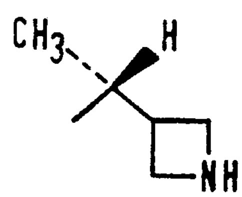 | $CH_2$ |

| Comp. No. | $R^1$ | Y |
| --- | --- | --- |
| 17. | 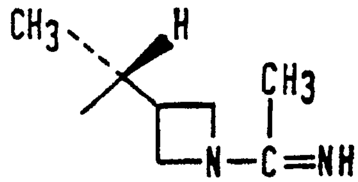 | $CH_2$ |
| 18. | 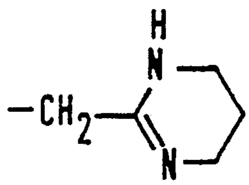 | $CH_2$ |
| 19. | 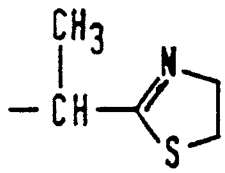 | $CH_2$ |
| 20. | 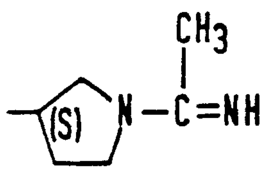 | 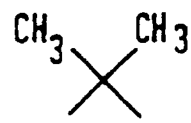 |
| 21. | 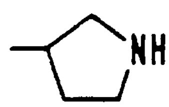 | 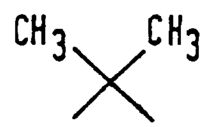 |
| 22. | 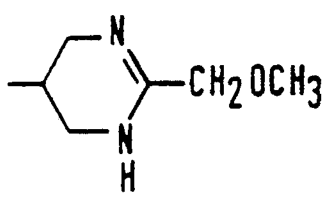 | 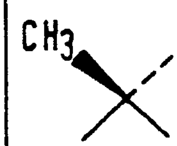 |

| Comp. No. | $R^1$ | Y |
|---|---|---|
| 23. | 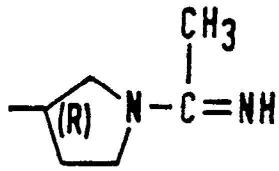 | 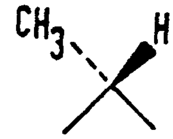 |
| 24. | 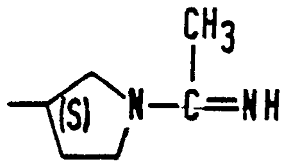 | 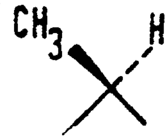 |
| 25. | 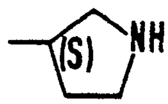 | 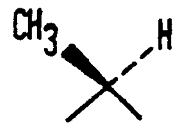 |
| 26. | 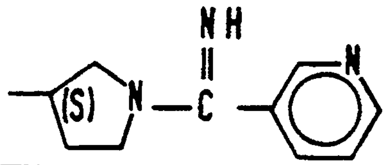 | $CH_2$ |
| 27. | 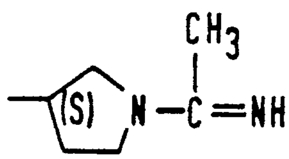 | 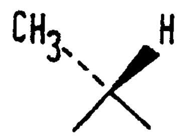 |
| 28. | 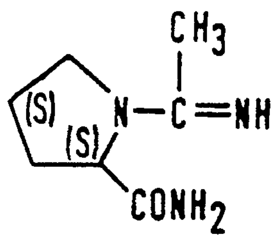 | $CH_2$ |

| Comp. No. | $R^1$ | Y |
|---|---|---|
| 29. | 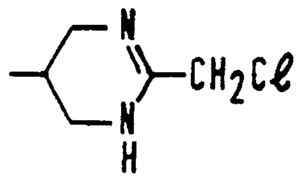 | $CH_2$ |
| 30. | 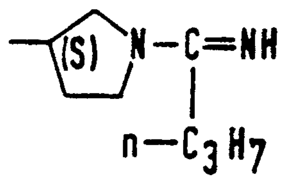 | $CH_2$ |
| 31. | 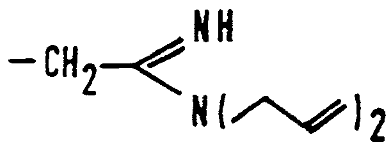 | $CH_2$ |
| 32. | 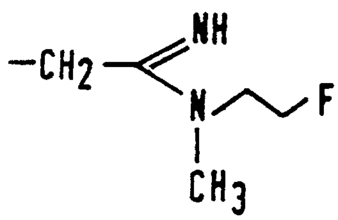 | 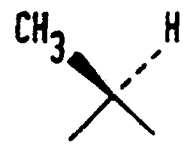 |
| 33. | 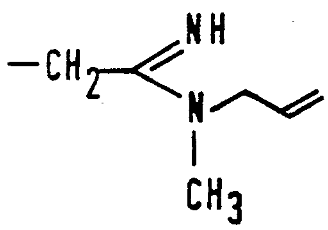 | 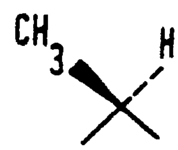 |
| 34. | 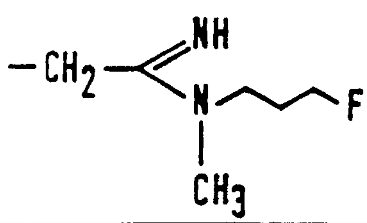 | 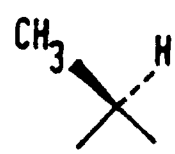 |

| Comp. No. | $R^1$ | Y |
|---|---|---|
| 35. | 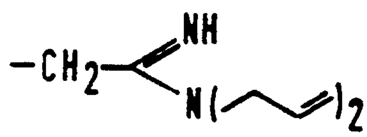 | 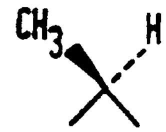 |
| 36. | $-CH_2CH_3$ | S |
| 37. | $-CH_2CH_2F$ | S |
| 38. | 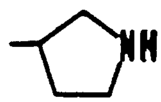 | S |
| 39. | $-CH_2CN$ | S |
| 40. | 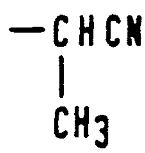 | S |
| 41. | 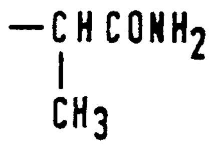 | S |
| 42. | $-CH_2CH_2NH_2$ | S |
| 43. | 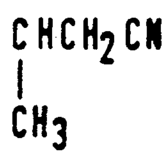 | $CH_2$ |

| Comp. No. | $R^1$ | Y |
|---|---|---|
| 44. | 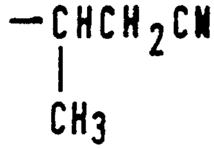 | S |
| 45. | $-CH_2CH_2CN$ | $CH_2$ |
| 46. | 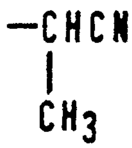 | $CH_2$ |
| 47. | 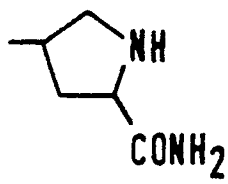 | $CH_2$ |
| 48. | 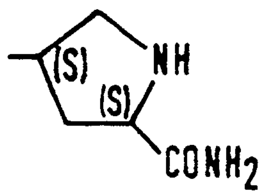 | 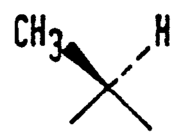 |
| 49. | 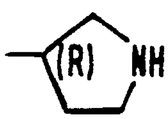 | 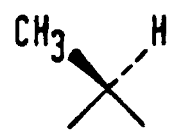 |
| 50. | 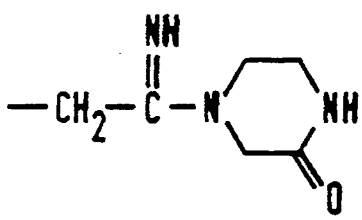 | 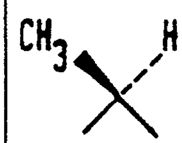 |

| Comp. No. | R1 | Y |
|---|---|---|
| 51. | 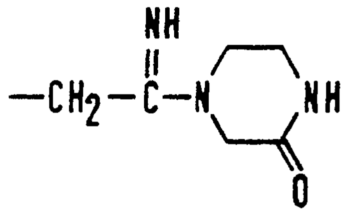 | $CH_2$ |
| 52. | 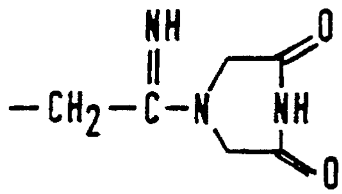 | $CH_2$ |
| 53. | 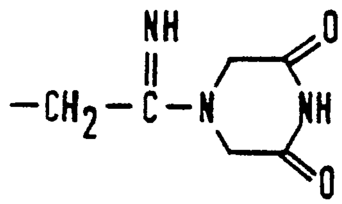 | 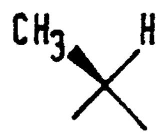 |
| 54. | 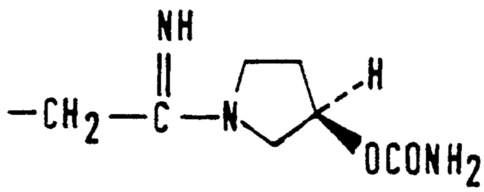 | $CH_2$ |
| 55. | 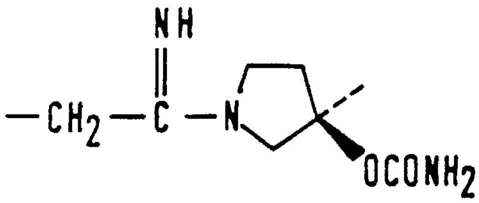 | 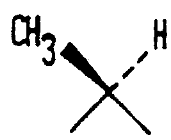 |
| 56. | 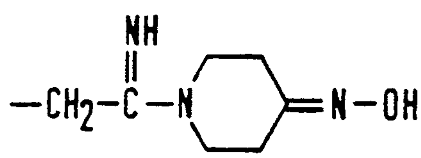 | $CH_2$ |

| Comp. No. | $R^1$ | Y |
|---|---|---|
| 57. | 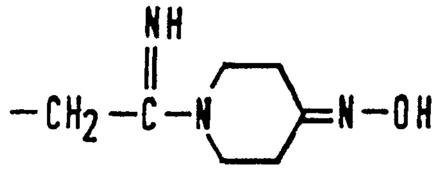 | 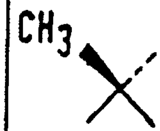 |
| 58. | 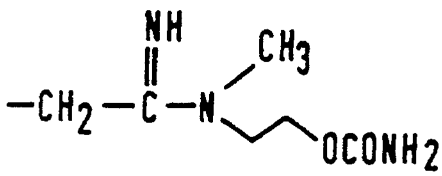 | $CH_2$ |
| 59. | 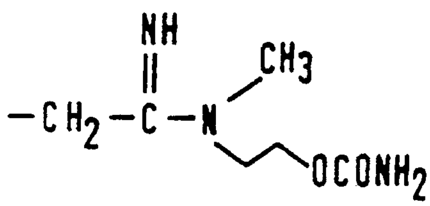 | 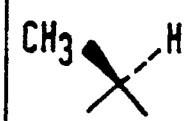 |
| 60. | 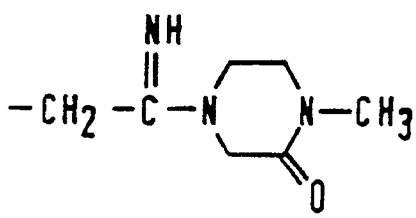 | $CH_2$ |
| 61. | 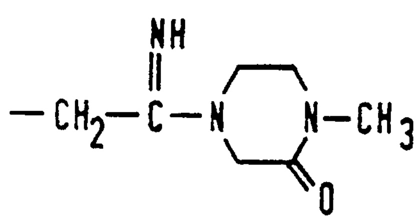 | 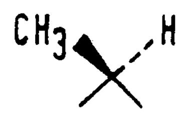 |
| 62. | 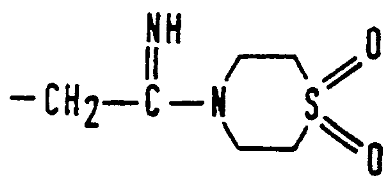 | $CH_2$ |

| Comp. No. | $R^1$ | Y |
|---|---|---|
| 63. | 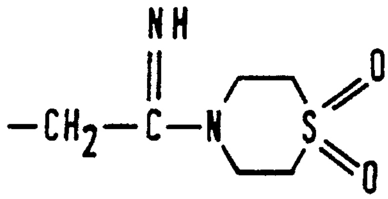 | 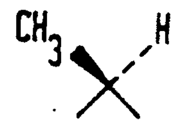 |
| 64. | 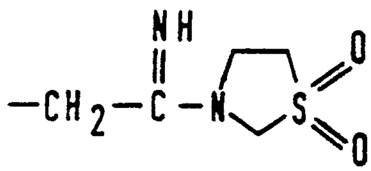 | $CH_2$ |
| 65. | 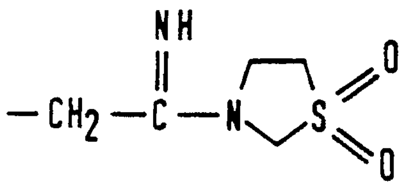 | 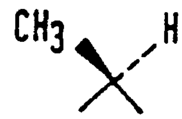 |
| 66. | 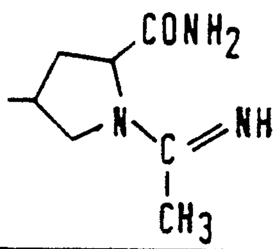 | $CH_2$ |
| 67. | 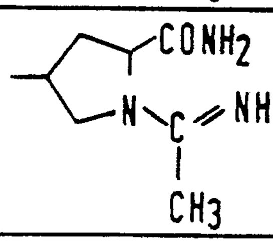 | 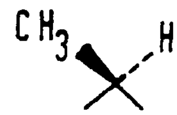 |
| 68. | 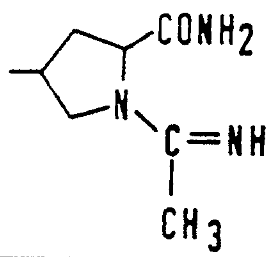 | 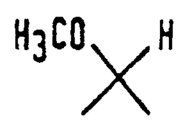 |

| Comp. No. | $R^1$ | Y |
|---|---|---|
| 69. | 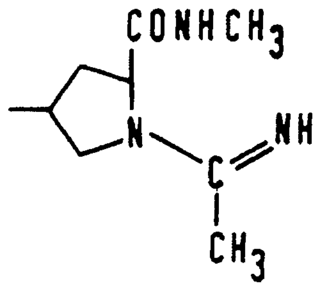 | $CH_2$ |
| 70. | 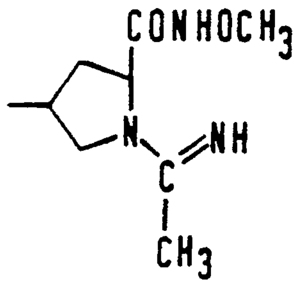 | $CH_2$ |
| 71. | 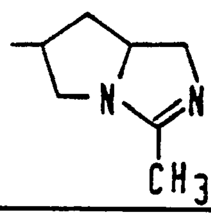 | $CH_2$ |
| 72. | 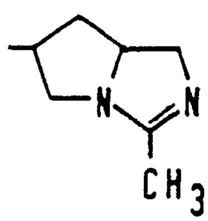 | 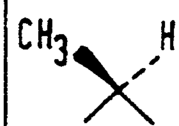 |
| 73. | 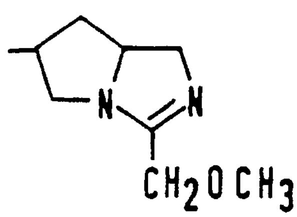 | $CH_2$ |

| Comp. No. | $R^1$ | Y |
|---|---|---|
| 74. | 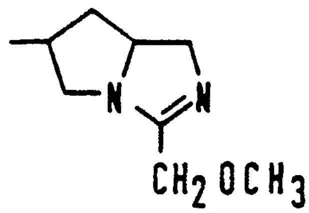 | 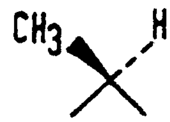 |
| 75 | 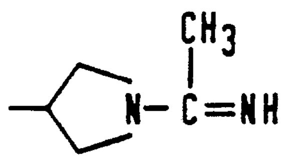 | $CH_2$ |
| 76. | 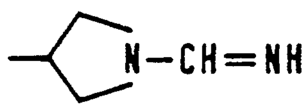 | $CH_2$ |
| 77. | 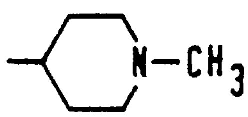 | $CH_2$ |
| 78. | 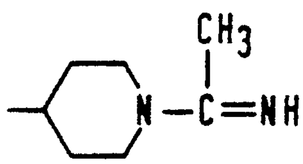 | $CH_2$ |
| 79. | 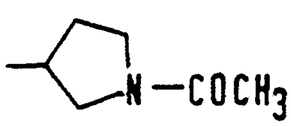 | $CH_2$ |

Of the compounds listed above, we particularly prefer those which have the same configuration as thienamycin, i.e. (5R,6S)-6-[1(R)-hydroxyethyl]. In particular, the following compounds are preferred:

(5R,6S)-2-{2-[(aminomethylene)amino]ethylthio}-6-[1(R)-hydroxyethyl]-2-carbapenem-3-carboxylic acid (isomer of Compound No. 1)

(5R,6S)-2-[(3S)-1-acetimidoylpyrrolidin-3-ylthio]-6-[1(R)-hydroxyethyl]-2-carbapenem-3-carboxylic acid (isomer of Compound No. 6)

(5R,6S)-2-[(3R)-1-acetimidoylpyrrolidin-3-ylthio]-6-[1(R)-hydroxyethyl]-2-carbapenem-3-carboxylic acid (isomer of Compound No. 7)

(5R,6S)-2-[(3R)-1-acetimidoylpyrrolidin-3-ylthio]-6-[1(R)-hydroxyethyl]-1(S)-methyl-2-carbapenem-3-carboxylic acid (isomer of Compound No. 23)

(5R,6S)-2-[(3S)-1-acetimidoylpyrrolidin-3-ylthio]-6-[1(R)-hydroxyethyl]-1(R)-methyl-2-carbapenem-3-carboxylic acid (isomer of Compound No. 24)

(5R,6S)-2-[(3S)-1-acetimidoylpyrrolidin-3-ylthio]-6-[1(R)-hydroxyethyl]-1(S)-methyl-2-carbapenem-3-carboxylic acid (isomer of Compound No. 27)
(5R,6S)-2-[(3S)-1-acetimidoyl-5(S)-carbamoylpyrrolidin-3-ylthio]-6-[1(R)-hydroxyethyl]-2-carbapenem-3-carboxylic acid (isomer of Compound No. 28)

The above compounds may likewise be employed in the form of their pharmaceutically acceptable salts or esters, examples of which are well-known to those skilled in the art and which are given, for example, in US Patent No. 4,552,873.

The protective effect against renal toxicity appears to be exhibited by the whole range of amino acids wherein the amino and carboxylic acid groups are attached to a saturated aliphatic carbon chain or carbon atom. However, we have found that best results are achieved when employing N-acylated derivatives of those amino acids which may be represented by the general formula II:

$H_2N$-X-COOH (II)

wherein X represents a $C_1$-$C_{10}$ alkylene group or a $C_1$-$C_{10}$ alkylene group having at least one substituent selected from hydroxy groups, $C_1$-$C_4$ alkoxy groups, $C_6$-$C_{14}$ aryloxy groups, substituted $C_6$-$C_{14}$ aryloxy groups, $C_7$-$C_9$ aralkyloxy groups, substituted $C_7$-$C_9$ aralkyloxy groups, mercapto groups, $C_1$-$C_4$ alkylthio groups, $C_6$-$C_{14}$ arylthio groups, substituted $C_6$-$C_{14}$ arylthio groups, $C_7$-$C_9$ aralkylthio groups, substituted $C_7$-$C_9$ aralkylthio groups, $C_2$-$C_5$ carboxyalkylthio groups, amino groups, amino groups having one or two substituents selected from
$C_1$-$C_4$ alkyl groups, $C_6$-$C_{14}$ aryl groups, substituted $C_6$-$C_{14}$ aryl groups, $C_7$-$C_9$ aralkyl groups, substituted $C_7$-$C_9$ aralkyl groups and carboxylic acyl groups,
$C_6$-$C_{14}$ aryl groups, substituted $C_6$-$C_{14}$ aryl groups, carboxy groups, amidino groups, sulpho groups, $C_1$-$C_4$ alkylsulphinyl groups, $C_1$-$C_4$ alkylsulphonyl groups and heterocyclic groups having from 5 to 14 ring atoms of which from 1 to 5 are nitrogen and/or oxygen and/or sulphur hetero-atoms, said substituted aryloxy, aralkyloxy, arylthio, aralkylthio, aryl and aralkyl groups having at least one substituent selected from $C_1$-$C_4$ alkyl groups, hydroxy groups, amino groups and $C_1$-$C_4$ alkoxy groups.

In general terms, the N-acylated derivatives of these amino acids may be represented by the general formula III:

$R^2$HN-X-COOH (III)

wherein $R^2$ represents a carboxylic acyl group and X is as defined above.

Examples of carboxylic acyl groups which may be represented by $R^2$ include:
alkanoyl groups, and preferably alkanoyl groups having from 1 to 18, more preferably from 1 to 10 and still more preferably from 1 to 8, e.g. from 2 to 5 or from 5 to 8, carbon atoms, for example the acetyl, propionyl, butyryl, isobutyryl, valeryl, isovaleryl, pivaloyl, hexanoyl, heptanoyl, octanoyl, nonanoyl and decanoyl groups; in the case of those amino acids which have relatively bulky and lipophilic groups, lower (e.g. $C_2$-$C_5$) alkanoyl groups are preferred; for others (e.g. glycine), higher (e.g. $C_5$-$C_8$) groups are preferred;
alkenoyl and alkynoyl groups, and more preferably such groups having from 3 to 8, more preferably 3 or 4, carbon atoms, for example the acryloyl, methacryloyl, crotonoyl or propioloyl groups;
aromatic acyl groups in which the aryl ring is a carbocyclic ring having from 6 to 14, preferably 6 to 10, carbon atoms and optionally having from 1 to 5, more preferably from 1 to 3, substituents preferably selected from $C_1$-$C_4$ alkyl groups, hydroxy groups, $C_1$-$C_4$ alkoxy groups, amino groups, sulpho groups and halogen atoms, for example the benzoyl and naphthoyl (1- or 2-naphthoyl) groups and the benzoyl and naphthoyl (1- or 2-naphthoyl) groups having one or more of the above substituents, for example the p-toluoyl, m-toluoyl, o-toluoyl, 4-butylbenzoyl, 4-hydroxybenzoyl, 3-hydroxybenzoyl, 2-hydroxybenzoyl, 4-methoxybenzoyl, 3-methoxybenzoyl, 2-methoxybenzoyl, 4-butoxybenzoyl, 4-aminobenzoyl, 3-aminobenzoyl, 2-aminobenzoyl, 3-sulphobenzoyl, 4-chlorobenzoyl, 3-fluorobenzoyl, 2-bromobenzoyl, 3-hydroxy-2-naphthoyl and 1-hydroxy-2-naphthoyl groups;
alicyclic acyl groups in which the carbocyclic ring has from 3 to 8 carbon atoms, more preferably from 3 to 6 carbon atoms, and in which the cycloalkane ring is unsubstituted or has at least one $C_1$-$C_4$ alkyl and/or phenyl substituent, for example the cyclopropanecarbonyl, cyclobutanecarbonyl, cyclopentanecarbonyl, cyclohexanecarbonyl, 1-phenyl-1-cyclopropanecarbonyl, 1-phenyl-1-cyclopentanecarbonyl, 1-methyl-1-cyclohexanecarbonyl and 1-phenyl-1-cyclohexanecarbonyl groups;
araliphatic acyl groups in which the aryl ring is a carbocyclic ring having from 6 to 14, preferably 6 to 10, carbon atoms and optionally having from 1 to 5, more preferably from 1 to 3, substituents preferably selected from $C_1$-$C_4$ alkyl groups, hydroxy groups, $C_1$-$C_4$alkoxy groups, amino groups, sulpho groups and

halogen atoms, and in which the alkyl moiety has from 1 to 4 carbon atoms, such as the phenylacetyl, α-phenyl-α-methylacetyl, α-phenyl-α-ethylacetyl, α,α-diphenylacetyl, α-phenyl-α-cyclopentyl-acetyl, 3-phenylpropionyl, 4-phenylbutyryl, 4-tolylacetyl, 4-hydroxyphenylacetyl, 4-aminophenylacetyl, 4-methoxyphenylacetyl, 3-sulphophenylacetyl and 4-chlorophenylacetyl groups;

heterocyclic acyl groups which may have saturated or unsaturated ring systems, the rings having 5 or 6 ring atoms, of which from 1 to 3 are nitrogen and/or sulphur and/or oxygen hetero-atoms and the ring being unsubstituted or having from 1 to 3 $C_1$-$C_4$ alkyl and/or hydroxy substituents, for example the nicotinoyl, 2-thiophenecarbonyl, 2-furoyl, 2-pyrazinecarbonyl, 2-piperidinecarbonyl, N-methylnicotinoyl and 6-hydroxynicotinoyl groups;

alkoxycarbonyl groups having a total of from 2 to 7 carbon atoms, for example the methoxycarbonyl, ethoxycarbonyl, propoxycarbonyl, isopropoxycarbonyl, butoxycarbonyl, t-butoxycarbonyl and pentyloxycarbonyl groups; and

aralkyloxycarbonyl groups in which the aralkyl moiety has from 7 to 9 carbon atoms and is unsubstituted or has from 1 to 5, more preferably from 1 to 3, substituents selected from amino groups, $C_1$-$C_4$ alkyl groups, $C_1$-$C_4$ alkoxy groups and hydroxy groups, for example the benzyloxycarbonyl, α-methylbenzyloxycarbonyl, phenethyloxycarbonyl, 3-phenylpropoxycarbonyl, 4-methoxybenzyloxycarbonyl, 4-hydroxybenzyloxycarbonyl, p-tolyloxycarbonyl and 4-aminobenzyloxycarbonyl groups.

In addition to the acyl groups listed above, $R^2$ can also represent an acyl group derived from an amino acid by removal of OH from the carboxylic acid group and N-acylation of the amino group with at least one of the above-mentioned acyl groups. Hence, $R^2$ can also represent such an acyl group connected to the parent amino acid via one or more amino acid residues, preferably from 0 to 5, more preferably from 0 to 3 and most preferably from 0 to 2, such residues. Thus, $R^2$ could represent a group derived from an N-acylated amino acid, for example the N-benzoylglycyl or N-benzoylglycylglycyl group. Hence, compounds of general formula I also include such oligopeptide compounds as N-benzoylglycylglycine, N-benzoylglycylglycylglycine and similar compounds.

Preferred examples of groups which may be represented by $R^2$ include: saturated aliphatic acyl groups having from 1 to 8 carbon atoms; aromatic acyl groups in which the aryl moiety has from 6 to 10 ring carbon atoms and is unsubstituted or has from 1 to 3 $C_1$-$C_4$ alkyl and/or $C_1$-$C_4$ alkoxy substituents; alicyclic acyl groups in which the cycloalkane ring has from 3 to 6 carbon atoms; araliphatic acyl groups in which the aryl ring has from 6 to 10 ring carbon atoms and the alkyl group has from 1 to 4 carbon atoms, the aryl ring being unsubstituted or having from 1 to 3 $C_1$-$C_4$ alkyl and/or $C_1$-$C_4$ alkoxy substituents; heterocyclic acyl groups in which the heterocyclic ring is saturated or unsaturated and has 5 or 6 ring atoms of which one is a nitrogen, sulphur or oxygen hetero-atom; alkoxycarbonyl groups having a total of from 2 to 7 carbon atoms; and aralkyloxycarbonyl groups in which the aralkyl moiety has from 7 to 9 carbon atoms and the aryl ring is unsubstituted or has from 1 to 3 $C_1$-$C_4$ alkyl and/or $C_1$-$C_4$ alkoxy substituents.

Particularly preferred groups which may be represented by $R^2$ include: aromatic acyl groups in which the aryl ring has from 6 to 10 ring atoms and which is unsubstituted or has a single substituent selected from $C_1$-$C_4$ alkyl groups, $C_1$-$C_4$ alkoxy groups, hydroxy groups and amino groups; alicyclic acyl groups in which the cycloalkane moiety has from 3 to 6 carbon atoms; phenylaliphatic acyl groups in which the phenyl group is unsubstituted or has a single $C_1$-$C_4$ alkyl substituent, and in which the alkyl part has from 1 to 4 carbon atoms; alkoxycarbonyl groups having a total of from 4 to 6 carbon atoms; and aralkyloxycarbonyl groups in which the aralkyl part has from 7 to 9 carbon atoms and has 0 or 1 $C_1$-$C_4$ alkyl or $C_1$-$C_4$ alkoxy substituent.

In addition, such acyl groups linked to the amino acid via at least one further amino acid residue are preferred.

Of the groups exemplified above, the following are most preferred: acetyl, benzoyl, cyclohexanecarbonyl, cyclopropanecarbonyl, hexanoyl, isobutyryl, crotonoyl, ethoxycarbonyl, 4-hydroxybenzoyl, anisoyl, 4-aminobenzoyl, naphthoyl, toluoyl, benzyloxycarbonyl and 4-methoxybenzyloxycarbonyl groups, of which the acetyl and benzoyl, particularly benzoyl, groups are most preferred. As explained previously, the lower alkanoyl groups, notably the acetyl group, are only most preferred in relation to their use with those amino acids which have relatively bulky and lipophilic groups.

In the compounds of general formula II, X represents an alkylene group having from 1 to 10, preferably from 1 to 8 and more preferably from 1 to 5, carbon atoms. Such groups may have the "free" valencies attached to different carbon atoms or to the same carbon atom. In the latter case, the groups are sometimes referred to as "alkylidene" groups. Examples include the methylene, ethylidene, ethylene, propylidene, 1-methylethylidene, 1-methylethylene, trimethylene, butylidene, 2-methylpropylidene, 1-methylpropylidene, 1,2-dimethylethylene, 1-ethylethylene, 1-methyltrimethylene, 2-methyltrimethylene, tetramethylene, pentylidene, 3-methylbutylidene, 2-methylbutylidene, 2,2-dimethylpropylidene, 1-ethylpropylidene, 1,2-dimethylpropylidene, 1-propylethylene, 1-(1-methylethyl)ethylene, 1-ethyl-2-methylethylene, 1-ethyltrimethylene, 2-ethyltrimethylene, 1,3-dimethyltrimethylene, 1-methyltetramethylene, 2-methyltetramethylene, pentamethylene, hexylidene, 4-methylpentylidene, 3-methylpentylidene, 2-methylpentylidene, 1-methylpentylidene, 2-ethylbutylidene, 1-ethylbutylidene, 1,3-dimethylbutylidene, 1,2-dimethylbutylidene, 3,3-dimethylbutylidene, 2,3-dimethylbutylidene, 1-butylethylene, 1-methyl-2-propylethylene, 1,2-diethylethylene, 1-methyl-1-propylethylene, 2-propyltrimethylene, 1-ethyl-3-methyltrimethylene, 1-ethyltetramethylene, 2-ethyltetramethylene, 1,3-dimethyltetramethylene, 1-methylpentamethylene, 2-methylpentamethylene, 3-methylpentamethylene, hexamethylene, heptylidene, 5-methylhexylidene, 4-methylhexylidene, 3-methylhexylidene, 1-methylhexylidene, 3-ethylpentylidene, 1-ethylpentylidene, 4,4-dimethylpentylidene, 2,4-dimethylpentylidene, 1,2-dimethylpentylidene, 1-propylbutylidene, 2-ethyl-1-methylbutylidene, 1-ethyl-2-methylbutylidene, 1,2,2-trimethylbutylidene, 1,2,3-trimethylbutylidene, 1-pentylethylene, 1-butyl-2-methylethylene, 1-ethyl-2-propylethylene, 1-butyl-1-methylethylene, 1-ethyl-1-propylethylene, 1-butyltrimethylene, 2-butyltrimethylene, 1,3-diethyltrimethylene, 1-methyl-3-propyltrimethylene, 1-propyltetramethylene, 2-propyltetramethylene, 1-ethyl-4-methyltetramethylene, 3-ethyl-1-methyltetramethylene, 1-ethylpentamethylene, 3-ethylpentamethylene, 1,3-dimethylpentamethylene, 1-methylhexamethylene, 3-methylhexamethylene, heptamethylene, octylidene, 6-methylheptylidene, 4-methylheptylidene, 2-methylheptylidene, 1-methylheptylidene, 4-ethylhexylidene, 3-ethylhexylidene, 2-ethylhexylidene, 1-ethylhexylidene, 3,5-dimethylhexylidene, 4,5-dimethylhexylidene, 2,4-dimethylhexylidene, 1,5-dimethylhexylidene, 1,4-dimethylhexylidene, 2-propylpentylidene, 1-propylpentylidene, 2-ethyl-4-methylpentylidene, 3-ethyl-2-methylpentylidene, 3-ethyl-1-methylpentylidene, 1-ethyl-3-methylpentylidene, 3-methyl-1-propylbutylidene, 2-methyl-1-propyl-butylidene, 1-ethyl-2,3-dimethylbutylidene, 1,2-diethylbutylidene, 1-hexylethylene, 1-methyl-2-pentylethylene, 1-butyl-2-ethylethylene, 1,2-dipropylethylene, 1-pentyltrimethylene, 2-pentyltrimethylene, 1-butyl-3-methyltrimethylene, 1-butyl-2-methyltrimethylene, 1-ethyl-3-propyl-trimethylene, 1,2-dimethyl-3-propyltrimethylene, 1-butyltetramethylene, 1-methyl-4-propyltetramethylene, 1-propylpentamethylene, 3-propylpentamethylene, 2-ethyl-4-methylpentamethylene, 1-ethylhexamethylene, 3-ethylhexamethylene, 1,3-dimethylhexamethylene, 1-methylheptamethylene, 4-methylheptamethylene and octamethylene groups.

The alkylene group represented by X, including those alkylene groups exemplified above, may be unsubstituted or may have at least 1, preferably from 1 to 4 and more preferably 1 or 2, substituents selected from the following groups:

hydroxy groups;

$C_1$-$C_4$ alkoxy groups, for example the methoxy or ethoxy groups;

aryloxy groups in which the aryl ring has from 6 to 14, more preferably from 6 to 10, ring carbon atoms and which is unsubstituted or has from 1 to 5, more preferably from 1 to 3, substituents selected from $C_1$-$C_4$ alkyl groups, hydroxy groups, amino groups and $C_1$-$C_4$ alkoxy groups, for example the phenoxy, p-tolyloxy, 4-hydroxyphenoxy, 4-aminophenoxy and 4-methoxyphenoxy groups;

$C_7$-$C_9$ aralkyloxy groups in which the aryl ring is unsubstituted or has from 1 to 5, more preferably from 1 to 3, substituents selected from $C_1$-$C_4$ alkyl groups, hydroxy groups, amino groups and $C_1$-$C_4$ alkoxy groups, for example the benzyloxy, 4-methylbenzyloxy, 4-hydroxybenzyloxy, 4-aminobenzyloxy and 4-methoxybenzyloxy groups;

mercapto groups;

$C_1$-$C_4$ alkylthio groups, for example the methylthio or ethylthio groups;

arylthio groups in which the aryl ring has from 6 to 14, more preferably from 6 to 10, ring carbon atoms and which is unsubstituted or has from 1 to 5, more preferably from 1 to 3, substituents selected from $C_1$-$C_4$ alkyl groups, hydroxy groups, amino groups and $C_1$-$C_4$ alkoxy groups, for example the phenylthio, p-tolylthio, 4-hydroxyphenylthio, 4-aminophenylthio and 4-methoxyphenylthio groups;

$C_7$-$C_9$ aralkylthio groups in which the aryl ring is unsubstituted or has from 1 to 5, more preferably from 1 to 3, substituents selected from $C_1$-$C_4$ alkyl groups, hydroxy groups, amino groups and $C_1$-$C_4$ alkoxy groups, for example the benzylthio, 4-methylbenzylthio, 4-hydroxybenzylthio, 4-aminobenzylthio and 4-methoxybenzylthio groups;

carboxyalkylthio groups having from 1 to 4 carbon atoms in the alkyl moiety, for example the carboxymethylthio and carboxyethylthio groups;

amino groups;

amino groups having one or two $C_1$-$C_4$ alkyl substituents, for example the methylamino, ethylamino and dimethylamino groups;

amino groups having one or two aryl substituents, wherein the aryl ring has from 6 to 14 ring carbon atoms and is unsubstituted or has from 1 to 5, preferably from 1 to 3, substituents selected from $C_1$-$C_4$ alkyl groups, hydroxy groups, amino groups, and $C_1$-$C_4$alkoxy groups, such as the phenylamino, p-tolylamino, 4-hydroxyphenylamino, 4-aminophenylamino and 4-methoxyphenylamino groups;

amino groups having one or two $C_7$-$C_9$ aralkyl substituents wherein the aryl moiety is unsubstituted or has from 1 to 5, preferably from 1 to 3, substituents selected from $C_1$-$C_4$ alkyl groups, hydroxy groups, amino groups and $C_1$-$C_4$ alkyl groups, such as the benzylamino, 4-methylbenzylamino, 4-hydroxybenzylamino, 4-aminobenzylamino and 4-methoxybenzylamino groups;
amino groups substituted by one or two carboxylic acyl groups as defined in relation to $R^2$;
aryl groups having from 6 to 14 ring carbon atoms, and being unsubstituted or having from 1 to 5, preferably from 1 to 3, substituents selected from $C_1$-$C_4$ alkyl groups, hydroxy groups, amino groups and $C_1$-$C_4$ alkoxy groups;
carboxy groups;
amidino groups;
sulpho groups;
$C_1$-$C_4$ alkylsulphinyl groups, such as the methanesulphinyl or ethanesulphinyl groups;
$C_1$-$C_4$ alkylsulphonyl groups, such as the methanesulphonyl or ethanesulphonyl groups; and
heterocyclic groups, such as the pyrrolyl, imidazolyl, pyrazolyl, pyridyl, pyrazinyl, pyrimidinyl, pyridazinyl, indolazinyl, indolyl and indazolyl groups.

Preferred groups which may be represented by X include $C_1$-$C_5$ alkylene groups which are unsubstituted or have one or two substituents selected from: hydroxy groups; $C_1$-$C_4$ alkoxy groups; aryloxy groups wherein the aryl ring has from 6 to 14 ring carbon atoms and which is unsubstituted or has from 1 to 3 substituents selected from $C_1$-$C_4$ alkyl groups, hydroxy groups, amino groups and $C_1$-$C_4$ alkoxy groups; $C_7$-$C_9$ aralkyloxy groups, wherein the aryl moiety is unsubstituted or has from 1 to 3 substituents selected from $C_1$-$C_4$ alkyl groups, hydroxy groups, amino groups and $C_1$-$C_4$ alkoxy groups; mercapto groups; $C_1$-$C_4$ alkylthio groups; arylthio groups wherein the aryl ring has from 6 to 14 ring carbon atoms and which is unsubstituted or has from 1 to 3 substituents selected from $C_1$-$C_4$ alkyl groups, hydroxy groups, amino groups and $C_1$-$C_4$ alkoxy groups; $C_7$-$C_9$ aralkylthio groups wherein the aryl ring is unsubstituted or has from 1 to 3 substituents selected from $C_1$-$C_4$ alkyl groups, hydroxy groups, amino groups and $C_1$-$C_4$ alkoxy groups; carboxyalkylthio groups in which the alkyl part has from 1 to 4 carbon atoms; amino groups; amino groups having one or two $C_1$-$C_4$ alkyl substituents; amino groups having one or two aryl substituents in which the aryl ring has from 6 to 14 ring carbon atoms and is unsubstituted or has from 1 to 3 substituents selected from $C_1$-$C_4$ alkyl groups, hydroxy groups, amino groups and $C_1$-$C_4$ alkoxy groups; amino groups having one or two $C_7$-$C_9$ aralkyl substituents in which the aryl part is unsubstituted or has from 1 to 3 substituents selected from $C_1$-$C_4$ alkyl groups, hydroxy groups, amino groups and $C_1$-$C_4$ alkoxy groups; amino groups having one or two carboxylic acyl substituents as defined in relation to $R^2$; aryl groups having from 6 to 14 ring carbon atoms and being unsubstituted or having from 1 to 3 substituents selected from $C_1$-$C_4$ alkyl groups, hydroxy groups, amino groups and $C_1$-$C_4$ alkoxy groups; carboxy groups; and heterocyclic groups having from 5 to 9 ring atoms, of which from 1 to 3 are nitrogen and/or oxygen and/or sulphur hetero-atoms.

More preferred groups which may be represented by X are $C_1$-$C_5$ alkylene groups which are unsubstituted or have 1 or 2 substituents selected from: hydroxy groups; $C_1$-$C_4$ alkoxy groups; mercapto groups; $C_1$-$C_4$ alkylthio groups; amino groups; amino groups having one or two $C_1$-$C_4$ alkyl substituents; amino groups having one or two carboxylic acyl substituents as defined for $R^2$; aryl groups having from 6 to 14 carbon atoms wherein the aryl ring is unsubstituted or has from 1 to 3 substituents selected from $C_1$-$C_4$ alkyl groups, hydroxy groups, amino groups and $C_1$-$C_4$ alkoxy groups; carboxy groups; and heterocyclic groups having from 5 to 9 ring atoms, of which from 1 to 3 are nitrogen and/or oxygen hetero-atoms.

Preferred amino acids which may be represented by general formula II include glycine, β-alanine, 4-aminobutyric acid, 5-aminovaleric acid, 6-aminohexanoic acid, 8-aminooctanoic acid, alanine, 2-aminobutyric acid, norvaline, valine, leucine, isoleucine, norleucine, tyrosine, O-methyltyrosine, aspartic acid, glutamic acid, 4-carboxyglutamic acid, 3-methylaspartic acid, 2-aminoadipic acid, 2-aminopimelic acid, 2-aminosuberic acid, 3-hydroxyaspartic acid, 3-hydroxyglutamic acid, 2,3-diaminopropionic acid, 2,4-diaminobutyric acid, 5-hydroxylysine, arginine, $N^δ$,$N^δ$-dimethylornithine, $N^ε$-methyllysine, cysteine, methionine, ethionine, S-carboxymethylcysteine, S-benzylcysteine, methionine S-oxide, ethionine S-oxide, methionine S,S-dioxide, cysteic acid, serine, O-methylserine, threonine, O-methylthreonine, homothreonine, ethoxinine ( = 2-amino-4-ethoxybutyric acid), 3-methoxyvaline, 3-phenylserine, 3-methyl-3-phenylalanine, histidine, tryptophan, 2-methylalanine, 2-methylserine, 2-hydroxyisoleucine, 2-methylmethionine, 2-ethyl-2-phenylglycine, 3-aminobutyric acid, 3-amino-4-methylvaleric acid, 3-amino-3-phenylpropionic acid, 3-amino-2-hydroxypropionic acid and 4-amino-3-hydroxybutyric acid.

More preferred amino acids are glycine, β-alanine, 4-aminobutyric acid, 5-aminovaleric acid, 6-aminohexanoic acid, 8-aminooctanoic acid, alanine, norvaline, valine, leucine, isoleucine, norleucine, $N^δ$,$N^δ$-dimethylornithine, methionine, ethionine, O-methylserine, O-methylthreonine, ethoxinine, 3-methoxyvaline, 3-phenylserine, 3-methyl-3-phenylalanine, histidine, 2-methylalanine, 2-methylserine, 2-hydroxyisoleucine, 2-ethylphenylglycine, 3-aminobutyric acid, 3-amino-4-methylvaleric acid and 3-amino-3-phenylpropionic acid.

The most preferred amino acids are β-alanine, 4-aminobutyric acid, 5-aminovaleric acid, 6-aminohexanoic acid, alanine, valine, leucine, norleucine, methionine, histidine and glycine.

When the amino acid derivative is an oligopeptide compound, such as a dipeptide or tripeptide, this type of compound is preferably formed by suitable combination of such amino acids as glycine, β-alanine, 4-aminobutyric acid, 5-aminovaleric acid, 6-aminohexanoic acid, alanine, valine, leucine, norleucine, phenylglycine, phenylalanine, methionine and histidine. Examples include leucylglycine, glycyl-β-alanine, glycylalanine, valylalanine, leucylalanine, glycylvaline, alanylvaline, leucylvaline, valylleucine, phenylalanylleucine, histidylleucine, glycylphenylalanine, alanylphenylalanine, leucylphenylalanine, glycylmethionine, valylmethionine, glycylhistidine, alanylvalylglycine, glycylalanylvaline, glycylphenylalanylleucine and glycylglycylhistidine.

Specific examples of the amino acid compounds which may be employed in the present invention are given in the following list. It should, of course, be appreciated that these compounds can exist in the D-, L- and DL- forms and any of these forms can be employed. The compounds are hereinafter referred to by the numbers appended to them in this list. In the case of the amino acids having 2 or more amino groups (e.g. 2,3-diaminopropionic acid, 2,4-diaminobutyric acid and arginine), mono-acylated derivatives (in which the acyl group can be on any amino group) and polyacylated derivatives are possible.

1. Glycine derivatives.

1-1. N-hexanoylglycine
1-2. N-heptanoylglycine
1-3. N-octanoylglycine
1-4. N-nonanoylglycine
1-5. N-decanoylglycine
1-6. N-(p-toluoyl)glycine
1-7. N-(4-methoxybenzoyl)glycine
1-8. N-(1-naphthoyl)glycine
1-9. N-(1-phenyl-1-cyclohexanecarbonyl)glycine
1-10. N-(α,α-diphenylacetyl)glycine
1-11. N-(α-phenyl-α-cyclopentylacetyl)glycine
1-12. N-butoxycarbonylglycine
1-13. N-octanoylleucylglycine
1-14. N--benzoylleucylglycine
1-15. N-butoxycarbonylleucylglycine
1-16. N-octanoylalanylvalylglycine
1-17. N-benzoylalanylvalylglycine
1-18. N-cyclohexanecarbonylalanylvalylglycine
1-19. N-butoxycarbonylalanylvalylglycine

2. β-Alanine derivatives

2-1. N-hexanoyl-β-alanine
2-2. N-heptanoyl-β-alanine
2-3. N-octanoyl-β-alanine
2-4. N-nonanoyl-β-alanine
2-5. N-(p-toluoyl)-β-alanine
2-6. N-(4-methoxybenzoyl)-β-alanine
2-7. N-(3-hydroxy-2-naphthoyl)-β-alanine
2-8. N-(1-phenyl-1-cyclopentanecarbonyl)-β-alanine
2-9. N-(α,α-diphenylacetyl)-β-alanine
2-10. N-(3-phenylpropionyl)-β-alanine
2-11. N-(4-phenylbutyryl)-β-alanine
2-12. N-(4-methoxyphenylacetyl)-β-alanine
2-13. N-t-butoxycarbonyl-β-alanine
2-14. N-benzyloxycarbonyl-β-alanine
2-15. N-(4-methoxybenzyloxycarbonyl)-β-alanine

2-16. N-(4-methylbenzyloxycarbonyl)-β-alanine
2-17. N-(α-methylbenzyloxycarbonyl)-β-alanine
2-18. N-benzoylglycyl-β-alanine
2-19. N-(1-naphthoyl)glycyl-β-alanine
2-20. N-cyclohexanecarbonylglycyl-β-alanine
2-21. N-benzyloxycarbonylglycyl-β-alanine
2-22. N-benzoyl-β-alanine

3. 4-Aminobutyric acid derivatives.

3-1. N-hexanoyl-4-aminobutyric acid
3-2. N-heptanoyl-4-aminobutyric acid
3-3. N-benzoyl-4-aminobutyric acid
3-4. N-(p-toluoyl)-4-aminobutyric acid
3-5. N-(3-methoxybenzoyl)-4-aminobutyric acid
3-6. N-cyclopentanecarbonyl-4-aminobutyric acid
3-7. N-cyclohexanecarbonyl-4-aminobutyric acid
3-8. N-(1-phenyl-1-cyclopropanecarbonyl)-4-aminobutyric acid
3-9. N-(1-phenyl-1-cyclopentanecarbonyl)-4-aminobutyric acid
3-10. N-phenylacetyl-4-aminobutyric acid
3-11. N-(3-phenylpropionyl)-4-aminobutyric acid
3-12. N-(p-tolylacetyl)-4-aminobutyric acid
3-13. N-nicotinoyl-4-aminobutyric acid
3-14. N-butoxycarbonyl-4-aminobutyric acid
3-15. N-benzyloxycarbonyl-4-aminobutyric acid
3-16. N-(3-phenylpropoxycarbonyl)-4-aminobutyric acid
3-17. N-(α-methylbenzyloxycarbonyl)-4-aminobutyric acid
3-18. N-(1-naphthoyl)-4-aminobutyric acid

4. 5-Aminovaleric acid derivatives

4-1. N-butyryl-5-aminovaleric acid
4-2. N-isobutyryl-5-aminovaleric acid
4-3. N-valeryl-5-aminovaleric acid
4-4. N-isovaleryl-5-aminovaleric acid
4-5. N-hexanoyl-5-aminovaleric acid
4-6. N-benzoyl-5-aminovaleric acid
4-7. N-(m-toluoyl)-5-aminovaleric acid
4-8. N-(2-methoxybenzoyl)-5-aminovaleric acid
4-9. N-cyclopentanecarbonyl-5-aminovaleric acid
4-10. N-cyclohexanecarbonyl-5-aminovaleric acid
4-11. N-(1-phenyl-1-cyclopropanecarbonyl)-5-aminovaleric acid
4-12. N-(1-phenyl-1-cyclohexanecarbonyl)-5-aminovaleric acid
4-13. N-phenylacetyl-5-aminovaleric acid
4-14. N-(α-phenyl-α-methylacetyl)-5-aminovaleric acid
4-15. N-nicotinoyl-5-aminovaleric acid
4-16. N-(2-thiophenecarbonyl)-5-aminovaleric acid
4-17. N-(2-furoyl)-5-aminovaleric acid
4-18. N-isopropoxycarbonyl-5-aminovaleric acid
4-19. N-pentyloxycarbonyl-5-aminovaleric acid
4-20. N-benzyloxycarbonyl-5-aminovaleric acid
4-21. N-(4-methoxybenzyloxycarbonyl)-5-aminovaleric acid
4-22. N-(4-methylbenzyloxycarbonyl)-5-aminovaleric acid
4-23. N-(4-hydroxyphenylacetyl)-5-aminovaleric acid
4-24. N(N-methylnicotinoyl)-5-aminovaleric acid

5. 6-Aminohexanoic acid derivatives.

5-1. N-acetyl-6-aminohexanoic acid
5-2. N-propionyl-6-aminohexanoic acid
5-3. N-butyryl-6-aminohexanoic acid
5-4. N-isobutyryl-6-aminohexanoic acid
5-5. N-isovaleryl-6-aminohexanoic acid
5-6. N-hexanoyl-6-aminohexanoic acid
5-7. N-acryloyl-6-aminohexanoic acid
5-8. N-methacryloyl-6-aminohexanoic acid
5-9. N-crotonoyl-6-aminohexanoic acid
5-10. N-propioloyl-6-aminohexanoic acid
5-11. N-benzoyl-6-aminohexanoic acid
5-12. N-(o-toluoyl)-6-aminohexanoic acid
5-13. N-(4-methoxybenzoyl)-6-aminohexanoic acid
5-14. N-(4-aminobenzoyl)-6-aminohexanoic acid
5-15. N-(1-naphthoyl)-6-aminohexanoic acid
5-16. N-cyclobutanecarbonyl-6-aminohexanoic acid
5-17. N-cyclopentanecarbonyl-6-aminohexanoic acid
5-18. N-cyclohexanecarbonyl-6-aminohexanoic acid
5-19. N-phenylacetyl-6-aminohexanoic acid
5-20. N-(3-phenylpropionyl)-6-aminohexanoic acid
5-21. N-nicotinoyl-6-aminohexanoic acid
5-22. N-(2-thiophenecarbonyl)-6-aminohexanoic acid
5-23. N-methoxycarbonyl-6-aminohexanoic acid
5-24. N-ethoxycarbonyl-6-aminohexanoic acid
5-25. N-butoxycarbonyl-6-aminohexanoic acid
5-26. N-pentyloxycarbonyl-6-aminohexanoic acid
5-27. N-benzyloxycarbonyl-6-aminohexanoic acid
5-28. N-phenethyloxycarbonyl-6-aminohexanoic acid
5-29. N-(3-phenylpropoxycarbonyl)-6-aminohexanoic acid
5-30. N-(4-methoxybenzyloxycarbonyl)-6-aminohexanoic acid
5-31. N-(4-methylbenzyloxycarbonyl)-6-aminohexanoic acid
5-32. N-(α-methylbenzyloxycarbonyl)-6-aminohexanoic acid
5-33. N-(N-methylnicotinoyl)-6-aminohexanoic acid
5-34. N-(4-chlorophenylacetyl)-6-aminohexanoic acid

6. 8-Aminooctanoic acid derivatives.

6-1. N-acetyl-8-aminooctanoic acid
6-2. N-valeryl-8-aminooctanoic acid
6-3. N-benzoyl-8-aminooctanoic acid
6-4. N-(3-hydroxybenzoyl)-8-aminooctanoic acid
6-5. N-(3-sulphobenzoyl)-8-aminooctanoic acid
6-6. N-cyclopropanecarbonyl-8-aminooctanoic acid
6-7. N-(4-aminophenylacetyl)-8-aminooctanoic acid
6-8. N-methoxycarbonyl-8-aminooctanoic acid
6-9. N-propoxycarbonyl-8-aminooctanoic acid
6-10. N-isopropoxycarbonyl-8-aminooctanoic acid
6-11. N-benzyloxycarbonyl-8-aminooctanoic acid
6-12. N-(4-hydroxybenzyloxycarbonyl)-8-aminooctanoic acid
6-13. N-(N-methylnicotinoyl)-8-aminooctanoic acid
6-14. N-(6-hydroxynicotinoyl)-8-aminooctanoic acid

7. Alanine derivatives.

7-1. N-valerylalanine
7-2. N-hexanoylalanine
7-3. N-benzoylalanine
7-4. N-(4-methoxybenzoyl)alanine

7-5. N-(1-naphthoyl)alanine
7-6. N-(1-phenyl-1-cyclopropanecarbonyl)alanine
7-7. N-phenylacetylalanine
7-8. N-butoxycarbonylalanine
7-9. N-benzyloxycarbonylalanine
7-10. N-(α-methylbenzyloxycarbonyl)alanine
7-11. N-octanoylglycylalanine
7-12. N-benzoylglycylalanine
7-13. N-butoxycarbonylglycylalanine
7-14. N-benzoylvalylalanine
7-15. N-(p-toluoyl)valylalanine
7-16. N-cyclopentanecarbonylvalylalanine
7-17. N-cyclohexanecarbonylvalylalanine
7-18. N-benzyloxycarbonylvalylalanine
7-19. N-benzoylleucylalanine
7-20. N-(4-methoxybenzoyl)leucylalanine
7-21. N-butoxycarbonylleucylalanine
7-22. N-benzyloxycarbonylleucylalanine
7-23. N-(2-bromobenzoyl)alanine

8. 2-Aminobutyric acid derivatives.

8-1. N-pivaloyl-2-aminobutyric acid
8-2. N-hexanoyl-2-aminobutyric acid
8-3. N-heptanoyl-2-aminobutyric acid
8-4. N-benzoyl-2-aminobutyric acid
8-5. N-(p-toluoyl)-2-aminobutyric acid
8-6. N-(1-phenyl-1-cyclopentanecarbonyl)-2-aminobutyric acid
8-7. N-(α,α-diphenylacetyl)-2-aminobutyric acid
8-8. N-ethoxycarbonyl-2-aminobutyric acid
8-9. N-benzyloxycarbonyl-2-aminobutyric acid
8-10. N-(4-methoxybenzyloxycarbonyl-2-aminobutyric acid

9. Norvaline derivatives.

9-1. N-valerylnorvaline
9-2. N-decanoylnorvaline
9-3. N-benzoylnorvaline
9-4. N-(m-toluoyl)norvaline
9-5. N-(3-sulphobenzoyl)norvaline
9-6. N-cyclohexanecarbonylnorvaline
9-7. N-(1-phenyl-1-cyclohexanecarbonyl)norvaline
9-8. N-(α-phenyl-α-ethylacetyl)norvaline
9-9. N-(4-methoxyphenylacetyl)norvaline
9-10. N-(2-pyrazinecarbonyl)norvaline
9-11. N-benzyloxycarbonylnorvaline
9-12. N-(4-methylbenzyloxycarbonyl)norvaline

10. Valine derivatives.

10-1. N-propionylvaline
10-2. N-butyrylvaline
10-3. N-isobutyrylvaline
10-4. N-valerylvaline
10-5. N-acryloylvaline
10-6. N-methacryloylvaline
10-7. N-crotonoylvaline
10-8. N-propioloylvaline

10-9. N-(2-methoxybenzoyl)valine
10-10. N-(4-butoxybenzoyl)valine
10-11. N-cyclopentanecarbonylvaline
10-12. N-cyclohexanecarbonylvaline
10-13. N-(1-phenyl-1-cyclopentanecarbonyl)valine
10-14. N-phenylacetylvaline
10-15. N-nicotinoylvaline
10-16. N-(2-piperidinecarbonyl)valine
10-17. N-ethoxycarbonylvaline
10-18. N-isopropoxycarbonylvaline
10-19. N-t-butoxycarbonylvaline
10-20. N-pentyloxycarbonylvaline
10-21. N-benzyloxycarbonylvaline
10-22. N-(p-p-tolylacetyl)valine
10-23. N-benzoylglycylvaline
10-24. N-(p-toluoyl)glycylvaline
10-25. N-(1-naphthoyl)glycylvaline
10-26. N-cyclopentanecarbonylglycylvaline
10-27. N-butoxycarbonylglycylvaline
10-28. N-octanoylalanylvaline
10-29. N-benzoylalanylvaline
10-30. N-(p-toluoyl)alanylvaline
10-31. N-(4-aminobenzoyl)alanylvaline
10-32. N-(1-naphthoyl)alanylvaline
10-33. N-cyclohexanecarbonylalanylvaline
10-34. N-phenylacetylalanylvaline
10-35. N-benzyloxycarbonylalanylvaline
10-36. N-benzoylleucylvaline
10-37. N-benzoylglycylalanylvaline
10-38. N-(p-toluoyl)glycylalanylvaline
10-39. N-(1-naphthoyl)glycylalanylvaline
10-40. N-cyclopentanecarbonylglycylalanylvaline
10-41. N-butoxycarbonylglycylalanylvaline
10-42. N-benzyloxycarbonylglycylalanylvaline
10-43. N-(N-methylnicotinoyl)valine
10-44. N-(3-fluorobenzoyl)valine
10-45. N-benzoylvaline

11. Leucine derivatives.

11-1. N-butyrylleucine
11-2. N-isovalerylleucine
11-3. N-benzoylleucine
11-4. N-(4-butylbenzoyl)leucine
11-5. N-(2-hydroxybenzoyl)leucine
11-6. N-(3-sulphobenzoyl)leucine
11-7. N-cyclopentanecarbonylleucine
11-8. N-cyclohexanecarbonylleucine
11-9. N-(1-phenyl-1-cyclopropanecarbonyl)leucine
11-10. N-phenylacetylleucine
11-11. N-nicotinoylleucine
11-12. N-ethoxycarbonylleucine
11-13. N-benzyloxycarbonylleucine
11-14. N-(4-hydroxyphenylacetyl)leucine
11-15. N-benzoylvalylleucine
11-16. N-ethoxycarbonylvalylleucine
11-17. N-benzoylphenylalanylleucine
11-18. N-phenylacetylphenylalanylleucine

11-19. N-benzyloxycarbonylphenylalanylleucine
11-20. N-benzoylhistidylleucine
11-21. N-(p-toluoyl)histidylleucine
11-22. N-(4-hydroxybenzoyl)histidylleucine
11-23. N-(1-naphthoyl)histidylleucine
11-24. N-benzoylglycylphenylalanylleucine
11-25. N-(4-methoxybenzoyl)glycylphenylalanylleucine
11-26. N-phenylacetylglycylphenylalanylleucine
11-27. N-t-butoxycarbonylleucine

12. Isoleucine derivatives.

12-1. N-valerylisoleucine
12-2. N-pivaloylisoleucine
12-3. N-octanoylisoleucine
12-4. N-benzoylisoleucine
12-5. N-(3-hydroxybenzoyl)isoleucine
12-6. N-cyclopentanecarbonylisoleucine
12-7. N-cyclohexanecarbonylisoleucine
12-8. N-(1-phenyl-1-cyclopentanecarbonyl)isoleucine
12-9. N-phenylacetylisoleucine
12-10. N-methoxycarbonylisoleucine
12-11. N-propoxycarbonylisoleucine
12-12. N-isopropoxycarbonylisoleucine
12-13. N-benzyloxycarbonylisoleucine

13. Norleucine derivatives.

13-1. N-propionylnorleucine
13-2. N-valerylnorleucine
13-3. N-pivaloylnorleucine
13-4. N-nonanoylnorleucine
13-5. N-benzoylnorleucine
13-6. N-(4-hydroxybenzoyl)norleucine
13-7. N-cyclohexanecarbonylnorleucine
13-8. N-(1-phenyl-1-cyclopropanecarbonyl)norleucine
13-9. N-(α-phenyl-α-ethylacetyl)norleucine
13-10. N-ethoxycarbonylnorleucine
13-11. N-propoxycarbonylnorleucine
13-12. N-butoxycarbonylnorleucine
13-13. N-benzyloxycarbonylnorleucine

14. Oligopeptide derivatives

14-1. N-benzoylglycylphenylalanine
14-2. N-(4-hydroxybenzoyl)glycylphenylalanine
14-3. N-(1-naphthoyl)glycylphenylalanine
14-4. N-ethoxycarbonylglycylphenylalanine
14-5. N-benzyloxycarbonylglycylphenylalanine
14-6. N-benzoylalanylphenylalanine
14-7. N-(p-toluoyl)alanylphenylalanine
14-8. N-(4-hydroxybenzoyl)alanylphenylalanine
14-9. N-(4-aminobenzoyl)alanylphenylalanine
14-10. N-(1-naphthoyl)alanylphenylalanine
14-11. N-benzyloxycarbonylalanylphenylalanine
14-12. N-benzoylleucylphenylaline
14-13. N-(4-hydroxybenzoyl)leucyphenylalanine
14-14. N-cyclohexanecarbonylleucylphenylalanine

14-15. N-benzyloxycarbonylleucylphenylalanine

15. Tyrosine derivatives.

15-1. N-benzoyltyrosine
15-2. N-(3-methoxybenzoyl)tyrosine
15-3. N-cyclohexanecarbonyltyrosine
15-4. N-benzyloxycarbonyltyrosine
15-5. N-phenethyloxycarbonyltyrosine

16. O-Methyltyrosine derivatives.

16-1. N-acetyl-O-methyltyrosine
16-2. N-propioloyl-O-methyltyrosine
16-3. N-benzoyl-O-methyltyrosine
16-4. N-(4-aminobenzoyl)-O-methyltyrosine
16-5. N-(1-phenyl-1-cyclopentanecarbonyl)-O-methyltyrosine
16-6. N-(1-phenyl-1-cyclohexanecarbonyl)-O-methyltyrosine
16-7. N-methoxycarbonyl-O-methyltyrosine
16-8. N-benzyloxycarbonyl-O-methyltyrosine
16-9. N-phenethyloxycarbonyl-O-methyltyrosine

17. Aspartic acid derivatives.

17-1. N-heptanoylaspartic acid
17-2. N-decanoylaspartic acid
17-3. N-(4-hydroxybenzoyl)aspartic acid
17-4. N-(3-hydroxy-2-naphthoyl)aspartic acid
17-5. N-(1-phenyl-1-cyclopentanecarbonyl)aspartic acid
17-6. N-(1-phenyl-1-cyclohexanecarbonyl)aspartic acid
17-7. N-benzyloxycarbonylaspartic acid
17-8. N-(4-methoxybenzyloxycarbonyl)aspartic acid

18. Glutamic acid derivatives.

18-1. N-nonanoylglutamic acid
18-2. N-(4-methoxybenzoyl)glutamic acid
18-3. N-(1-naphthoyl)glutamic acid
18-4. N-(1-phenyl-1-cyclopentanecarbonyl)glutamic acid
18-5. N-benzyloxycarbonylglutamic acid
18-6. N-benzoylglutamic acid

19. 4-Carboxyglutamic acid derivatives.

19-1. N-heptanoyl-4-carboxyglutamic acid
19-2. N-(4-methoxybenzoyl)-4-carboxyglutamic acid
19-3. N-(1-naphthoyl)-4-carboxyglutamic acid
19-4. N-(1-hydroxy-2-naphthoyl)-4-carboxyglutamic acid
19-5. N-phenylacetyl-4-carboxyglutamic acid

20. 3-Methylaspartic acid derivatives.

20-1. N-octanoyl-3-methylaspartic acid
20-2. N-(4-methoxybenzoyl)-3-methylaspartic acid
20-3. N-(α-phenyl-α-cyclopentylacetyl)-3-methylaspartic acid

21. 2-Aminoadipic acid derivatives.

21-1. N-hexanoyl-2-aminoadipic acid
21-2. N-benzoyl-2-aminoadipic acid
21-3. N-(p-toluoyl)-2-aminoadipic acid
21-4. N-(1-naphthoyl)-2-aminoadipic acid
21-5. N-(4-phenylbutyryl)-2-aminoadipic acid
21-6. N-phenylacetyl-2-aminoadipic acid
21-7. N-ethoxycarbonyl-2-aminoadipic acid

22. 2-Aminopimelic acid derivatives.

22-1. N-valeryl-2-aminopimelic acid
22-2. N-benzoyl-2-aminopimelic acid
22-3. N-(3-phenylpropionyl)-2-aminopimelic acid
22-4. N-methoxycarbonyl-2-aminopimelic acid
22-5. N-ethoxycarbonyl-2-aminopimelic acid
22-6. N-benzyloxycarbonyl-2-aminopimelic acid

23. 2-Aminosuberic acid derivatives.

23-1. N-butyryl-2-aminosuberic acid
23-2. N-benzoyl-2-aminosuberic acid
23-3. N-(1-naphthoyl)-2-aminosuberic acid
23-4. N-(α-phenyl-α-cyclopentylacetyl)-2-aminosuberic acid
23-5. N-methoxycarbonyl-2-aminosuberic acid
23-6. N-propoxycarbonyl-2-aminosuberic acid

24. 3-Hydroxyaspartic acid derivatives.

24-1. N-(1-naphthoyl)-3-hydroxyaspartic acid
24-2. N-(1-phenyl-1-cyclohexanecarbonyl)-3-hydroxyaspartic acid
24-3. N-(α-phenyl-α-ethylacetyl)-3-hydroxyaspartic acid

25. 3-Hydroxyglutamic acid derivatives.

25-1. N-(1-naphthoyl)-3-hydroxyglutamic acid
25-2. N-(1-phenyl-1-cyclohexanecarbonyl)-3-hydroxyglutamic acid
25-3. N-(α,α-diphenylacetyl)-3-hydroxyglutamic acid

26. 2,3-Diaminopropionic acid derivatives.

26-1. $N^{\alpha}$-hexanoyl-2,3-diaminopropionic acid
26-2. $N^{\alpha}$-(4-butylbenzoyl)-2,3-diaminopropionic acid
26-3. $N^{\alpha}$,$N^{\beta}$-dibenzoyl-2,3-diaminopropionic acid
26-4. $N^{\alpha}$-(1-phenyl-1-cyclopentanecarbonyl)-2,3-diaminopropionic acid
26-5. $N^{\alpha}$-(α-phenyl-α-ethylacetyl)-2,3-diaminopropionic acid

27. 2,4-Diaminobutyric acid derivatives.

27-1. $N^{\alpha}$-(1-naphthoyl)-2,4-diaminobutyric acid
27-2. $N^{\alpha}$,$N^{\gamma}$-dibenzoyl-2,4-diaminobutyric acid
27-3. $N^{\alpha}$-(1-phenyl-1-cyclopentanecarbonyl)-2,4-diaminobutyric acid
27-4. $N^{\alpha}$-(α-phenyl-α-ethylacetyl)-2,4-diaminobutyric acid

28. 5-Hydroxylysine derivatives.

28-1. $N^{\alpha}$-(p-toluoyl)-5-hydroxylysine
28-2. $N^{\alpha}$,$N^{\delta}$-dibenzoyl-5-hydroxylysine
28-3. $N^{\alpha}$-(1-phenyl-1-cyclopentanecarbonyl)-5-hydroxylysine

28-4. $N^{\alpha}$-($\alpha$-phenyl-$\alpha$-cyclopentylacetyl)-5-hydroxylysine
28-5. $N^{\alpha}$-(1-phenyl-1-cyclopentanecarbonyl)-5-hydroxylysine

29. Arginine derivatives.

29-1. $N^{\alpha}$-heptanoylarginine
29-2. $N^{\alpha}$-(2-methoxybenzoyl)arginine

30. $N^{\delta}$,$N^{\delta}$-Dimethylornithine derivatives.

30-1. $N^{\alpha}$-pivaloyl-$N^{\delta}$,$N^{\delta}$-dimethylornithine
30-2. $N^{\alpha}$-octanoyl-$N^{\delta}$,$N^{\delta}$-dimethylornithine
30-3. $N^{\alpha}$-acryloyl-$N^{\delta}$,$N^{\delta}$-dimethylornithine
30-4. $N^{\alpha}$-benzoyl-$N^{\delta}$,$N^{\delta}$-dimethylornithine
30-5. $N^{\alpha}$-(4-hydroxybenzoyl)-$N^{\delta}$,$N^{\delta}$-dimethylornithine
30-6. $N^{\alpha}$-cyclohexanecarbonyl-$N^{\delta}$,$N^{\delta}$-dimethylornithine
30-7. $N^{\alpha}$-($\alpha$-phenyl-$\alpha$-methylacetyl)-$N^{\delta}$,$N^{\delta}$-dimethylornithine
30-8. $N^{\alpha}$-ethoxycarbonyl-$N^{\alpha}$,$N^{\alpha}$-dimethylornithine
30-9. $N^{\alpha}$-butoxycarbonyl-$N^{\delta}$,$N^{\delta}$-dimethylornithine
30-10. $N^{\alpha}$-benzyloxycarbonyl-$N^{\delta}$,$N^{\delta}$-dimethylornithine

31. $N^{\epsilon}$-Methyllysine derivatives.

31-1. $N^{\alpha}$-hexanoyl-$N^{\epsilon}$-methyllysine
31-2. $N^{\alpha}$-nonanoyl-$N^{\epsilon}$-methyllysine
31-3. $N^{\alpha}$-acryloyl-$N^{\epsilon}$-methyllysine
31-4. $N^{\alpha}$-benzoyl-$N^{\epsilon}$-methyllysine
31-5. $N^{\alpha}$-(4-butoxybenzoyl)-$N^{\epsilon}$-methyllysine
31-6. $N^{\alpha}$-(3-sulphobenzoyl)-$N^{\epsilon}$-methyllysine
31-7. $N^{\alpha}$-cyclobutanecarbonyl-$N^{\epsilon}$-methyllysine
31-8. $N^{\alpha}$-cyclohexanecarbonyl-$N^{\epsilon}$-methyllysine
31-9. $N^{\alpha}$-phenylacetyl-$N^{\epsilon}$-methyllysine
31-10. $N^{\alpha}$-propoxycarbonyl-$N^{\epsilon}$-methyllysine
31-11. $N^{\alpha}$-isopropoxycarbonyl-$N^{\epsilon}$-methyllysine
31-12. $N^{\alpha}$-benzyloxycarbonyl-$N^{\epsilon}$-methyllysine

32. Cysteine derivatives.

32-1. N-phenylacetylcysteine

33. Methionine derivatives.

33-1. N-valerylmethionine
33-2. N-acryloylmethionine
33-3. N-methacryloylmethionine
33-4. N-benzoylmethionine
33-5. N-(p-toluoyl)methionine
33-6. N-(4-methoxybenzoyl)methionine
33-7. N-(4-aminobenzoyl)methionine
33-8. N-cyclopentanecarbonylmethionine
33-9. N-cyclohexanecarbonylmethionine
33-10. N--(1-phenyl-1-cyclohexanecarbonyl)methionine
33-11. N-phenylacetylmethionine
33-12. N-($\alpha$-phenyl-$\alpha$-methylacetyl)methionine
33-13. N-methoxycarbonylmethionine
33-14. N-ethoxycarbonylmethionine
33-15. N-butoxycarbonylmethionine
33-16. N-benzyloxycarbonylmethionine

33-17. N-(4-methylbenzyloxycarbonyl)methionine
33-18. N-benzoylglycylmethionine
33-19. N-(4-methoxybenzoyl)glycylmethionine
33-20. N-benzyloxycarbonylglycylmethionine
33-21. N-benzoylvalylmethionine
33-22. N-cyclopentanecarbonylvalylmethionine
33-23. N-ethoxycarbonylvalylmethionine

34. Ethionine derivatives.

34-1. N-butyrylethionine
34-2. N-benzoylethionine
34-3. N-(p-toluoyl)ethionine
34-4. N-(m-toluoyl)ethionine
34-5. N-(4-butylbenzoyl)ethionine
34-6. N-(4-hydroxybenzoyl)ethionine
34-7. N-(4-aminobenzoyl)ethionine
34-8. N-(3-sulphobenzoyl)ethionine
34-9. N-(1-phenyl-1-cyclopropanecarbonyl)ethionine
34-10. N-phenylacetylethionine
34-11. N-methoxycarbonylethionine
34-12. N-ethoxycarbonylethionine
34-13. N-benzyloxycarbonylethionine
34-14. N-(4-methoxybenzyloxycarbonyl)ethionine
34-15. N-cyclohexanecarbonylethionine

35. S-Carboxymethylcysteine derivatives.

35-1. N-propionyl-S-carboxymethylcysteine
35-2. N-acryloyl-S-carboxymethylcysteine
35-3. N-benzoyl-S-carboxymethylcysteine
35-4. N-(p-toluoyl)-S-carboxymethylcysteine
35-5. N-(4-methoxybenzoyl)-S-carboxymethylcysteine
35-6. N-(4-butoxybenzoyl)-S-carboxymethylcysteine
35-7. N-cyclohexanecarbonyl-S-carboxymethylcysteine
35-8. N-(1-phenyl-1-cyclopentanecarbonyl)-S-carboxymethylcysteine
35-9. N-(α-methylbenzyloxycarbonyl)-S-carboxymethylcysteine

36. S-Benzylcysteine derivatives.

36-1. N-benzoyl-S-benzylcysteine
36-2. N-(4-hydroxybenzoyl)-S-benzylcysteine
36-3. N-(3-sulphobenzoyl)-S-benzylcysteine
36-4. N-cyclopropanecarbonyl-S-benzylcysteine
36-5. N-methoxycarbonyl-S-benzylcysteine
36-6. N-ethoxycarbonyl-S-benzylcysteine
36-7. N-propoxycarbonyl-S-benzylcysteine
36-8. N-(4-hydroxybenzyloxycarbonyl)-S-benzylcysteine

37. Methionine S-oxide derivatives.

37-1. N-(p-toluoyl)methionine S-oxide
37-2. N-pentyloxycarbonylmethionine S-oxide
37-3. N-benzyloxycarbonylmethionine S-oxide

38. Ethionine S-oxide derivatives.

38-1. N-benzoylethionine S-oxide

38-2. N-benzyloxycarbonylethionine S-oxide

39. Methionine S,S-dioxide derivatives.

39-1. N-(1-napthoyl)methionine S,S-dioxide
39-2. N cyclohexanecarbonylmethionine S,S-dioxide
39-3. N-pentyloxycarbonylmethionine S,S-dioxide

40. Cysteic acid derivatives.

40-1. N-(p-toluoyl)cysteic acid
40-2. n-(1-napthoyl)cysteic acid
40-3. N-(3-hydroxy-2-napthoyl)cysteic acid
40-4. N-(1-phenyl-1-cyclohexanecarbonyl)cysteic acid

41. Serine derivatives.

41-1. N-octanoylserine
41-2. N-benzoylserine
41-3. N-(m-toluoyl)serine
41-4. N-(4-methoxybenzoyl)serine
41-5. N-(1-napthoyl)serine
41-6. N-(1-phenyl-1-cyclopentanecarbonyl)serine
41-7. N-benzyloxycarbonylserine
41-8. N-(α-methylbenzyloxycarbonyl)serine

42. O-methylserine derivatives.

42-1. N-valeryl-O-methylserine
42-2. N-benzoyl-O-methylserine
42-3. N-cyclohexanecarbonyl-O-methylserine
42-4. N-phenylacetyl-O-methylserine
42-5. N-(α-phenyl-α-methylacetyl)-O-methylserine
42-6. N-(3-phenylpropionyl)-O-methylserine
42-7. N-phenethyloxycarbonyl-O-methylserine

43. Threonine derivatives.

43-1. N-hexanoylthreonine
43-2. N-nonanoylthreonine
43-3. N-benzoylthreonine
43-4. N-(3-hydroxy-2-naphthoyl)threonine
43-5. N-cyclohexanecarbonylthreonine
43-6. N-(α,α-diphenylacetyl)threonine
43-7. N-butoxycarbonylthreonine
43-8. N-benzyloxycarbonylthreonine
43-9. N-(4-methoxybenzyloxycarbonyl)threonine

44. O-Methylthreonine derivatives.

44-1. N-butyryl-O-methylthreonine
44-2. N-(4-methoxybenzyoyl)-O-methylthreonine
44-3. N-(1-naphthoyl)-O-methylthreonine
44-4. N-(1-phenyl-1-cyclopentanecarbonyl)-O-methylthreonine
44-5. N-ethyoxycarbonyl-O-methylthreonine
44-6. N-(3-phenylpropoxycarbonyl)-O-methylthreonine

45. Homoserine derivatives.

45-1. N-heptanoylhomoserine
45-2. N-benzoylhomoserine
45-3. N-(3-methoxybenzoyl)homoserine
45-4. N-(α-phenyl-α-cyclopentylacetyl)homoserine
45-5. N-(4-hydroxybenzyloxycarbonyl)homoserine
45-6. N-(4-methylbenzyloxycarbonyl)homoserine

46. Ethoxinine derivatives.

46-1. N-benzoylethoxinine
46-2. N-(4-butoxybenzoyl)ethoxinine
46-3. N-cyclohexanecarbonylethoxinine
46-4. N-methoxycarbonylethoxinine

47. 3-Methoxyvaline derivatives.

47-1. N-isovaleryl-3-methoxyvaline
47-2. N-(p-toluoyl)-3-methoxyvaline
47-3. N-(1-naphthoyl)-3-methoxyvaline
47-4. N-cyclopentanecarbonyl-3-methoxyvaline
47-5. N-cyclohexanecarbonyl-3-methoxyvaline
47-6. N-methoxycarbonyl-3-methoxyvaline
47-7. N-ethoxycarbonyl-3-methoxyvaline

48. 3-Phenylserine derivatives.

48-1. N-propionyl-3-phenylserine
48-2. N-(4-aminobenzoyl)-3-phenylserine
48-3. N-(1-naphthoyl)-3-phenylserine
48-4. N-benzoyl-3-phenylserine
48-5. N-cyclohexanecarbonyl-3-phenylserine
48-6. N-phenylacetyl-3-phenylserine
48-7. N-methoxycarbonyl-3-phenylserine
48-8. N-butoxycarbonyl-3-phenylserine
48-9. N-benzyloxycarbonyl-3-phenylserine
48-10. N-(α-methylbenzyloxycarbonyl)-3-phenylserine

49. 3-Methyl-3-phenylalanine derivatives.

49-1. N-acetyl-3-methyl-3-phenylalanine
49-2. N-hexanoyl-3-methyl-3-phenylalanine
49-3. N-benzoyl-3-methyl-3-phenylalanine
49-4. N-(4-aminobenzoyl)-3-methyl-3-phenylalanine
49-5. N-(3-sulphobenzoyl)-3-methyl-3-phenylalanine
49-6. N-cyclobutanecarbonyl-3-methyl-3-phenylalanine
49-7. N-cyclopentanecarbonyl-3-methyl-3-phenylalanine
49-8. N-phenylacetyl-3-methyl-3-phenylalanine
49-9. N-isopropoxycarbonyl-3-methyl-3-phenylalanine
49-10. N-butoxycarbonyl-3-methyl-3-phenylalanine
49-11. N-(4-aminobenzyloxycarbonyl)-3-methyl-3-phenylalanine

50. Histidine derivatives.

50-1. N-acetylhistidine
50-2. N-hexanoylhistidine
50-3. N-acryloylhistidine
50-4. N-methacryloylhistidine
50-5. N-benzoylhistidine

50-6. N-(p-toluoyl)histidine
50-7. N-(4-methoxybenzoyl)histidine
50-8. N-(4-butoxybenzoyl)histidine
50-9. N-cyclopentanecarbonylhistidine
50-10. N-cyclohexanecarbonylhistidine
50-11. N-(1-phenyl-1-cyclopentanecarbonyl)histidine
50-12. N-phenylacetylhistidine
50-13. N-(α-phenyl-α-cyclopentylacetyl)histidine
50-14. N-(4-methoxybenzyloxycarbonyl)histidine
50-15. N-benzoylglycylhistidine
50-16. N-(4-butylbenzoyl)glycylhistidine
50-17. N-phenylacetylglycylhistidine
50-18. N-ethoxycarbonylglycylhistidine
50-19. N-benzyloxycarbonylglycylhistidine
50-20. N-benzoylglycylglyclhistidine
50-21. N-ethoxycarbonylglycylglycylhistidine
50-22. N-benzyloxycarbonylglycylglycylhistidine
50-23. N-t-butoxycarbonylhistidine

51. Tryptophan derivatives.

51-1. N-(4-hydroxybenzoyl)tryptophan
51-2. N-benzyloxycarbonyltryptophan

52. 2-Methylalanine derivatives.

52-1. N-propionyl-2-methylalanine
52-2. N-benzoyl-2-methylalanine
52-3. N-(m-toluoyl)-2-methylalanine
52-4. N-(3-methoxybenzoyl)-2-methylalanine
52-5. N-cyclobutanecarbonyl-2-methylalanine
52-6. N-phenylacetyl-2-methylalanine
52-7. N-phenethyloxycarbonyl-2-methylalanine

53. 2-Methylserine derivatives.

53-1. N-valeryl-2-methylserine
53-2. N-octanoyl-2-methylserine
53-3. N-benzoyl-2-methylserine
53-4. N-(o-toluoyl)-2-methylserine
53-5. N-(4-methoxybenzoyl)-2-methylserine
53-6. N-(1-naphthoyl)-2-methylserine
53-7. N-cyclopentanecarbonyl-2-methylserine
53-8. N-(α,α-diphenylacetyl)-2-methylserine
53-9. N-pentyloxycarbonyl-2-methylserine

54. 2-Hydroxyisoleucine derivatives.

54-1. N-valeryl-2-hydroxyisoleucine
54-2. N-heptanoyl-2-hydroxyisoleucine
54-3. N-benzoyl-2-hydroxyisoleucine
54-4. N-(4-butylbenzoyl)-2-hydroxyisoleucine
54-5. N-(3-hydroxy-2-naphthoyl)-2-hydroxyisoleucine
54-6. N-cyclohexanecarbonyl-2-hydroxyisoleucine
54-7. N-phenylacetyl-2-hydroxyisoleucine

55. 2-Methylmethionine derivatives.

55-1. N-hexanoyl-2-methylmethionine
55-2. N-benzoyl-2-methylmethionine
55-3. N-(4-hydroxybenzoyl)-2-methylmethionine
55-4. N-propoxycarbonyl-2-methylmethionine
55-5. N-isopropoxycarbonyl-2-methylmethionine

56. 2-Ethyl-2-phenylglycine derivatives.

56-1. N-acetyl-2-ethyl-2-phenylglycine
56-2. N-butyryl-2-ethyl-2-phenylglycine
56-3. N-(3-sulphobenzoyl)-2-ethyl-2-phenylglycine
56-4. N-ethoxycarbonyl-2-ethyl-2-phenylglycine
56-5. N-propoxycarbonyl-2-ethyl-2-phenylglycine

57. 3-Aminobutyric acid derivatives.

57-1. N-hexanoyl-3-aminobutyric acid
57-2. N-benzoyl-3-aminobutyric acid
57-3. N-(4-methoxybenzoyl)-3-aminobutyric acid
57-4. N-(3-sulphobenzoyl)-3-aminobutyric acid
57-5. N-(1-naphthoyl)-3-aminobutyric acid
57-6. N-cyclopropanecarbonyl-3-aminobutyric acid
57-7. N-(α,α-diphenylacetyl)-3-aminobutyric acid
57-8. N-(4-phenylbutyl)-3-aminobutyric acid
57-9. N-(α-methylbenzyloxycarbonyl)-3-aminobutyric acid

58. 3-Amino-4-methylvaleric acid derivatives.

58-1. N-valeryl-3-amino-4-methylvaleric acid
58-2. N-isovaleryl-3-amino-4-methylvaleric acid
58-3. N-heptanoyl-3-amino-4-methylvaleric acid
58-4. N-benzoyl-3-amino-4-methylvaleric acid
58-5. N-(m-toluoyl)-3-amino-4-methylvaleric acid
58-6. N-(3-sulphobenzoyl)-3-amino-4-methylvaleric acid
58-7. N-(1-naphthoyl)-3-amino-4-methylvaleric acid
58-8. N-phenylacetyl-3-amino-4-methylvaleric acid
58-9. N-(3-phenylpropionyl)-3-amino-4-methylvaleric acid
58-10. N-butoxycarbonyl-3-amino-4-methylvaleric acid
58-11. N-(4-methylbenzyloxycarbonyl)-3-amino-4-methylvaleric acid

59. 3-Amino-3-phenylpropionic acid derivatives.

59-1. N-butyryl-3-amino-3-phenylpropionic acid
59-2. N-valeryl-3-amino-3-phenylpropionic acid
59-3. N-benzoyl-3-amino-3-phenylpropionic acid
59-4. N-(4-aminobenzoyl)-3-amino-3-phenylpropionic acid
59-5. N-cyclopropanecarbonyl-3-amino-3-phenylpropionic acid
59-6. N-cyclobutanecarbonyl-3-amino-3-phenylpropionic acid
59-7. N-cyclopentanecarbonyl-3-amino-3-phenylpropionic acid
59-8. N-methoxycarbonyl-3-amino-3-phenylpropionic acid
59-9. N-propoxycarbonyl-3-amino-3-phenylpropionic acid
59-10. N-butoxycarbonyl-3-amino-3-phenylpropionic acid
59-11. N-(4-aminobenzyloxycarbonyl)-3-amino-3-phenyl-propionic acid

60. 3-Amino-2-hydroxypropionic acid derivatives.

60-1. N-valeryl-3-amino-2-hydroxypropionic acid
60-2. N-heptanoyl-3-amino-2-hydroxypropionic acid

60-3. N-benzoyl-3-amino-2-hydroxypropionic acid
60-4. N-(3-methoxybenzoyl)-3-amino-2-hydroxypropionic acid
60-5. N-cyclohexanecarbonyl-3-amino-2-hydroxypropionic acid
60-6. N-benzyloxycarbonyl-3-amino-2-hydroxypropionic acid
60-7. N-(3-phenylpropoxycarbonyl)-3-amino-2-hydroxypropionic acid

61. 4-Amino-3-hydroxybutyric acid derivatives

61-1. N-isobutyryl-4-amino-3-hydroxybutyric acid
61-2. N-decanoyl-4-amino-3-hydroxybutyric acid
61-3. N-benzoyl-4-amino-3-hydroxybutyric acid
61-4. N-(o-toluoyl)-4-amino-3-hydroxybutyric acid
61-5. N-(3-aminobenzoyl)-4-amino-3-hydroxybutyric acid
61-6. N-(1-phenyl-1-cyclohexanecarbonyl)-4-amino-3-hydroxybutyric acid
61-7. N-(α-phenyl-α-methylacetyl)-4-amino-3-hydroxybutyric acid
61-8. N-(4-methoxybenzyloxycarbonyl)-4-amino-3-hydroxybutyric acid

Of the amino acid derivatives listed above, the following are particularly preferred: Compounds No. 2-5, 2-6, 2-7, 2-18, 2-22, 4-6, 5-11, 5-18, 5-33, 6-3, 7-3, 7-5, 7-14, 8-4, 9-3, 10-4, 10-29, 10-45, 11-3, 11-24, 13-5, 14-1, 14-6, 14-14, 16-3, 33-4, 33-11, 33-21, 34-2, 34-14, 43-3, 50-5, 50-6, 50-7, 57-3 and 59-1. Of these, Compounds No. 2-22 and 10-45, especially No. 2-22, are most preferred.

The amino acid derivatives employed in the present invention are acids and, as such, are capable of forming salts; any pharmaceutically acceptable salt of these amino acids may be employed. Examples of such salts include: alkali metal salts such as the sodium or potassium salts; alkaline earth metal salts, such as the calcium salt; other metal salts, such as the magnesium, aluminium, iron, zinc, copper, nickel and cobalt salts; the ammonium salt; and salts with amino sugars, such as glucosamine and galactosamine.

The compositions of the invention may be prepared by any suitable method which involves mixing the antibiotic with the amino acid derivative and the invention is not intended to be limited by any particular method of preparation. Since the amino acid derivatives employed in this invention have, in general, very limited solubility in water, it is preferred that they should first be dispersed in water and then converted to a suitable salt by adding an aqueous solution of an appropriate base, for example: a metal compound, such as sodium hydroxide or potassium hydroxide; ammonia; or an amino sugar, such as glucosamine or galactosamine. Sufficient of the base is preferably added to adjust the pH of the mixture to a value within the range from 5.5 to 9, more preferably from 6 to 9.

The penem or carbapenem antibiotic is then added to the resulting solution. The mixed solution thus obtained may be employed as such or it may first be lyophilized to give a powdery mixture, which may be subsequently formulated into an appropriate dosage form suitable for the chosen route of administration, either by the manufacturer or prior to use.

The above mixing and preparation steps may take place at any temperature at which the components are fluid (especially the media) and are not decomposed, e.g. from 0 to 100°C, more conveniently from 0 to 50°C and most conveniently at about ambient temperature.

Although it is convenient to administer the antibiotic and the amino acid derivative simultaneously in a single composition, it is, of course, clear that the two compounds may be administered separately, provided that they are administered sufficiently closely in time to each other that the amino acid derivative has a suitable concentration in the blood for all or most of the time that the antibiotic is present. Normally, it is anticipated that this will be achieved if the two compounds are administered within about one hour of each other, the amino acid preferably being administered before the antibiotic.

The composition of the invention is particularly suitable for use by intravenous administration.

There is no particular restriction on the relative proportions of the amino acid derivative and the penem or carbapenem antibiotic; in general, we have found that weight proportions of amino acid derivative to antibiotic of from 0.1:1 to 4:1 give good results, but equally proportions outside this range may successfully be employed. Approximately equal weights are generally most convenient.

The invention is further illustrated by the following Examples and Activity Tests. In the following, the penem or carbapenem antibiotics are referred to by the numbers appended to them in the foregoing list and are identified as "(Carba)-Penem Cpd No" whilst the amino acid derivatives are also identified by the numbers appended to them in the foregoing list and are referred to as "Amino Acid Cpd No."

EXAMPLE 1

5 g of N-benzoylvaline (Amino Acid Compound No. 10-45) were weighed out and dispersed in 80 ml of water. A 1N aqueous solution of sodium hydroxide was slowly added to this dispersion, and dissolved the N-benzoylvaline when the pH of the solution reached a value of 7-8. Then, 5 g of (5R,6S)-2-[(3S)-1-acetimidoylpyrrolidin-3-ylthio]-6-[1(R)-hydroxyethyl]-2-carbapenem-3-carboxylic acid (Carbapenem Compound No. 6) were dissolved in this solution, to give a total volume of 100 ml.

EXAMPLE 2

5 g of N-benzoyl-β-alanine (Amino Acid Compound No. 2-22) were weighed out and dispersed in 40 ml of water. A 1N aqueous solution of sodium hydroxide was slowly added to this dispersion, and dissolved the N-benzoyl-β-alanine when the pH of the solution reached a value of 7-8. Then, 5 g of (5R,6S)-2-[3S)-1-acetimidoylpyrrolidin-3-ylthio]-6-[1(R)-hydroxyethyl]-2-carbapenem-3-carboxylic acid (Carbapenem Compound No. 6) were dissolved in this solution, to give a total volume of 50 ml.

EXAMPLE 3

Similar procedures to those described in Examples 1 and 2 were carried out, using the other penem or carbapenem antibiotic substances and the other acylated amino acid derivatives shown in the following Table, in the amounts shown in that Table.

In this Table, where the amino acid derivatives are not specified as being D- or L-, then they are the DL-form.

ACTIVITY TESTS

The preparation obtained by the procedure described in Example 1 was injected into the ear vein of a rabbit (about 3 kg body weight) in an amount of 3 ml/kg [that is 150 mg/kg of the Carbapenem Compound No.6 + 150 mg/kg of N-benzoylvaline (Amino Acid Compound No. 10-45)]. A preparation which had been obtained by the same procedure as that described in Example 1 but not including the N-benzoylvaline was injected into another rabbit, as a control, in a similar manner to the above.

After one week, the kidneys of both rabbits were excised and examined. A change was observed in the renal tissue of the rabbits to which had been administered the preparation without the N-benzoylvaline, but no such change at all was observed in the renal tissue of rabbits to which had been administered the preparation containing the N-benzoylvaline.

Similar experiments were carried out on other preparations which were prepared from other penem or carbapenem antibiotic substances and other amino acid derivatives. The Table also shows the results of these experiments.

The observed change in the renal tissue of the control rabbits was a degenerative necrosis of the proximal renal tubules in the region of the renal cortex. In the following Table, where the proportion of the total area of this region which exhibited such necrosis was from 0 to 25%, this is shown as + + +. Where the area is less than 50% this is shown as + + and where it is less than 75% this is shown as +. Where different animals within each test group exhibited different responses, this is shown as e.g. + + + - + + or + + - +.

It should be noted that, whenever penem or carbapenem antibiotic substances which were not combined with amino acid derivatives were administered, a change in the renal tissue was observed. Hence, all of the experiments carried out (even where the effect is only "+") demonstrated a significant protective effect of the amino acid derivative.

Table

| (Carba)-Penem Cpd. No. | Amount mg/kg | Amino Acid Cpd. No. | Amount mg/kg | Effect |
|---|---|---|---|---|
| 6 | 150 | 1-5 | 150 | + |
| 6 | 150 | 1-8 | 150 | ++ |
| 6 | 150 | 1-10 | 150 | + |
| 6 | 150 | 1-11 | 150 | ++ |
| 6 | 150 | 1-12 | 150 | + |
| 6 | 150 | 2-3 | 150 | ++ |
| 6 | 150 | 2-4 | 150 | ++ |
| 6 | 150 | 2-5 | 150 | +++ |
| 6 | 250 | 2-6 | 250 | +++ |
| 6 | 150 | 2-7 | 150 | +++ |
| 6 | 150 | 2-10 | 150 | ++ |
| 6 | 150 | 2-11 | 150 | ++ |
| 6 | 150 | 2-13 | 150 | ++ |
| 6 | 150 | 2-14 | 150 | ++ |
| 6 | 150 | 2-15 | 150 | ++ |

Table (cont)

| (Carba)-penem Cpd. No. | Amount mg/kg | Amino Acid Cpd. No. | Amount mg/kg | Effect |
|---|---|---|---|---|
| 6 | 250 | 2-18 | 250 | +++ |
| 6 | 150 | 2-22 | 150 | +++ |
| 6 | 200 | 2-22 | 200 | +++ |
| 6 | 300 | 2-22 | 300 | +++ |
| 6 | 300 | 2-22 | 150 | ++ |
| 6 | 400 | 3-3 | 400 | +++ |
| 6 | 150 | 3-6 | 150 | + |
| 6 | 150 | 3-8 | 150 | + |
| 6 | 150 | 3-15 | 150 | + |
| 6 | 150 | 4-1 | 150 | + |
| 6 | 150 | 4-4 | 150 | + |
| 6 | 150 | 4-5 | 150 | ++ |
| 5 | 150 | 4-6 | 150 | +++ |
| 6 | 150 | 5-3 | 150 | ++ |
| 6 | 150 | 5-11 | 150 | ++ |
| 6 | 150 | 5-14 | 150 | + |
| 6 | 250 | 5-18 | 250 | ++ |
| 6 | 150 | 5-33 | 150 | +++ |
| 6 | 150 | 6-2 | 150 | ++ |
| 6 | 150 | 6-3 | 150 | +++ |
| 6 | 150 | 6-11 | 150 | ++ |
| 6 | 150 | 7-2 | 150 | + |

Table (cont)

| (Carba)-Penem Cpd. No. | Amount mg/kg | Amino Acid Cpd. No. | Amount mg/kg | Effect |
|---|---|---|---|---|
| 6 | 150 | 7-3 | 150 | +++ |
| 6 | 150 | 7-5 | 150 | +++ |
| 6 | 150 | 7-12 | 150 | ++ |
| 6 | 150 | 7-18 | 150 | ++ |
| 6 | 150 | 8-2 | 150 | + |
| 6 | 150 | 8-4 | 150 | +++ |
| 6 | 150 | 8-8 | 150 | ++ |
| 6 | 150 | 8-10 | 150 | + |
| 6 | 150 | 9-3 | 150 | +++ |
| 6 | 150 | 9-5 | 150 | + |
| 6 | 150 | 10-4 | 150 | +++ |
| 6 | 250 | 10-19 | 250 | ++ |
| 6 | 150 | 10-24 | 150 | + |
| 6 | 150 | 10-30 | 150 | + |
| 6 | 150 | 10-32 | 150 | + |
| 6 | 150 | 10-33 | 150 | + |
| 6 | 150 | 10-34 | 150 | ++ |
| 6 | 150 | 10-35 | 150 | ++ |
| 6 | 150 | 10-45 | 150 | +++ |
| 6 | 400 | 10-45 | 400 | +++ |
| 6 | 400 | 10-45 | 200 | ++ |
| 6 | 150 | 11-3 | 150 | +++ |

Table (cont)

| (Carba)-Penem Cpd. No. | Amount mg/kg | Amino Acid Cpd. No. | Amount mg/kg | Effect |
|---|---|---|---|---|
| 6 | 150 | 11-6 | 150 | + |
| 6 | 150 | 11-7 | 150 | + |
| 6 | 150 | 11-8 | 150 | + |
| 6 | 150 | 11-24 | 150 | +++ |
| 6 | 150 | 12-1 | 150 | ++ |
| 6 | 150 | 12-4 | 150 | ++ |
| 6 | 150 | 13-1 | 150 | ++ |
| 6 | 150 | 13-2 | 150 | ++ |
| 6 | 150 | 13-5 | 150 | +++ |
| 6 | 150 | 13-12 | 150 | + |
| 6 | 150 | 14-6 | 150 | +++ |
| 6 | 150 | 14-7 | 150 | + |
| 6 | 150 | 14-8 | 150 | ++ |
| 6 | 150 | 14-9 | 150 | + |
| 6 | 150 | 14-11 | 150 | + |
| 6 | 150 | 14-14 | 150 | +++ |
| 6 | 150 | 15-1 | 150 | + |
| 6 | 150 | 16-3 | 150 | +++ |
| 6 | 150 | 17-1 | 150 | ++ |
| 6 | 150 | 17-3 | 150 | + |
| 6 | 150 | 17-8 | 150 | + |

Table (cont)

| (Carba)-Penem Cpd. No. | Amount mg/kg | Amino Acid Cpd. No. | Amount mg/kg | Effect |
|---|---|---|---|---|
| 6 | 150 | 18-2 | 150 | + |
| 6 | 150 | 19-2 | 150 | + |
| 6 | 150 | 19-3 | 150 | + |
| 6 | 150 | 21-4 | 150 | + |
| 6 | 150 | 22-6 | 150 | + |
| 6 | 150 | 23-3 | 150 | ++ |
| 6 | 150 | 24-2 | 150 | ++ |
| 6 | 150 | 25-2 | 150 | + |
| 6 | 150 | 26-1 | 150 | + |
| 6 | 150 | 27-3 | 150 | + |
| 6 | 150 | 30-9 | 150 | + |
| 6 | 150 | 31-5 | 150 | + |
| 6 | 400 | 33-4 | 400 | +++ |
| 6 | 150 | 33-5 | 150 | ++ |
| 6 | 150 | 33-7 | 150 | + |
| 6 | 150 | 33-11 | 150 | +++ |
| 6 | 150 | 33-14 | 150 | ++ |
| 6 | 150 | 33-15 | 150 | + |
| 6 | 150 | 33-16 | 150 | + |
| 6 | 150 | 33-20 | 150 | ++ |
| 6 | 150 | 34-2 | 150 | +++ |

Table (cont)

| (Carba)-Penem Cpd. No. | Amount mg/kg | Amino Acid Cpd. No. | Amount mg/kg | Effect |
|---|---|---|---|---|
| 6 | 150 | 34-3 | 150 | ++ |
| 6 | 150 | 34-6 | 150 | + |
| 6 | 150 | 34-8 | 150 | + |
| 6 | 150 | 34-12 | 150 | ++ |
| 6 | 150 | 34-13 | 150 | ++ |
| 6 | 150 | 34-14 | 150 | +++ |
| 6 | 150 | 35-3 | 150 | + |
| 6 | 150 | 36-3 | 150 | ++ |
| 6 | 150 | 37-3 | 150 | ++ |
| 6 | 150 | 39-2 | 150 | ++ |
| 6 | 150 | 41-2 | 150 | + |
| 6 | 150 | 41-4 | 150 | ++ |
| 6 | 150 | 41-7 | 150 | + |
| 6 | 150 | 42-2 | 150 | ++ |
| 6 | 150 | 43-1 | 150 | + |
| 6 | 150 | 43-3 | 150 | +++ |
| 6 | 150 | 43-5 | 150 | + |
| 6 | 150 | 43-7 | 150 | ++ |
| 6 | 150 | 43-8 | 150 | ++ |
| 6 | 150 | 44-3 | 150 | ++ |
| 6 | 150 | 45-5 | 150 | + |

Table (cont)

| (Carba)-Penem Cpd. No. | Amount mg/kg | Amino Acid Cpd. No. | Amount mg/kg | Effect |
|---|---|---|---|---|
| 6 | 150 | 46-1 | 150 | ++ |
| 6 | 150 | 47-3 | 150 | ++ |
| 6 | 150 | 48-2 | 150 | + |
| 6 | 150 | 48-5 | 150 | ++ |
| 6 | 150 | 48-9 | 150 | + |
| 6 | 150 | 49-2 | 150 | + |
| 6 | 150 | 49-7 | 150 | + |
| 6 | 400 | 50-5 | 400 | +++ |
| 6 | 150 | 50-6 | 150 | +++ |
| 6 | 150 | 50-7 | 150 | +++ |
| 6 | 150 | 50-10 | 150 | ++ |
| 6 | 150 | 50-12 | 150 | ++ |
| 6 | 150 | 51-1 | 150 | ++ |
| 6 | 150 | 52-6 | 150 | ++ |
| 6 | 150 | 53-1 | 150 | ++ |
| 6 | 150 | 54-1 | 150 | ++ |
| 6 | 150 | 55-3 | 150 | ++ |
| 6 | 150 | 56-3 | 150 | ++ |
| 6 | 150 | 57-3 | 150 | +++ |
| 6 | 150 | 57-4 | 150 | ++ |
| 6 | 150 | 57-6 | 150 | + |

Table (cont)

| (Carba)-Penem Cpd. No. | Amount mg/kg | Amino Acid Cpd. No. | Amount mg/kg | Effect |
|---|---|---|---|---|
| 6 | 150 | 57-7 | 150 | + |
| 6 | 150 | 58-1 | 150 | + |
| 6 | 150 | 58-3 | 150 | ++ |
| 6 | 150 | 58-6 | 150 | + |
| 6 | 150 | 58-9 | 150 | + |
| 6 | 150 | 59-1 | 150 | +++ |
| 6 | 150 | 59-4 | 150 | ++ |
| 6 | 150 | 59-7 | 150 | ++ |
| 6 | 150 | 60-2 | 150 | + |
| 6 | 150 | 60-6 | 150 | + |
| 6 | 150 | 61-2 | 150 | ++ |
| 1 | 150 | 2-22 | 150 | ++ |
| 1 | 150 | 3-3 | 150 | ++ |
| 1 | 250 | 5-11 | 250 | +++ |
| 1 | 150 | 5-18 | 150 | +++ |
| 1 | 150 | 10-45 | 150 | ++ |
| 1 | 150 | 14-6 | 150 | +++ |
| 1 | 150 | 33-4 | 150 | +++ |
| 1 | 150 | 34-10 | 150 | ++ |
| 1 | 150 | 50-5 | 150 | ++ |
| 2 | 150 | 4-6 | 150 | +++ |

Table (cont)

| (Carba)-Penem Cpd. No. | Amount mg/kg | Amino Acid Cpd. No. | Amount mg/kg | Effect |
|---|---|---|---|---|
| 2 | 150 | 5-11 | 150 | +++ |
| 2 | 150 | 7-14 | 150 | +++ |
| 2 | 250 | 9-3 | 250 | +++ |
| 2 | 150 | 10-23 | 150 | ++ |
| 2 | 150 | 33-15 | 150 | ++ |
| 2 | 250 | 50-5 | 250 | ++ |
| 2 | 150 | 50-23 | 150 | ++ |
| 3 | 150 | 5-11 | 150 | +++ |
| 3 | 250 | 10-29 | 250 | +++ |
| 3 | 250 | 13-5 | 250 | ++ |
| 3 | 150 | 13-13 | 150 | ++ |
| 3 | 150 | 33-20 | 150 | ++ |
| 3 | 250 | 34-2 | 250 | ++ |
| 3 | 150 | 50-12 | 150 | ++ |
| 7 | 150 | 2-22 | 150 | +++ |
| 7 | 250 | 5-11 | 250 | ++ |
| 7 | 150 | 10-45 | 150 | +++ |
| 7 | 150 | 14-1 | 150 | +++ |
| 7 | 150 | 50-6 | 150 | +++ |
| 8 | 150 | 2-22 | 150 | ++ |
| 8 | 150 | 6-11 | 150 | +-++ |

Table (cont)

| (Carba)-Penem Cpd. No. | Amount mg/kg | Amino Acid Cpd. No. | Amount mg/kg | Effect |
|---|---|---|---|---|
| 8 | 250 | 50-5 | 250 | ++-+++ |
| 9 | 150 | 33-6 | 150 | + |
| 9 | 250 | 50-5 | 250 | ++ |
| 10 | 150 | 13-5 | 150 | ++ |
| 10 | 150 | 18-6 | 150 | + |
| 11 | 150 | 2-22 | 150 | ++ |
| 15 | 150 | 10-45 | 150 | +++ |
| 19 | 150 | L-10-45 | 150 | ++ |
| 22 | 250 | 33-4 | 250 | ++ |
| 22 | 150 | 34-15 | 150 | +-++ |
| 23 | 150 | 2-22 | 150 | +++ |
| 24 | 150 | 2-22 | 150 | +++ |
| 24 | 150 | 10-45 | 150 | +++ |
| 24 | 150 | D-10-45 | 150 | +++ |
| 24 | 150 | L-10-45 | 150 | +++ |
| 24 | 150 | 18-5 | 150 | + |
| 25 | 150 | 9-3 | 150 | ++ |

Table (cont)

| (Carba)-Penem Cpd. No. | Amount mg/kg | Amino Acid Cpd. No. | Amount mg/kg | Effect |
|---|---|---|---|---|
| 28 | 150 | 3-18 | 150 | + |
| 28 | 150 | 33-21 | 150 | +++ |
| 32 | 150 | 2-22 | 150 | ++ |
| 38 | 150 | 2-22 | 150 | +++ |
| 39 | 150 | 10-45 | 150 | ++ |
| 40 | 150 | 2-22 | 150 | +++ |
| 66 | 250 | 3-3 | 250 | ++ |
| 67 | 150 | 5-19 | 150 | ++ |
| 67 | 150 | 12-4 | 150 | +-++ |
| 71 | 250 | 2-18 | 250 | +++ |
| 71 | 250 | 5-21 | 250 | ++ |
| 73 | 150 | 11-27 | 150 | + |
| 73 | 150 | 34-10 | 150 | ++ |
| 75 | 150 | 14-6 | 150 | ++ |
| 75 | 150 | 33-9 | 150 | ++ |

**Claims**

**Claims for the following Contracting States : GB, DE, FR, IT, CH, BE, NL, SE, LU**

**1.** A composition comprising:

a penem or carbapenem antibiotic; and

a pharmaceutically acceptable N-acylated derivative of an amino acid wherein the amino group and the carboxylic acid group are attached to a saturated aliphatic carbon chain or carbon atom, or a salt thereof, provided that, where the composition comprises only one amino acid derivative, the amino acid is not ornithine, lysine, phenylalanine or phenylglycine, the amount of amino acid derivative being sufficient to alleviate any renal toxicity or the antibiotic, preferably in a weight ratio of said N-acylated amino acid to said antibiotic of from 0.1:1 to 4:1.

2. A composition as claimed in Claim 1, wherein said amino acid is a compound of general formula II:

$H_2N-X-COOH$ (II)

wherein X represents a $C_1$-$C_{10}$ alkylene group or a $C_1$-$C_{10}$ alkylene group having at least one substituent selected from hydroxy groups, $C_1$-$C_4$ alkoxy groups, $C_6$-$C_{14}$ aryloxy groups, substituted $C_6$-$C_{14}$ aryloxy groups, $C_7$-$C_9$ aralkyloxy groups, substituted $C_7$-$C_9$ aralkyloxy groups, mercapto groups, $C_1$-$c_4$ alkylthio groups, $C_6$-$C_{14}$ arylthio groups, substituted $C_6$-$C_{14}$ arylthio groups, $C_7$-$C_9$ aralkylthio groups, substituted $C_7$-$C_9$ aralkylthio groups, $C_2$-$C_5$ carboxyalkylthio groups, amino groups, amino groups having one or two substituents selected from

$C_1$-$C_4$ alkyl groups, $C_6$-$C_{14}$ aryl groups, substituted $C_6$-$C_{14}$ aryl groups, $C_7$-$C_9$ aralkyl groups, substituted $C_7$-$C_9$ aralkyl groups and carboxylic acyl groups,

$C_6$-$C_{14}$ aryl groups, substituted $C_6$-$C_{14}$ aryl groups, carboxy groups, amidino groups, sulpho groups, $C_1$-$C_4$ alkylsulphinyl groups, $C_1$-$C_4$ alkylsulphonyl groups and heterocyclic groups having from 5 to 14 ring atoms of which from 1 to 5 are nitrogen and/or oxygen and/or sulphur hetero-atoms, said substituted aryloxy, arylthio, aralkylthio, aryl and aralkyl groups having at least one substituent selected from $C_1$-$C_4$ alkyl groups, hydroxy groups amino groups and $C_1$-$C_4$ alkoxy groups,

or a pharmaceutically acceptable salt thereof.

3. A composition as claimed in Claim 2, wherein X represents a $C_1$-$C_5$ alkylene group which is unsubstituted or has one or two substituents selected from: hydroxy groups; $C_1$-$C_4$ alkoxy groups; aryloxy groups wherein the aryl ring has from 6 to 14 ring carbon atoms and which is unsubstituted or has from 1 to 3 substituents selected from $C_1$-$C_4$ alkyl groups, hydroxy groups, amino groups and $C_1$-$C_4$ alkoxy groups; $C_7$-$C_9$ aralkyloxy groups, wherein the aryl moiety is unsubstituted or has from 1 to 3 substituents selected from $C_1$-$C_4$ alkyl groups, hydroxy groups, amino groups and $C_1$-$C_4$ alkoxy groups; mercapto groups; $C_1$-$C_4$ alkylthio groups; arylthio groups wherein the aryl ring has from 6 to 14 ring carbon atoms and which is unsubstituted or has from 1 to 3 substituents selected from $C_1$-$C_4$ alkyl groups, hydroxy groups, amino groups and $C_1$-$C_4$ alkoxy groups; $C_7$-$C_9$ aralkylthio groups wherein the aryl ring is unsubstituted or has from 1 to 3 substituents selected from $C_1$-$C_4$ alkyl groups, hydroxy groups, amino groups and $C_1$-$C_4$ alkoxy groups;
carboxyalkylthio groups in which the alkyl part has from 1 to 4 carbon atoms; amino groups; amino groups having one or two $C_1$-$C_4$ alkyl substituents; amino groups having one or two aryl substituents in which the aryl ring has from 6 to 14 ring carbon atoms and is unsubstituted or has from 1 to 3 substituents selected from $C_1$-$C_4$ alkyl groups, hydroxy groups, amino groups and $C_1$-$C_4$ alkoxy groups; amino groups having one or two $C_7$-$C_9$ aralkyl substituents in which the aryl part is unsubstituted or has from 1 to 3 substituents selected from $C_1$-$C_4$ alkyl groups, hydroxy groups, amino groups and $C_1$-$C_4$ alkoxy groups; amino groups having one or two carboxylic acyl substituents; aryl groups having from 6 to 14 ring carbon atoms and being unsubstituted or having from 1 to 3 substituents selected from $C_1$-$C_4$ alkyl groups, hydroxy groups, amino groups and $C_1$-$C_4$ alkoxy groups; carboxy groups; and heterocyclic groups having from 5 to 9 ring atoms, of which from 1 to 3 are nitrogen and/or oxygen and/or sulphur hetero-atoms.

4. A composition as claimed in Claim 2, wherein X represents a $C_1$-$C_5$ alkylene group which is unsubstituted or has 1 or 2 substituents selected from: hydroxy groups; $C_1$-$C_4$ alkoxy groups; mercapto groups; $C_1$-$C_4$ alkylthio groups; amino groups; amino groups having one or two $C_1$-$C_4$ alkyl substituents; amino groups having one or two carboxylic acyl substituents; aryl groups having from 6 to 14 carbon atoms wherein the aryl ring is unsubstituted or has from 1 to 3 substituents selected from $C_1$-$C_4$ alkyl groups, hydroxy groups, amino groups and $C_1$-$C_4$ alkoxy groups; carboxy groups; and heterocyclic groups having from 5 to 9 ring atoms, of which from 1 to 3 are nitrogen and/or oxygen hetero-atoms.

5. A composition as claimed in any one of Claims 1 to 4, wherein the N-acyl group is: a $C_1$-$C_{18}$ alkanoyl group; a $C_3$-$C_8$ alkenoyl group; a $C_3$-$C_8$ alkynoyl group; an aromatic acyl group wherein the aryl part is $C_6$-$C_{14}$ carbocyclic aryl and is unsubstituted or has from 1 to 5 substituents selected from $C_1$-$C_4$ alkyl groups, hydroxy groups, $C_1$-$C_4$ alkoxy groups, amino groups, sulpho groups and halogen atoms; a

cycloalkanecarbcnyl group where the cycloalkane part is $C_3$-$C_8$ and is unsubstituted or has at least one substituent selected from $C_1$-$C_4$ alkyl groups and phenyl groups; an araliphatic acyl group in which the aryl ring is a carbocyclic ring having from 6 to 14 carbon atoms and which is unsubstituted or has from 1 to 5 substituents selected from $C_1$-$C_4$ alkyl groups, hydroxy groups, $C_1$-$C_4$ alkoxy groups, amino groups, sulpho groups and halogen atoms, and in which the alkyl moiety has from 1 to 4 carbon atoms; a heterocyclic acyl group which has a saturated or unsaturated ring system, the rings having 5 or 6 ring atoms, of which from 1 to 3 are nitrogen and/or sulphur and/or oxygen hetero-atoms and the ring being unsubstituted or having from 1 to 3 substituents selected from $C_1$-$C_4$ alkyl groups and hydroxy groups; a $C_2$-$C_7$ alkoxycarbonyl group; an aralkyloxycarbonyl group where the aralkyl part has from 7 to 9 carbon atoms and is unsubstituted or has from 1 to 5 substituents selected from amino groups, $C_1$-$C_4$ alkyl groups, $C_1$-$C_4$ alkoxy groups and hydroxy groups; or an acyl group derived from an amino acid by removal of OH from the carboxylic acid group and N-acylation of the amino group with at least one of the above-mentioned acyl groups.

6. A composition as claimed in any one of Claims 1 to 4, wherein the N-acyl group is: a saturated aliphatic acyl group having from 1 to 8 carbon atoms; an aromatic acyl group in which the aryl moiety has from 6 to 10 ring carbon atoms and is unsubstituted or has from 1 to 3 substituents selected from $C_1$-$C_4$ alkyl groups and $C_1$-$C_4$ alkoxy groups; an alicyclic acyl group in which the cycloalkane ring has from 3 to 6 carbon atoms; an araliphatic acyl group in which the aryl ring has from 6 to 10 ring carbon atoms and the alkyl group has from 1 to 4 carbon atoms, the aryl ring being unsubstituted or having from 1 to 3 substituents selected from $C_1$-$C_4$ alkyl groups and $C_1$-$C_4$ alkoxy groups; a heterocyclic acyl group in which the heterocyclic ring is saturated or unsaturated and has 5 or 6 ring atoms of which one is a nitrogen, sulphur or oxygen hetero-atom; an alkoxycarbonyl group having a total of from 2 to 7 carbon atoms; an aralkyloxycarbonyl group in which the aralkyl moiety has from 7 to 9 carbon atoms and the aryl ring is unsubstituted or has from 1 to 3 substituents selected $C_1$-$C_4$ alkyl groups and $C_1$-$C_4$ alkoxy groups; or an acyl group derived from an amino acid by removal of OH from the carboxylic acid group and N-acylation of the amino group with at least one of the above-mentioned acyl groups.

7. A composition as claimed in any; one of Claims 1 to 4, wherein the N-acyl group is: an aromatic acyl group in which the aryl ring has from 6 to 10 ring atoms and which is unsubstituted or has a single substituent selected from $C_1$-$C_4$ alkyl groups, $C_1$-$C_4$ alkoxy groups, hydroxy groups and amino groups; an alicyclic acyl group in which the cycloalkane moiety has from 3 to 6 carbon atoms; a phenylaliphatic acyl group in which the phenyl group is unsubstituted or has a single $C_1$-$C_4$ alkyl substituent, and in which the alkyl part has from 1 to 4 carbon atoms; an alkoxycarbonyl group having a total of from 4 to 6 carbon atoms; an aralkyloxycarbonyl group in which the aralkyl part has from 7 to 9 carbon atoms and has 0 to 1 substituent selected from $C_1$-$C_4$ alkyl groups and $C_1$-$C_4$ alkoxy groups; or an acyl group derived from an amino acid by removal of OH from the carboxylic acid group and N-acylation of the amino group with at least one of the above-mentioned acyl groups.

8. A composition as claimed in any one of Claims 1 to 4 wherein the N-acyl group is an acetyl, benzoyl, cyclohexanecarbonyl, cyclopropanecarbonyl, hexanoyl, isobutyryl, crotonoyl, ethoxycarbonyl, 4-hydroxybenzoyl, anisoyl, 4-aminobenzoyl, naphthoyl, toluoyl, benzyloxycarbonyl or 4-methoxybenzyloxycarbonyl group.

9. A composition as claimed in any one of Claims 1 and 5 to 8, wherein said amino acid is glycine, β-alanine, 4-aminobutyric acid, 5-aminovaleric acid, 6-aminohexanoic acid, 8-aminooctanoic acid, alanine, 2-aminobutyric acid, norvaline valine, leucine, isoleucine, norleucine, tyrosine, O-methyltyrosine, aspartic acid, glutamic acid, 4-carboxyglutamic acid, 3-methylaspartic acid, 2-aminoadipic acid, 2-aminopimelic acid, 2-aminosuberic acid, 3-hydroxyaspartic acid, 3-hydroxyglutamic acid, 2,3-diaminopropionic acid, 2,4-diaminobutyric acid, 5-hydroxylysine, arginine, $N^{\delta}$,$N^{\delta}$-dimethylornithine, $N^{\epsilon}$-methyllysine, cysteine, methionine, ethionine,S-carboxymethylcysteine, S-benzylcysteine, methionine S-oxide, ethionine S-oxide, methionine S,S-dioxide, cysteic acid, serine, O-methylserine, threonine, O-methylthreonine, homothreonine, ethoxinine, 3-methoxyvaline, 3-phenylserine, 3-methyl-3-phenylalanine, histidine, tryptophan, 2-methylalanine, 2-methylserine, 2-hydroxyisoleucine, 2-methylmethionine, 2-ethyl-2-phenylglycine, 3-aminobutyric acid, 3-amino-4-methylvaleric acid, 3-amino-3-phenylpropionic acid, 3-amino-2-hydroxypropionic acid or 4-amino-3-hydroxybutyric acid.

10. A composition as claimed in any one of Claims 1 and 5 to 8, wherein said amino acid is glycine, β-alanine, 4-aminobutyric acid, 5-aminovaleric acid, 6-aminohexanoic acid, 8-aminooctanoic acid, alanine, norvaline, valine, leucine, isoleucine, norleucine, $N^{\delta}$,$N^{\delta}$-dimethylornithine, methionine, ethionine, O-methylserine, O-methylthreonine, ethoxinine, 3-methoxyvaline, 3-phenylserine, 3-methyl-3-phenylalanine, histidine, 2-methylalanine, 2-methylserine, 2-hydroxyisoleucine, 2-ethylphenylglycine, 3-aminobutyric acid, 3-amino-4-methylvaleric acid or 3-amino-3-phenylpropionic acid.

**11.** A composition as claimed in any one of Claims 1 and 5 to 8, wherein said amino acid is β-alanine, 4-aminobutyric acid, 5-aminovaleric acid, 6-aminohexanoic acid, alanine, valine, leucine, norleucine, methionine, histidine or glycine.

**12.** A composition as claimed in any one of Claims 1 and 5 to 8, wherein said amino acid is leucylglycine, glycyl-β-alanine, glycylalanine, valylalanine, leucylalanine, glycylvaline, alanylvaline, leucylvaline, valylleucine, phenylalanylleucine, histidylleucine, glycylphenylalanine, alanylphenylalanine, leucylphenylalanine, glycylmethionine, valylmethionine, glycylhistidine, alanylvalylglycine, glycylalanylvaline, glycylphenylalanylleucine or glycylglycylhistidine.

**13.** A composition as claimed in Claim 1, wherein said N-acylated amino acid is:

N-(p-toluoyl)-β-alanine
N-(4-methoxybenzoyl)-β-alanine
N-(3-hydroxy-2-naphthoyl)-β-alanine
N-benzoylglycyl-β-alanine
N-benzoyl-β-alanine
N-benzoyl-5-aminovaleric acid
N-benzoyl-6-aminohexanoic acid
N-cyclohexanecarbonyl-6-aminohexanoic acid
N-(N-methylnicotinoyl)-6-aminohexanoic acid
N-benzoyl-8-aminooctanoic acid
N-benzoylalanine
N-(1-naphthoyl)alanine
N-benzoylvalylalanine
N-benzoyl-2-aminobutyric acid
N-benzoylnorvaline
N-valerylvaline
N-benzoylalanylvaline
N-benzoylvaline
N-benzoylleucine
N-benzoylglycylphenylalanylleucine
N-benzoylnorleucine
N-benzoylglycylphenylalanine
N-benzoylalanylphenylalanine
N-cyclohexanecarbonylleucylphenylalanine
N-benzoyl-O-methyltyrosine
N-benzoylmethionine
N-phenylacetylmethionine
N-benzoylvalylmethionine
N-benzoylethionine
N-(4-methoxybenzyloxycarbonyl)ethionine
N-benzoylthreonine
N-benzoylhistidine
N-(p-toluoyl)histidine
N-(4-methoxybenzoyl)histidine
N-(4-methoxybenzoyl)-3-aminobutyric acid
or
N-butyryl-3-amino-3-phenylpropionic acid.

**14.** A composition as claimed in any one of the preceding Claims, wherein said antibiotic is a compound of general formula I:

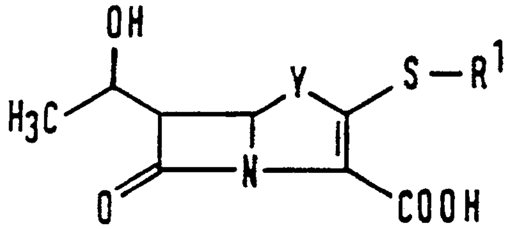

(I)

in which:

Y represents a sulphur atom, a methylene group or a methylene group having 1 or 2 methyl and/or methoxy substituents; and

$R^1$ represents a $C_1$-$C_6$ alkyl group, a $C_1$-$C_6$ alkyl group having at least one of substituents (i) or a heterocyclic group having from 4 to 14 ring atoms of which from 1 to 5 are nitrogen and/or oxygen and/or sulphur hetero-atoms where said heterocyclic group is unsubstituted or has at least one of substituents (ii);

substituents (i):
halogen atoms, amino groups, amino groups having at least one of substituents (iii), $C_1$-$C_4$ alkylideneamino groups, $C_1$-$C_4$ aminoalkylideneamino groups, amidino groups, amidino groups having from 1 to 3 of substituents (iii), heterocyclic groups having from 4 to 14 ring atoms of which from 1 to 5 are nitrogen and/or oxygen and/or sulphur hetero-atoms wherein said heterocyclic group is unsubstituted or has at least one of substituents (ii), imino groups, cyano groups, carbamoyl groups and carbamoyl groups having at least one substituent selected from $C_1$-$C_4$ alkyl groups and $C_1$-$C_4$ alkoxy groups;
substituents (ii):
$C_1$-$C_6$ alkanimidoyl groups, $C_1$-$C_6$ alkyl groups, alkoxyalkyl groups where the alkoxy and alkyl parts are each $C_1$-$C_4$, carbamoyl groups, carbamoyl groups having at least one substituent selected from $C_1$-$C_4$ alkyl groups and $C_1$-$C_4$ alkoxy groups, $C_1$-$C_4$ haloalkyl groups, heterocyclic acylimidoyl groups where the heterocyclic part has from 5 to 9 ring atoms of which from 1 to 3 are nitrogen and/or oxygen and/or sulphur hetero-atoms, amidino groups, amidino groups having from 1 to 3 of substituents (iii), imino groups, oxygen atoms, $C_1$-$C_6$ alkanoyl groups, $C_1$-$C_6$ alkanesulphonyl groups, $C_1$-$C_6$ alkanesulphinyl groups, hydroximino groups, $C_1$-$C_4$ alkoximino groups, carbamoyloxy groups, carbamoyloxy groups having at least one substituent selected from $C_1$-$C_4$ alkyl groups and $C_1$-$C_4$ alkoxy groups, carbamoyloxylalkyl groups where the alkyl part is $C_1$-$C_4$ and the carbamoyl part is unsubstituted or has at least one substituent selected from $C_1$-$C_4$ alkyl groups and $C_1$-$C_4$ alkoxy groups and $C_1$-$C_4$ iminoalkyl groups;
substituents (iii):
$C_1$-$C_6$ alkyl groups, $C_2$-$C_6$ alkenyl groups, $C_2$-$C_6$ alkynyl groups, and said alkyl, alkenyl and alkynyl groups having at least one substituent selected from halogen atoms, carbamoyloxy groups and carbamoyloxy groups having at least one substituent selected from $C_1$-$C_4$ alkyl groups and $C_1$-$C_4$ alkoxy groups;

and pharmaceutically acceptable salts thereof.

15. A composition as claimed in Claim 14, wherein Y represents a sulphur atom, a methylene group, or the group $CH_3$-CH<, $CH_3O$-CH< or $(CH_3)_2C$<.

16. A composition as claimed in Claim 14 or Claim 15, wherein $R^1$ represents an ethyl, 2-fluoroethyl, 2-(aminomethyleneamino)ethyl, $N^1,N^1$-dimethylamidinomethyl, $N^1,N^1,N^2$-trimethylamidinomethyl, 3-pyrrolidinyl, 1-formimidoyl-3-pyrrolidinyl, 1-acetimidoyl-3-pyrrolidinyl, 1-propionimidoyl-3-pyrrolidinyl, 2-methyl-1,4,5,6-tetrahydro-5-pyrimidinyl, 2-methoxymethyl-1,4,5,6-tetrahydro-5-pyrimidinyl, 3-azetidinyl, 1-acetimidoyl-3-azetidinyl, $N^1$-methyl-$N^1$-(2-propynyl)amidinomethyl, $N^1$-(2-fluoroethyl)-$N^1$-methylamidinomethyl, $N^1$-(3-fluoropropyl)-$N^1$-methylamidinomethyl, $N^1$-methyl-$N^1$-(2,2,2-trifluoroethyl)-

amidinomethyl,1-(3-azetidinyl)ethyl, 1-(1-acetimidoyl-3-azetidinyl)ethyl, 1,4,5,6-tetrahydro-2-pyrimidinylmethyl, 1-(4,5-dihydro-2-thiazolyl)ethyl, 5-carbamoyl-3-pyrrolidinyl, 1-acetimidoyl-5-carbamoyl-3-pyrrolidinyl,2-chloromethyl-1,4,5,6-tetrahydro-5-pyrimidinyl, 1-butyrimidoyl-3-pyrrolidinyl, 1-nicotinimidoyl-3-pyrrolidinyl, $N^1$,$N^1$-diallylamidinomethyl, $N^1$-methyl-$N^1$-(2-propynyl)amidino, $N^1$-(2-fluoroethyl)-$N^1$-methylamidino, $N^1$-(3-fluoropropyl)-$N^1$-methylamidino, $N^1$-methyl-$N^1$-(2,2,2-trifluoroethyl)-amidino, $N^1$-allyl-$N^1$-methylamidinomethyl, cyanomethyl, 2-cyanoethyl, 1-cyanoethyl, 2-cyano-1-methylethyl, 2-aminoethyl, 1-carbamoylethyl, 2-(1-aminoethylideneamino)ethyl, 1-amidino-3-pyrrolidinyl, 2-methyl-1,3-diazabicyclo-[3.3.0]oct-2-en-7-yl, 2-methoxymethyl-1,3-diazabicyclo[3.3.0]oct-2-en-7-yl, 5-imino-2-pyrrolidinyl, 2-imino-5-piperidinyl, 1-acetimidoyl-5-methylcarbamoyl-3-pyrrolidinyl, 1-acetimidoyl-5-methoxycarbamoyl-3-pyrrolidinyl, 2-imino-2-(S-oxothiomorpholino)ethyl, 2-imino-2-(1,1-dioxo-1,3-thiazolidin-3-yl)ethyl, 2-imino-2-(S,S-dioxothiomorpholino)ethyl, 2-imino-2-(3,5-dioxo-1-piperazinyl)ethyl, 2-imino-2-(4-methyl-3,5-dioxo-1-piperazinyl)ethyl, 2-imino-2-(-3-oxo-1-piperazinyl)-ethyl, 2-imino-2-(4-methyl-3-oxo-1-piperazinyl)ethyl, 2-imino-2-(4-acetyl-3-oxo-1-piperazinyl)ethyl, 2-imino-2-(4-methanesulphonyl-3-oxo-1-piperazinyl)ethyl, $N^1$-(2-carbamoyloxyethyl)-$N^1$-methylamidinomethyl, 2-(3-hydroximino-1-pyrrolidinyl)2-iminoethyl, 2-imino-2-(3-methoximino-1-pyrrolidinyl)ethyl, 2-(4-hydroximinopiperidino)-2-iminoethyl, 2-imino-2-(4-methoximinopiperidino)ethyl, 2-(3-carbamoyloxy-1-pyrrolidinyl)-2-iminoethyl, 2-imino-2-(3-oxo-1-piperazinyl)ethyl, 2-(3-carbamoylpiperidino)-2-iminoethyl, 2-(3-carbamoyloxypiperidino)-2-iminoethyl, 2-(2-carbamoyloxy-1-pyrrolidinyl)-2-iminoethyl, 2-(2-carbamoyloxymethyl-1-pyrrolidinyl)-2-iminoethyl, 2-(4-carbamoyloxypiperidino)-2-iminoethyl, 2-(4-formyl-1-piperazinyl)-2-iminoethyl, 2-(4-acetyl-1-piperazinyl)-2-iminoethyl, 1-formyl-3-azetidinyl, 1-iminomethyl-3-azetidinyl, 1-methyl-4-piperidyl, 1-acetimidoyl-4-piperidyl or 1-acetyl-3-pyrrolidinyl group.

**17.** A composition as claimed in Claim 14, wherein said antibiotic is:

(5R,6S)-2-{2-[(aminomethylene)amino]ethylthio}-6-[1(R)-hydroxyethyl]-2-carbapenem-3-carboxylic acid

(5R,6S)-2[(3S)-1-acetimidoylpyrrolidin-3-ylthio]-6-[1(R)-hydroxyethyl]-2-carbapenem-3-carboxylic acid

(5R,6S)-2-[(3R)-1-acetimidoylpyrrolidin-3-ylthio]-6-[1(R)-hydroxyethyl]-2-carbapenem-3-carboxylic acid

(5R,6S)-2-[(3R)-1-acetimidoylpyrrolidin-3-ylthio]-6-[1(R)-hydroxyethyl]-1(S)-methyl-2-carbapenem-3-carboxylic acid

(5R,6S)-2-[(3S)-1-acetimidoylpyrrolidin-3-ylthio]-6-[1(R)-hydroxyethyl]-1(R)-methyl-2-carbapenem-3-carboxylic acid

(5R,6S)-2-[(3S)-1-acetimidoylpyrrolidin-3-ylthio]-6-[1(R)-hydroxyethyl]-1(S)-methyl-2-carbapenem-3-carboxylic acid
or
(5R,6S)-2-[(3S)-1-acetimidoyl-5(S)-carbamoylpyrrolidin-3-ylthio]-6-[1(R)-hydroxyethyl]-2-carbapenem-3-carboxylic acid.

**18.** A composition as claimed in any one of the preceding Claims, wherein the weight ratio of said N-acylated amino acid to said antibiotic is from 0.1:1 to 4:1.

**19.** A packaged pharmaceutical preparation comprising:
(a) in one part, a penem or carbapenem antibiotic; and
(b) in another part, a pharmaceutically acceptable N-acylated derivative of an amino acid wherein the amino group and the carboxylic acid group are attached to a saturated aliphatic carbon chain or carbon atom, or a salt thereof, provided that the amino acid is not ornithine, lysine, phenylalanine or phenylglycine alone.

**20.** A preparation as claimed in Claim 19, wherein said N-acylated amino acid is as defined in any one of Claims 2 to 13.

**21.** A preparation as claimed in Claim 19 or Claim 20, wherein said antibiotic is as defined in any one of Claims 14 to 17.

**22.** The use for the manufacture of a medicament for the treatment of bacterial infections of:
(a) a penem or carbapenem antibiotic;
in association with:
(b) a pharmaceutically acceptable N-acylated derivative of an amino acid wherein the amino group and the carboxylic acid group are attached to a saturated aliphatic carbon chain or carbon atom, or a salt thereof, provided that the amino acid is not ornithine, lysine, phenylalanine or phenylglycine alone.

**23.** The use as claimed in Claim 22, wherein said N-acylated amino acid is as defined in any one of Claims 2 to 13.

**24.** The use as claimed in Claim 22 or Claim 23, wherein said antibiotic is as defined in any one of Claims 14 to 17.

**Claims for the following contracting States : AT, ES**

**1.** A method of making a pharmaceutical composition by mixing:

a penem or carbapenem antibiotic; and

a pharmaceutically acceptable N-acylated derivative of an amino acid wherein the amino group and the carboxylic acid group are attached to a saturated aliphatic carbon chain or carbon atom, or a salt thereof, provided that, where the composition comprises only one amino acid derivative, the amino acid is not ornithine, lysine, phenylalanine or phenylglycine, the amount of amino acid derivative being sufficient to alleviate any renal toxicity of the antibiotic, preferably in a weight ratio of said N-acylated amino acid to said antibiotic of from 0.1:1 to 4:1.

**2.** A method as claimed in Claim 1, wherein said amino acid is a compound of general formula II:

$H_2N$-X-COOH (II)

wherein X represents a $C_1$-$C_{10}$ alkylene group or a $C_1$-$C_{10}$ alkylene group having at least one substituent selected from hydroxy groups, $C_1$-$C_4$ alkoxy groups, $C_6$-$C_{14}$ aryloxy groups, substituted $C_6$-$C_{14}$ aryloxy groups, $C_7$-$C_9$ aralkyloxy groups, substituted $C_7$-$C_9$ aralkyloxy groups, mercapto groups, $C_1$-$C_4$ alkylthio groups, $C_6$-$C_{14}$ arylthio groups, substituted $C_6$-$C_{14}$ arylthio groups, $C_7$-$C_9$ aralkylthio groups, substituted $C_7$-$C_9$ aralkylthio groups, $C_2$-$C_5$ carboxyalkylthio groups, amino groups, amino groups having one or two substituents selected from

$C_1$-$C_4$ alkyl groups, $C_6$-$C_{14}$ aryl groups, substituted $C_6$-$C_{14}$ aryl groups, $C_7$-$C_9$ aralkyl groups, substituted $C_7$-$C_9$ aralkyl groups and carboxylic acyl groups,

$C_6$-$C_{14}$ aryl groups, substituted $C_6$-$C_{14}$ aryl groups, carboxy groups, amidino groups, sulpho groups, $C_1$-$C_4$ alkylsulphinyl groups, $C_1$-$C_4$ alkylsulphonyl groups and heterocyclic groups having from 5 to 14 ring atoms of which from 1 to 5 are nitrogen and/or oxygen and/or sulphur hetero-atoms, said substituted aryloxy, aralkyloxy, arylthio, aralkylthio, aryl and aralkyl groups having at least one substituent selected from $C_1$-$C_4$ alkyl groups, hydroxy; groups, amino groups and $C_1$-$C_4$ alkoxy groups,

or a pharmaceutically acceptable salt thereof.

**3.** A method as claimed in Claim 2, wherein X represents a $C_1$-$C_5$ alkylene group which is unsubstituted or has one or two substituents selected from: hydroxy groups; $C_1$-$C_4$ alkoxy groups; aryloxy groups wherein the aryl ring has from 6 to 14 ring carbon atoms and which is unsubstituted or has from 1 to 3 substituents selected from $C_1$-$C_4$ alkyl groups, hydroxy groups, amino groups and $C_1$-$C_4$ alkoxy groups; $C_7$-$C_9$ aralkyloxy groups, wherein the aryl moiety is unsubstituted or has from 1 to 3 substituents selected from $C_1$-$C_4$ alkyl groups, hydroxy groups, amino groups and $C_1$-$C_4$ alkoxy groups; mercapto groups; $C_1$-$C_4$ alkylthio groups; arylthio groups wherein the aryl ring has from 6 to 14 ring carbon atoms and which is unsubstituted or has from 1 to 3 substituents selected from $C_1$-$C_4$ alkyl groups, hydroxy groups, amino groups and $C_1$-$C_4$ alkoxy groups; $C_7$-$C_9$ aralkylthio groups wherein the

aryl ring is unsubstituted or has from 1 to 3 substituents selected from $C_1$-$C_4$ alkyl groups, hydroxy groups, amino groups and $C_1$-$C_4$ alkoxy groups; carboxyalkylthio groups in which the alkyl part has from 1 to 4 carbon atoms; amino groups; amino groups having one or two $C_1$-$C_4$ alkyl substituents; amino groups having one or two aryl substituents in which the aryl ring has from 6 to 14 ring carbon atoms and is unsubstituted or has from 1 to 3 substituents selected from $C_1$-$C_4$ alkyl groups, hydroxy groups, amino groups and $C_1$-$C_4$ alkoxy groups; amino groups having one or two $C_7$-$C_9$ aralkyl substituents in which the aryl part is unsubstituted or has from 1 to 3 substituents selected from $C_1$-$C_4$ alkyl groups, hydroxy groups, amino groups and $C_1$-$C_4$ alkoxy groups; amino groups having one or two carboxylic acyl substituents; aryl groups having from 6 to 14 ring carbon atoms and being unsubstituted or having from 1 to 3 substituents selected from $C_1$-$C_4$ alkyl groups, hydroxy groups, amino groups and $C_1$-$C_4$ alkoxy groups; carboxy groups; and heterocyclic groups having from 5 to 9 ring atoms, of which from 1 to 3 are nitrogen and/or oxygen and/or sulphur hetero-atoms.

4. A method as claimed in Claim 2, wherein X represents a $C_1$-$C_5$ alkylene group which is unsubstituted or has 1 or 2 substituents selected from: hydroxy groups; $C_1$-$C_4$ alkoxy groups; mercapto groups; $C_1$-$C_4$ alkylthio groups; amino groups; amino groups having one or two $C_1$-$C_4$ alkyl substituents; amino groups having one or two carboxylic acyl substituents; aryl groups having from 6 to 14 carbon atoms wherein the aryl ring is unsubstituted or has from 1 to 3 substituents selected from $C_1$-$C_4$ alkyl groups, hydroxy groups, amino groups and $C_1$-$C_4$ alkoxy groups; carboxy groups; and heterocyclic groups having from 5 to 9 ring atoms, of which from 1 to 3 are nitrogen and/or oxygen hetero-atoms.

5. A method as claimed in any one of Claims 1 to 4, wherein the N-acyl group is: a $C_1$-$C_{18}$ alkanoyl group; a $C_3$-$C_8$ alkenoyl group; a $C_3$-$C_8$ alkynoyl group; an aromatic acyl group wherein the aryl part is $C_6$-$C_{14}$ carbocyclic aryl and is unsubstituted or has from 1 to 5 substituents selected from $C_1$-$C_4$ alkyl groups, hydroxy groups, $C_1$-$C_4$ alkoxy groups, amino groups, sulpho groups and halogen atoms; a cycloalkanecarbonyl group where the cycloalkane part is $C_3$-$C_8$ and is unsubstituted or has at least one substituent selected from $C_1$-$C_4$ alkyl groups and phenyl groups; an araliphatic acyl group in which the aryl ring is a carbocyclic ring having from 6 to 14 carbon atoms and which is unsubstituted or has from 1 to 5 substituents selected from $C_1$-$C_4$ alkyl groups, hydroxy groups, $C_1$-$C_4$ alkoxy groups, amino groups, sulpho groups and halogen atoms, and in which the alkyl moiety has from 1 to 4 carbon atoms; a heterocyclic acyl group which has a saturated or unsaturated ring system, the rings having 5 or 6 ring atoms, of which from 1 to 3 are nitrogen and/or sulphur and/or oxygen hetero-atoms and the ring being unsubstituted or having from 1 to 3 substituents selected from $C_1$-$C_4$ alkyl groups and hydroxy groups; a $C_2$-$C_7$ alkoxycarbonyl groups; an aralkyloxycarbonyl group where the aralkyl part has from 7 to 9 carbon atoms and is unsubstituted or has from 1 to 5 substituents selected from amino groups, $C_1$-$C_4$ alkyl groups, $C_1$-$C_4$ alkoxy groups and hydroxy groups; or an acyl group derived from an amino acid by removal of OH from the carboxylic acid group and N-acylation of the amino group with at least one of the above-mentioned acyl groups.

6. A method as claimed in any one of Claims 1 to 4, wherein the N-acyl group is: a saturated aliphatic acyl group having from 1 to 8 carbon atoms; an aromatic acyl group in which the aryl moiety has from 6 to 10 ring carbon atoms and is unsubstituted or has from 1 to 3 substituents selected from $C_1$-$C_4$ alkyl groups and $C_1$-$C_4$ alkoxy groups; an alicyclic acyl group in which the cycloalkane ring has from 3 to 6 carbon atoms; an araliphatic acyl group in which the aryl ring has from 6 to 10 ring carbon atoms and the alkyl group has from 1 to 4 carbon atoms, the aryl ring being unsubstituted or having from 1 to 3 substituents selected from $C_1$-$C_4$ alkyl groups and $C_1$-$C_4$ alkoxy groups; a heterocyclic acyl group in which the heterocyclic ring is saturated or unsaturated and has 5 or 6 ring atoms of which one is a nitrogen, sulphur or oxygen hetero-atom; an alkoxycarbonyl group having a total of from 2 to 7 carbon atoms; an aralkyloxycarbonyl group in which the aralkyl moiety has from 7 to 9 carbon atoms and the aryl ring is unsubstituted or has from 1 to 3 substituents selected from $C_1$-$C_4$ alkyl groups and $C_1$-$C_4$ alkoxy groups; or an acyl group derived from an amino acid by removal of OH from the carboxylic acid group and N-acylation of the amino group with at least one of the above-mentioned acyl groups.

7. A method as claimed in any one of Claims 1 to 4, wherein the N-acyl group is: an aromatic acyl group in which the aryl ring has from 6 to 10 ring atoms and which is unsubstituted or has a single substituent selected from $C_1$-$C_4$ alkyl groups, $C_1$-$C_4$ alkoxy groups, hydroxy groups and amino groups; an alicyclic acyl group in which the cycloalkane moiety has from 3 to 6 carbon atoms; a phenylaliphatic acyl group in which the phenyl group is unsubstituted or has a single $C_1$-$C_4$ alkyl substituent, and in which the

alkyl part has from 1 to 4 carbon atoms; an alkoxycarbonyl group having a total of from 4 to 6 carbon atoms; an aralkyloxycarbonyl group in which the aralkyl part has from 7 to 9 carbon atoms and has 0 or 1 substituent selected from $C_1$-$C_4$ alkyl groups and $C_1$-$C_4$ alkoxy groups; or an acyl group derived from am amino acid by removal of OH from the carboxylic acid group and N-acylation of the amino group with at least one of the above-mentioned acyl groups.

**8.** A method as claimed in any one of Claims 1 to 4, wherein the N-acyl group is an acetyl, benzoyl, cyclohexanecarbonyl, cyclopropanecarbonyl, hexanoyl, isobutyryl, crotonoyl ethoxycarbonyl, 4-hydroxybenzoyl, anisoyl, 4-aminobenzoyl, naphthoyl, toluoyl, benzyloxycarbonyl or 4-methoxybenzyloxycarbonyl group.

**9.** A method as claimed in any one of Claims 1 and 5 to 8, wherein said amino acid is glycine, β-alanine, 4-aminobutyric acid, 5-aminovaleric acid, 6-aminohexanoic acid, 8-aminooctanoic acid, alanine, 2-aminobutyric acid, norvaline, valine, leucine, isoleucine, norleucine, tyrosine, O-methyltyrosine, aspartic acid, glutamic acid, 4-carboxyglutamic acid, 3-methylaspartic acid, 2-aminoadipic acid, 2-aminopimelic acid, 2-aminosuberic acid, 3-hydroxyaspartic acid, 3-hydroxyglutamic acid, 2,3-diaminopropionic acid 2,4-diaminobutyric acid, 5-hydroxylysine, arginine, $N^{\delta}$,$N^{\delta}$-dimethylornithine, $N^{\epsilon}$-methyllysine, cysteine, methionine, ethionine, S-carboxymethylcysteine, S-benzylcysteine, methionine S-oxide, ethionine S-oxide, methionine S,S-dioxide, cysteic acid, serine, O-methylserine, threonine, O-methylthreonine, homothreonine, ethoxinine, 3-methoxyvaline, 3-phenylserine, 3-methyl-3-phenylalanine, histidine, tryptophan, 2-methylalanine, 2-methylserine 2-hydroxyisoleucine, 2-methylmethionine, 2-ethyl-2-phenylglycine, 3-aminobutyric acid, 3-amino-4-methylvaleric acid, 3-amino-3-phenylpropionic acid, 3-amino-2-hydroxypropionic acid or 4-amino-3-hydroxybutyric acid.

**10.** A method as claimed in any one of Claims 1 and 5 to 8, wherein said amino acid is glycine, β-alanine, 4-aminobutyric acid, 5-aminovaleric acid, 6-aminohexanoic acid, 8-aminooctanoic acid, alanine, norvaline, valine, leucine, isoleucine, norleucine, $N^{\delta}$,$N^{\delta}$-dimethylornithine, methionine, ethionine, O-methylserine, O-methylthreonine, ethoxinine, 3-methoxyvaline, 3-phenylserine, 3-methyl-3-phenylalanine, histidine, 2-methylalanine, 2-methylserine, 2-hydroxyisoleucine, 2-ethylphenylglycine, 3-aminobutyric acid, 3-amino-4-methylvaleric acid or 3-amino-3-phenylpropionic acid.

**11.** A method as claimed in any one of Claims 1 and 5 to 8, wherein said amino acid is β-alanine, 4-aminobutyric acid, 5-aminovaleric acid, 6-aminohexanoic acid, alanine valine leucine, norleucine methionine, histidine or glycine.

**12.** A method as claimed in any one of Claims 1 and 5 to 8, wherein said amino acid is leucylglycine, glycyl-β-alanine, glycylalanine, valylalanine, leucylalanine, glycylvaline, alanylvaline, leucylvaline, valylleucine phenylalanylleucine, histidylleucine, glycylphenylalanine, alanylphenylalanine, leucylphenylalanine, glycylmethionine valylmethionine, glycylhistidine, alanylvalylglycine, glycylalanylvaline, glycylphenylalanylleucine or glycylglycylhistidine.

**13.** A method as claimed in Claim 1, wherein said N-acylated amino acid is:

N-(p-toluoyl)-β-alanine
N-(4-methoxybenzoyl)-β-alanine
N-(3-hydroxy-2-naphthoyl)-β-alanine
N-benzoylglycyl-β-alanine
N-benzoyl-β-alanine
N-benzoyl-5-aminovaleric acid
N-benzoyl-6-aminohexanoic acid
N-cyclohexanecarbonyl-6-aminohexanoic acid
N-(N-methylnicotinoyl)-6-aminohexanoic acid
N-benzoyl-8-aminooctanoic acid
N-benzoylalanine
N-(1-naphthoyl)alanine
N-benzoylvalylalanine
N-benzoyl-2-aminobutyric acid
N-benzoylnorvaline

N-valerylvaline
N-benzoylalanylvaline
N-benzoylvaline
N-benzoylleucine
N-benzoylglycylphenylalanylleucine
N-benzoylnorleucine
N-benzoylglycylphenylalanine
N-benzoylalanylphenylalanine
N-cyclohexanecarbonylleucylphenylalanine
N-benzoyl-O-methyltyrosine
N-benzoylmethionine
N-phenylacetylmethionine
N-benzoylvalylmethionine
N-benzoylethionine
N-(4-methoxybenzyloxycarbonyl)ethionine
N-benzoylthreonine
N-benzoylhistidine
N-(p-toluoyl)histidine
N-(4-methoxybenzoyl)histidine
N-(4-methoxybenzoyl)-3-aminobutyric acid
or
N-butyryl-3-amino-3-phenylpropionic acid.

**14.** A method as claimed in any one of the preceding Claims, wherein said antibiotic is a compound of general formula I:

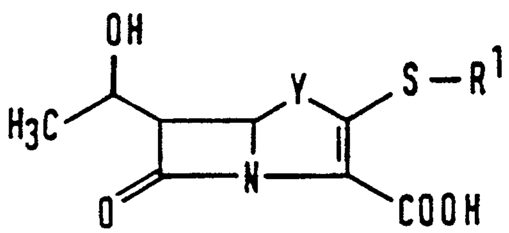

(I)

in which:

Y represents a sulphur atom, a methylene group or a methylene group having 1 or 2 methyl and/or methoxy substituents; and

$R^1$ represents a $C_1$-$C_6$ alkyl group, a $C_1$-$C_6$ alkyl group having at least one of substituents (i) or a heterocyclic group having from 4 to 14 ring atoms of which from 1 to 5 are nitrogen and/or oxygen and/or sulphur hetero-atoms where said heterocyclic group is unsubstituted or has at least one of substituents (ii);

substituents (i):
halogen atoms, amino groups, amino groups having at least one of substituents (iii), $C_1$-$C_4$ alkylideneamino groups, $C_1$-$C_4$ aminoalkylideneamino groups, amidino groups, amidino groups having from 1 to 3 of substituents (iii), heterocyclic groups having from 4 to 14 ring atoms of which from 1 to 5 are nitrogen and/or oxygen and/or sulphur hetero-atoms wherein said heterocyclic group is unsubstituted or has at least one of substituents (ii), imino groups, cyano groups, carbamoyl groups and carbamoyl groups having at least one substituent selected from $C_1$-$C_4$ alkyl groups and $C_1$-$C_4$ alkoxy groups;
substituents (ii):
$C_1$-$C_6$ alkanimidoyl groups, $C_1$-$C_6$ alkyl groups, alkoxyalkyl groups where the alkoxy and alkyl parts

are each $C_1$-$C_4$, carbamoyl groups, carbamoyl groups having at least one substituent selected from $C_1$-$C_4$ alkyl groups and $C_1$-$C_4$ alkoxy groups, $C_1$-$C_4$ haloalkyl groups, heterocyclic acylimidoyl groups where the heterocyclic part has from 5 to 9 ring atoms of which from 1 to 3 are nitrogen and/or oxygen and/or sulphur hetero-atoms, amidino groups, amidino groups having from 1 to 3 of substituents (iii), amino groups, oxygen atoms, $C_1$-$C_6$ alkanoyl groups, $C_1$-$C_6$ alkanesulphonyl groups, $C_1$-$C_6$ alkanesulphinyl groups, hydroximino groups, $C_1$-$C_4$ alkoximino groups, carbamoyloxy groups, carbamoyloxy groups having at least one substituent selected from $C_1$-$C_4$ alkyl groups and $C_1$-$C_4$ alkoxy groups, carbamoyloxyalkyl groups where the alkyl part is $C_1$-$C_4$ and the carbamoyl part is unsubstituted or has at least one substituent selected from $C_1$-$C_4$ alkyl groups and $C_1$-$C_4$ alkoxy groups and $C_1$-$C_4$ iminoalkyl groups;

substituents (iii):

$C_1$-$C_6$ alkyl groups, $C_2$-$C_6$ alkenyl groups, $C_2$-$C_6$ alkynyl groups, and said alkyl, alkenyl and alkynyl groups having at least one substituent selected from halogen atoms, carbamoyloxy groups and carbamoyloxy groups having at least one substituent selected from $C_1$-$C_4$ alkyl groups and $C_1$-$C_4$ alkoxy groups;

and pharmaceutically acceptable salts thereof.

**15.** A method as claimed in Claim 14, wherein Y represents a sulphur atom, a methylene group, or the group $CH_3$-CH<, $CH_3O$-CH< or $(CH_3)_2C$<.

**16.** A method as claimed in Claim 14 or Claim 15, wherein $R^1$ represents an ethyl, 2-fluoroethyl, 2-(aminomethyleneamino)ethyl, $N^1$,$N^1$-dimethylamidinomethyl, $N^1$,$N^1$,$N^2$-trimethylamidinomethyl, 3-pyrrolidinyl, 1-formimidoyl-3-pyrrolidinyl, 1-acetimidoyl-3-pyrrolidinyl, 1-propionimidoyl-3-pyrrolidinyl, 2-methyl-1,4,5,6-tetrahydro-5-pyrimidinyl, 2-methoxymethyl-1,4,5,6-tetrahydro-5-pyrimidinyl, 3-azetidinyl, 1-acetimidoyl-3-azetidinyl, $N^1$-methyl-$N^1$-(2-propynyl)amidinomethyl, $N^1$-(2-fluoroethyl)-$N^1$-methylamidinomethyl, $N^1$-(3-fluoropropyl)-$N^1$-methylamidinomethyl, $N^1$-methyl-$N^1$-(2,2,2-trifluoroethyl)-amidinomethyl, 1-(3-azetidinyl)ethyl, 1-(1-acetimidoyl-3-azetidinyl)ethyl, 1,4,5,6-tetrahydro-2-pyrimidinylmethyl, 1-(4,5-dihydro-2-thiazolyl)ethyl, 5-carbamoyl-3-pyrrolidinyl, 1-acetimidoyl-5-carbamoyl-3-pyrrolidinyl,2-chloromethyl-1,4,5,6-tetrahydro-5-pyrimidinyl, 1-butyrimidoyl-3-pyrrolidinyl, 1-nicotinimidoyl-3-pyrrolidinyl, $N^1$-,$N^1$-diallylamidinomethyl, $N^1$-methyl-$N^1$-(2-propynyl)amidino, $N^1$-(2-fluoroethyl)-$N^1$-methylamidino, $N^1$-(3-fluoropropyl)-$N^1$-methylamidino, $N^1$-methyl-$N^1$-(2,2,2-trifluoroethyl)-amidino, $N^1$-allyl-$N^1$-methylamidinomethyl, cyanomethyl, 2-cyanoethyl, 1-cyanoethyl, 2-cyano-1-methylethyl, 2-aminoethyl, 1-carbamoylethyl, 2-(1-aminoethylideneamino)ethyl, 1-amidino-3-pyrrolidinyl, 2-methyl-1-3-diazabicyclo-[3.3.0]oct-2-en-7-yl, 2-methoxymethyl-1,3-diazabicyclo[3.3.0]oct-2-en-7-yl, 5-imino-2-pyrrolidinyl, 2-imino-5-piperidinyl, 1-acetimidoyl-5-methylcarbamoyl-3-pyrrolidinyl, 1-acetimidoyl-5-methoxycarbamoyl-3-pyrrolidinyl, 2-imino-2-(S-oxothiomorpholino)ethyl, 2-imino-2-(1,1-dioxo-1,3-thiazolidin-3-yl)ethyl, 2-imino-2-(S,S-dioxothiomorpholino)ethyl, 2-imino-2-(3,5-dioxo-1-piperazinyl)ethyl, 2-imino-2-(4-methyl-3,5-dioxo-1-piperazinyl)ethyl, 2-imino-2-(3-oxo-1-piperazinyl)ethyl, 2-imino-2-(4-methyl-3-oxo-1-piperazinyl)ethyl, 2-imino-2-(4-acetyl-3-oxo-1-piperazinyl)ethyl, 2-imino-2-(4-methanesulphonyl-3-oxo-1-piperazinyl)ethyl, $N^1$-(2-carbamoyloxyethyl)$N^1$-methylamidinomethyl, 2-(3-hydroximino-1-pyrrolidinyl)-2-iminoethyl, 2-imino-2-(3-methoximino-1-pyrrolidinyl)ethyl, 2-(4-hydroximinopiperidino)-2-iminoethyl, 2-imino-2-(4-methoximinopiperidino)ethyl, 2-(3-carbamoyloxy-1-pyrrolidinyl)-2-iminoethyl, 2-imino-2-(3-oxo-1-piperazinyl)ethyl, 2-(3-carbamoylpiperidino)-2-iminoethyl, 2-(3-carbamoyloxypiperidino)-2-iminoethyl, 2-(2-carbamoyloxy-1-pyrrolidinyl)-2-iminoethyl, 2-(2-carbamoyloxymethyl-1-pyrrolidinyl)-2-iminoethyl, 2-(4-carbamoyloxypiperidino)-2-iminoethyl, 2-(4-formyl-1-piperazinyl)-2-iminoethyl, 2-(4-acetyl-1-piperazinyl)-2-iminoethyl, 1-formyl-3-azetidinyl, 1-iminomethyl-3-azetidinyl, 1-methyl-4-piperidyl, 1-acetimidoyl-4-piperidyl or 1-acetyl-3-pyrrolidinyl group.

**17.** A method as claimed in Claim 14, wherein said antibiotic is:

(5R,6S)-2-{2-[(aminomethylene)amino]ethylthio}-6-[1(R)-hydroxyethyl]-2-carbapenem-3-carboxylic acid

(5R,6S)-2-[(3S-1-acetimidoylpyrrolidin-3-ylthio]-6-[1R)-hydroxyethyl]-2-carbapenem-3-carboxylic acid

(5R,6S)-2-[(3R)-1-acetimidoylpyrrolidin-3-ylthio]-6-[1(R)-hydroxyethyl]-2-carbapenem-3-carboxylic acid

(5R,6S)-2-[(3R)-1-acetimidoylpyrrolidin-3-ylthio]-6-[1(R)-hydroxyethyl]-1(S)-methyl-2-carbapenem-3-carboxylic acid

(5R,6S)-2-[(3S)-1-acetimidoylpyrrolidin-3-ylthio]-6-[1(R)-hydroxyethyl]-1(R)-methyl-2-carbapenem-3-carboxylic acid

(5R,6S-2-[(3S)-1-acetimidoylpyrrolidin-3-ylthio]-6-[1(R-hydroxyethyl]-1(S)-methyl-2-carbapenem-3-carboxylic acid
or
(5R,6S)-2-[(3S)-1-acetimidoyl-5(S)-carbamoylpyrrolidin-3-ylthio]-6-[1(R-hydroxyethyl]-2-carbapenem-3-carboxylic acid.

**18.** A method as claimed in any one of the preceding Claims, wherein the weight ratio of said N-acylated amino acid to said antibiotic is from 1:1 to 4:1.

**19.** A method according to any one of the preceding Claims, which comprises: solubilizing said N-acylated amino acid in water; adding and dissolving said antibiotic in the resulting; and optionally lyophilizing the solution to provide a powdery mixture.

**20.** A method according to Claim 19, in which said N-acylated amino acid is solubilized by dispersing it in water and adding sufficient of a base to adjust the pH to a value of from 5.5 to 9.

**21.** The use for the manufacture of a medicament for the treatment of bacterial infections of:
(a) a penem or carbapenem antibiotic;
in association with:
(b) a pharmaceutically acceptable N-acylated derivative of an amino acid wherein the amino group and the carboxylic acid group are attached to a saturated aliphatic carbon chain or carbon atom, or a salt thereof, provided that the amino acid is not ornithine, lysine, phenylalanine or phenylglycine alone.

**22.** The use as claimed in Claim 21, wherein said N-acylated amino acid is:

N-(p-toluoyl)-β-alanine
N-(4-methoxybenzoyl)-β-alanine
N-(3-hydroxy-2-naphthoyl)-β-alanine
N-benzoylglycyl-β-alanine
N-benzoyl-β-alanine
N-benzoyl-5-aminovaleric acid
N-benzoyl-6-aminohexanoic acid
N-cyclohexanecarbonyl-6-aminohexanoic acid
N-(N-methylnicotinoyl)-6-aminohexanoic acid
N-benzoyl-8-aminooctanoic acid
N-benzoylalanine
N-(1-naphthoyl)alanine
N-benzoylvalylalanine
N-benzoyl-2-aminobutyric acid
N-benzoylnorvaline
N-valerylvaline
N-benzoylalanylvaline
N-benzoylvaline
N-benzoylleucine
N-benzoylglycylphenylalanylleucine
N-benzoylnorleucine
N-benzoylglycylphenylalanine
N-benzoylalanylphenylalanine
N-cyclohexanecarbonylleucylphenylalanine
N-benzoyl-O-methyltyrosine
N-benzoylmethionine
N-phenylacetylmethionine

N-benzoylvalylmethionine
N-benzoylethionine
N-(4-methoxybenzyloxycarbonyl)ethionine
N-benzoylthreonine
N-benzoylhistidine
N-(p-toluoyl)histidine
N-(4-methoxybenzoyl)histidine
N-(4-methoxybenzoyl)-3-aminobutyric acid
or
N-butyryl-3-amino-3-phenylpropionic acid.

23. The use as claimed in Claim 21 or Claim 22, wherein said antibiotic is:

(5R,6S)-2-{2-[(aminomethylene)amino]ethylthio}-6-[1(R)-hydroxyethyl]-2-carbapenem-3-carboxylic acid

(5R,6S)-2-[(3S)-1-acetimidoylpyrrolidin-3-ylthio]-6-[1(R)-hydroxyethyl]-2-carbapenem-3-carboxylic acid

(5R,6S)-2-[(3R)-1-acetimidoylpyrrolidin-3-ylthio]-6-[1(R)-hydroxyethyl]-2-carbapenem-3-carboxylic acid

(5R,6S)-2-[(3R)-1-acetimidoylpyrrolidin-3-ylthio]-6-[1(R)-hydroxyethyl]-1(S)-methyl-2-carbapenem-3-carboxylic acid

(5R,6S)-2-[(3S-1-acetimidoylpyrrolidin-3-ylthio]-6-[1(R)-hydroxyethyl]-1(R)-methyl-2-carbapenem-3-carboxylic acid

(5R,6S)-2-[(3S)-1-acetimidoylpyrrolidin-3-ylthio]-6-[1(R)-hydroxyethyl]-1(S-methyl-2-carbapenem-3-carboxylic acid
or
(5R,6S)-2-[(3S)-1-acetimidoyl-5(S)-carbamoylpyrrolidin-3-ylthio]-6-[1(R)-hydroxyethyl]-2-carbapenem-3-carboxylic acid.

**Revendications**
**Revendications pour les Etats contractants suivants : GB, DE, FR, IT, CH, BE, NL, SE, LU**

1. Composition comprenant:
un antibiotique pénème ou carbapénème, et
un dérivé N-acylé pharmaceutiquement acceptable d'un aminoacide dans lequel le groupe amino et le groupe carboxy sont liés à un atome de carbone saturé ou à une chaîne carbonée aliphatique saturée, ou un sel de celui-ci, étant entendu que lorsque la composition ne comprend qu'un seul dérivé d'aminoacide, l'aminoacide n'est pas l'ornithine, la lysine, la phénylalanine ni la phénylglycine, la quantité du dérivé d'aminoacide étant suffisante pour atténuer toute toxicité rénale de l'antibiotique, de préférence en un rapport pondéral dudit aminoacide N-acylé audit antibiotique allant de 0,1:1 à 4:1.

2. Composition selon la revendication 1, dans laquelle ledit aminoacide est un composé de formule générale II:

$H_2N\text{-}X\text{-}COOH$ (II)

dans laquelle X représente un groupe alkylène en $C_1$-$C_{10}$ ou un groupe alkylène en $C_1$-$C_{10}$ comportant au moins un substituant choisi parmi des groupes hydroxy, alcoxy en $C_1$-$C_4$, aryloxy en $C_6$-$C_{14}$, aryloxy en $C_6$-$C_{14}$ substitués, aralkyloxy en $C_7$-$C_9$, aralkyloxy en $C_7$-$C_9$ substitués, mercapto, alkylthio en $C_1$-$C_4$, arylthio en $C_6$-$C_{14}$, arylthio en $C_6$-$C_{14}$ substitués, aralkylthio en $C_7$-$C_9$, aralkylthio en $C_7$-$C_9$ substitués, carboxyalkylthio en $C_2$-$C_5$, le groupe amino, des groupes amino comportant un ou deux substituants choisis parmi
des radicaux alkyle en $C_1$-$C_4$, aryle en $C_6$-$C_{14}$, aryle en $C_6$-$C_{14}$ substitués, aralkyle en $C_7$-$C_9$, aralkyle en $C_7$-$C_9$ substitués et carboxy,
des groupes aryle en $C_6$-$C_{14}$, aryle en $C_6$-$C_{14}$ substitués, carboxy, amidino, sulfo, alkyl($C_1$-$C_4$)-sulfinyle, alkyl($C_1$-$C_4$)-sulfonyle et des groupes hétérocycliques ayant de 5 à 14 atomes formant le

cycle, dont de 1 à 5 sont des hétéroatomes d'azote et/ou d'oxygène et/ou de soufre, lesdits groupes aryloxy, aralkyloxy, arylthio, aralkylthio, aryle et aralkyle substitués comportant au moins un substituant choisi parmi des groupes alkyle en $C_1$-$C_4$, hydroxy, amino et alcoxy en $C_1$-$C_4$,
ou un sel pharmaceutiquement acceptable de celui-ci.

3. Composition selon la revendication 2, dans laquelle X représente un groupe alkylène en $C_1$-$C_5$ qui n'est pas substitué ou comporte un ou deux substituants choisi(s) parmi des groupes hydroxy, alcoxy en $C_1$-$C_4$, des groupes aryloxy dans lequel le cycle aryle possède de 6 à 14 atomes de carbone formant le cycle et n'est pas substitué ou comporte de un à trois substituants choisi(s) parmi des radicaux alkyle en $C_1$-$C_4$, hydroxy, amino et alcoxy en $C_1$-$C_4$; des groupes aralkyloxy en $C_7$-$C_9$ dans lesquels le fragment aryle n'est pas substitué ou comporte de un à trois substituants choisi(s) parmi des radicaux alkyle en $C_1$-$C_4$, hydroxy, amino et alcoxy en $C_1$-$C_4$; le groupe mercapto, des groupes alkylthio en $C_1$-$C_4$, des groupes arylthio dans lesquels le cycle aryle a de 6 à 14 atomes de carbone formant le cycle et n'est pas substitué ou comporte de un à trois substituants choisi(s) parmi des radicaux alkyle en $C_1$-$C_4$, hydroxy, amino et alcoxy en $C_1$-$C_4$; des groupes aralkylthio en $C_7$-$C_9$ dans lesquels le fragment aryle n'est pas substitué ou comporte de un à trois substituants choisi(s) parmi des radicaux alkyle en $C_1$-$C_4$, hydroxy, amino et alcoxy en $C_1$-$C_4$; des groupes carboxyalkylthio dans lesquels le fragment alkyle a de 1 à 4 atomes de carbone; le groupe amino; des groupes amino comportant un ou deux substituants alkyle en $C_1$-$C_4$; des groupes amino comportant un ou deux substituants aryle dans lesquels le noyau aryle a de 6 à 14 atomes de carbone formant le cycle et n'est pas substitué ou comporte de un à trois substituants choisi(s) parmi des radicaux alkyle en $C_1$-$C_4$, hydroxy, amino et alcoxy en $C_1$-$C_4$; des groupes amino comportant un ou deux substituants aralkyle en $C_7$-$C_9$ dans lesquels le fragment aryle n'est pas substitué ou comporte de un à trois substituants choisi(s) parmi des radicaux alkyle en $C_1$-$C_4$, hydroxy, amino et alcoxy en $C_1$-$C_4$; des groupes amino comportant un ou deux substituants acyle carboxyliques; des groupes aryle ayant de 6 à 14 atomes de carbone formant le cycle et n'étant pas substitués ou comportant de un à trois substituants choisi(s) parmi des radicaux alkyle en $C_1$-$C_4$, hydroxy, amino et alcoxy en $C_1$-$C_4$; le groupe carboxy et des groupes hétérocycliques ayant de 5 à 9 atomes formant le cycle, dont de 1 à 3 sont des hétéroatomes d'azote et/ou d'oxygène et/ou de soufre.

4. Composition selon la revendication 2, dans laquelle X représente un groupe alkylène en $C_1$-$C_5$ qui n'est pas substitué ou comporte un ou deux susbtituants choisi(s) parmi: des groupes hydroxy; alcoxy en $C_1$-$C_4$; mercapto; alkylthio en $C_1$-$C_4$; amino; des groupes amino comportant un ou deux substituants alkyle en $C_1$-$C_4$; des groupes amino comportant un ou deux substituants acyle carboxylique; des groupes aryle ayant de 6 à 14 atomes de carbone, dans lesquels le noyau aryle n'est pas substitué ou comporte de 1 à 3 substituants choisi(s) parmi des radicaux alkyle en $C_1$-$C_4$, hydroxy, amino et alcoxy en $C_1$-$C_4$; le groupe carboxy et des groupes hétérocycliques ayant de 5 à 9 atomes formant le cycle, dont de 1 à 3 sont des hétéroatomes d'azote et/ou d'oxygène.

5. Composition selon l'une quelconque des revendications 1 à 4, dans laquelle le groupe N-acyle est: un groupe alcanoyle en $C_1$-$C_{18}$, alcénoyle en $C_3$-$C_8$, alcynoyle en $C_3$-$C_8$,un groupe acyle aromatique dans lequel le fragment aryle est un reste aryle carbocyclique en $C_6$-$C_{14}$ et n'est pas substitué ou comporte de 1 à 5 substituants choisi(s) parmi des radicaux alkyle en $C_1$-$C_4$, hydroxy, alcoxy en $C_1$-$C_4$, amino, sulfo et des atomes d'halogène; un groupe cycloalcanecarbonyle dans lequel le fragment cycloalcane est en $C_3$-$C_8$ et n'est pas substitué ou comporte au moins un substituant choisi parmi des radicaux alkyle en $C_1$-$C_4$ et phényle; un groupe acyle araliphatique dans lequel le noyau aryle est un noyau carbocyclique ayant de 6 à 14 atomes de carbone qui n'est pas substitué ou comporte de 1 à 5 substituants choisi(s) parmi des groupes alkyle en $C_1$-$C_4$, hydroxy, alcoxy en $C_1$-$C_4$, amino, sulfo et des atomes d'halogène, et dans lequel le fragment alkyle a de 1 à 4 atomes de carbone; un groupe acyle hétérocyclique qui comporte un système cyclique saturé ou insaturé, les cycles ayant 5 ou 6 atomes formant le cycle, dont de 1 à 3 sont des hétéroatomes d'azote et/ou de soufre et/ou d'oxygène et le cycle n'étant pas substitué ou ayant de 1 à 3 substituants choisi(s) parmi des groupes alkyle en $C_1$-$C_4$ et hydroxy; un groupe alcoxy($C_2$-$C_7$)-carbonyle; un groupe aralkyloxycarbonyle dans lequel le fragment aralkyle a de 7 à 9 atomes de carbone et n'est pas substitué ou comporte de 1 à 5 substituants choisi(s) parmi des radicaux amino, alkyle en $C_1$-$C_4$, alcoxy en $C_1$-$C_4$ et hydroxy; ou un groupe acyle dérivé d'un aminoacide par enlèvement d'OH hors du groupe carboxy et N-acylation du groupe amino par au moins un des groupes acyle mentionnés plus haut.

6. Composition selon l'une quelconque des revendications 1 à 4, dans laquelle le groupe N-acyle est: un groupe acyle aliphatique saturé ayant de 1 à 8 atomes de carbone; un groupe acyle aromatique dans lequel le fragment aryle a de 6 à 10 atomes de carbone formant le cycle et n'est pas substitué ou comporte de 1 à 3 substituants choisi(s) parmi des radicaux alkyle en $C_1$-$C_4$ et alcoxy en $C_1$-$C_4$; un groupe acyle alicyclique dans lequel le cycle cycloalcane a de 3 à 6 atomes de carbone; un groupe acyle araliphatique dans lequel le cycle aryle a de 6 à 10 atomes de carbone formant le cycle et le groupe alkyle a de 1 à 4 atomes de carbone, le cycle aryle n'étant pas susbtitué ou comportant de 1 à 3 substituants choisi(s) parmi des radicaux alkyle en $C_1$-$C_4$ et alcoxy en $C_1$-$C_4$; un groupe acyle hétérocyclique dans lequel le noyau hétérocyclique est saturé ou insaturé et a 5 ou 6 atomes formant le cycle, dont un est un hétéroatome d'azote, soufre ou oxygène; un groupe alcoxycarbonyle ayant au total de 2 à 7 atomes de carbone; un groupe aralkyloxycarbonyle dans lequel le fragment aralkyle a de 7 à 9 atomes de carbone et le cycle aryle n'est pas substitué ou comporte de 1 à 3 substituants choisi-(s) parmi des radicaux alkyle en $C_1$-$C_4$ et alcoxy en $C_1$-$C_4$; ou un groupe acyle dérivé d'un aminoacide par enlèvement d'OH hors du groupe carboxy et N-acylation du groupe amino par au moins un des groupes acyle mentionnés ci-dessus.

7. Composition selon l'une quelconque des revendications 1 à 4, dans laquelle le groupe N-acyle est: un groupe acyle aromatique dans lequel le fragment aryle a de 6 à 10 atomes formant le cycle et n'est pas substitué ou comporte un seul substituant choisi parmi des radicaux alkyle en $C_1$-$C_4$, alcoxy en $C_1$-$C_4$, hydroxy et amino; un groupe acyle alicyclique dans lequel le fragment cycloalcane a de 3 à 6 atomes de carbone; un groupe acyle phénylaliphatique dans lequel le fragment phényle n'est pas substitué ou comporte un seul substituant alkyle en $C_1$-$C_4$ et dans lequel le fragment alkyle a de 1 à 4 atomes de carbone; un groupe alcoxycarbonyle ayant au total de 4 à 6 atomes de carbone; un groupe aralkyloxycarbonyle dans lequel le fragment aralkyle a de 7 à 9 atomes de carbone et comporte 0 ou 1 substituant choisi parmi des groupes alkyle en $C_1$-$C_4$ et alcoxy en $C_1$-$C_4$; ou un groupe acyle dérivé d'un aminoacide par enlèvement d'OH hors du groupe carboxy et N-acylation du groupe amino par au moins un des groupes acyle mentionnés ci-dessus.

8. Composition selon l'une quelconque des revendications 1 à 4, dans laquelle le groupe N-acyle est le groupe acétyle benzoyle, cyclohexanecarbonyle, cyclopropanecarbonyle, hexanoyle, isobutyryle, crotonoyle, éthoxycarbonyle, 4-hydroxybenzoyle, anisoyle, 4-aminobenzoyle, naphtoyle, toluyle, benzyloxycarbonyle ou 4-méthoxybenzyloxycarbonyle.

9. Composition selon l'une quelconque des revendications 1 et 5 à 8, dans laquelle ledit aminoacide est la glycine, la $\beta$-alanine, l'acide 4-aminobutyrique, l'acide 5-aminovalérique, l'acide 6-aminohexanoïque, l'acide 8-aminooctanoïque, l'alanine, l'acide 2-aminobutyrique, la norvaline, la valine, la leucine, l'isoleucine, la norleucine, la tyrosine, l'O-méthyltyrosine, l'acide aspartique, l'acide glutamique, l'acide 4-carboxyglutamique, l'acide 3-méthylaspartique, l'acide 2-aminoadipique, l'acide 2-aminopimélique, l'acide 2-aminosubérique, l'acide 3-hydroxyaspartique, l'acide 3-hydroxyglutamique, l'acide 2,3-diaminopropionique, l'acide 2,4-diaminobutyrique, la 5-hydroxylysine, l'arginine, la $N^\delta$,$N^\delta$-diméthylornithine, la $N^\epsilon$-méthyllysine, la cystéine, la méthionine, l'éthionine, la S-carboxyméthylcystéine, la S-benzylcystéine, le S-oxyde de méthionine, le S-oxyde d'éthionine, le S,S-dioxyde de méthionine, l'acide cystéique, la sérine, l'O-méthylsérine, la thréonine, l'O-méthylthréonine, l'homothréonine, l'éthoxinine, la 3-méthoxyvaline, la 3-phénylsérine, la 3-méthyl-3-phénylalanine, l'histidine, le tryptophane, la 2-méthylalanine, la 2-méthylsérine, la 2-hydroxy-isoleucine, la 2-méthylméthionine, la 2-éthyl-2-phénylglycine, l'acide 3-aminobutyrique, l'acide 3-amino-4-méthylvalérique, l'acide 3-amino-3-phénylpropionique, l'acide 3-amino-2-hydroxypropionique ou l'acide 4-amino-3-hydroxybutyrique.

10. Composition selon l'une quelconque des revendications 1 et 5 à 8, dans laquelle ledit aminoacide est la glycine, la $\beta$-alanine, l'acide 4-aminobutyrique, l'acide 5-aminovalérique, l'acide 6-aminohexanoïque, l'acide 8-amino-octanoïque, l'alanine, la norvaline, la valine, la leucine, l'isoleucine, la norleucine, la $N^\delta$,$N^\delta$-diméthylornithine, la méthionine, l'éthionine, l'O-méthylsérine, l'O-méthylthréonine, l'éthoxinine, la 3-méthoxyvaline, la 3-phénylsérine, la 3-méthyl-3-phénylalanine, l'histidine, la 2-méthylalanine, la 2-méthylsérine, la 2-hydroxy-isoleucine, la 2-éthylphénylglycine, l'acide 3-aminobutyrique, l'acide 3-amino-4-méthylvalérique ou l'acide 3-amino-3-phénylpropionique.

11. Composition selon l'une quelconque des revendications 1 et 5 à 8, dans laquelle ledit aminoacide est la $\beta$-alanine, l'acide 4-aminobutyrique, l'acide 5-aminovalérique, l'acide 6-aminohexanoïque, l'alanine, la

valine, la leucine, la norleucine, la méthionine, l'histidine ou la glycine.

12. Composition selon l'une quelconque des revendications 1 et 5 à 8, dans laquelle l'aminoacide est la leucylglycine, la glycyl-β-alanine, la glycylalanine, la valylalanine, la leucylalanine, la glycylvaline, l'alanylvaline, la leucylvaline, la valylleucine, la phénylalanylleucine, l'histidylleucine, la glycylphénylalanine, l'alanylphénylalanine, la leucylphénylalanine, la glycylméthionine, la valylméthionine, la glycylhistidine, l'alanylvalylglycine, la glycylalanylvaline, la glycylphénylalanylleucine ou la glycylglycylhistidine.

13. Composition selon la revendication 1, dans laquelle ledit aminoacide N-acylé est:
la N-(p-toluyl)-β-alanine
la N-(4-méthoxybenzoyl)-β-alanine
la N-(3-hydroxy-2-naphtoyl)-β-alanine
la N-benzoylglycyl-β-alanine
la N-benzoyl-β-alanine
l'acide N-benzoyl-5-aminovalérique
l'acide N-benzoyl-6-aminohexanoïque
l'acide N-cyclohexanecarbonyl-6-aminohexanoïque
l'acide N-(N-méthylnicotinoyl)-6-aminohexanoïque
l'acide N-benzoyl-8-amino-octanoïque
la N-benzoylalanine
la N-(1-naphtoyl)alanine
la N-benzoylvalylalanine
l'acide N-benzoyl-2-aminobutyrique
la N-benzoylnorvaline
la N-valérylvaline
la N-benzoylalanylvaline
la N-benzoylvaline
la N-benzoylleucine
la N-benzoylglycylphénylalanylleucine
la N-benzoylnorleucine
la N-benzoylglycylphénylalanine
la N-benzoylalanylphénylalanine
la N-cyclohexanecarbonylleucylphénylalanine
la N-benzoyl-O-méthyltyrosine
la N-benzoylméthionine
la N-phénylacétylméthionine
la N-benzoylvalylméthionine
la N-benzoyléthionine
la N-(4-méthoxybenzyloxycarbonyl)éthionine
la N-benzoylthréonine
la N-benzoylhistidine
la N-(p-toluyl)histidine
la N-(4-méthoxybenzoyl)histidine
l'acide N-(4-méthoxybenzoyl)-3-aminobutyrique ou
l'acide N-butyryl-3-amino-3-phénylpropionique.

14. Composition selon l'une quelconque des revendications précédentes, dans laquelle ledit antibiotique est un composé de formule générale I:

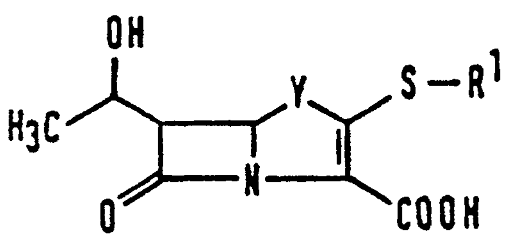

(II) (I)

dans laquelle
Y représente un atome de soufre, le groupe méthylène ou un groupe méthylène comportant un ou deux substituants méthyle et/ou méthoxy; et
$R^1$ représente un groupe alkyle en $C_1$-$C_6$, un groupe alkyle en $C_1$-$C_6$ comportant au moins un des substituants (I) ou un groupe hétérocyclique ayant de 4 à 14 atomes formant le cycle, dont de 1 à 5 sont des hétéroatomes d'azote et/ou d'oxygène et/ou de soufre, ledit groupe hétérocyclique n'étant pas substitué ou comportant au moins un des substituants (II):

substituants (I):

des atomes d'halogène, le groupe amino, des groupes amino comportant au moins un des substituants (III), des groupes alkylidène($C_1$-$C_4$)-amino, amino-alkylidène($C_1$-$C_4$)-amino, le groupe amidino, des groupes amidino comportant de 1 à 3 des substituants (III), des groupes hétérocycliques ayant de 4 à 14 atomes formant le cycle, dont de 1 à 5 sont des hétéroatomes d'azote, et/ou d'oxygène et/ou de soufre, ledit groupe hétérocyclique n'étant pas substitué ou comportant au moins un des substituants (II), le groupe imino, cyano, carbamoyle et des groupes carbamoyle comportant au moins un substituant choisi parmi des radicaux alkyle en $C_1$-$C_4$ et alcoxy en $C_1$-$C_4$;

substituants (II):

des groupes alcane($C_1$-$C_6$)-imidoyle, alkyle en $C_1$-$C_6$, des groupes alcoxyalkyle dans lesquels les fragments alkyle et alcoxy sont chacun en $C_1$-$C_4$, le groupe carbamoyle, des groupes carbamoyle comportant au moins un substituant choisi parmi des radicaux alkyle en $C_1$-$C_4$ et alcoxy en $C_1$-$C_4$, des groupes halogénoalkyle en $C_1$ -$C_4$, des groupes acylimidoyle hétérocycliques dans lesquels le fragment hétérocyclique a de 5 à 9 atomes formant le cycle, dont de 1 à 3 sont des hétéroatomes d'azote et/ou d'oxygène et/ou de soufre, le groupe amidino, des groupes amidino comportant de 1 à 3 substituants (III), le groupe imino, des atomes d'oxygène, des groupes alcanoyle en $C_1$-$C_6$, alcane-($C_1$-$C_6$)-sulfonyle, alcane($C_1$-$C_6$)-sulfinyle, hydroximino, alcoximino en $C_1$-$C_4$, carbamoyloxy, des groupes carbamoyloxy comportant au moins un substituant choisi parmi des radicaux alkyle en $C_1$-$C_4$ et alcoxy en $C_1$-$C_4$, des groupes carbamoyloxyalkyle dans lesquels le fragment alkyle est en $C_1$-$C_4$ et le fragment carbamoyle n'est pas substitué ou comporte au moins un substituant choisi parmi des radicaux alkyle en $C_1$-$C_4$ et alcoxy en $C_1$-$C_4$ et des groupes iminoalkyle en $C_1$-$C_4$;

substituants (III):

des groupes alkyle en $C_1$-$C_6$, alcényle en $C_2$-$C_6$, alcynyle en $C_2$-$C_6$, lesdits groupes alkyle, alcényle et alcynyle comportant au moins un substituant choisi parmi des atomes d'halogène, des radicaux carbamoyloxy et des radicaux carbamoyloxy comportant au moins un substituant choisi parmi des restes alkyle en $C_1$-$C_4$ et alcoxy en $C_1$-$C_4$;

ou les sels pharmaceutiquement acceptables de celui-ci.

**15.** Composition selon la revendication 14, dans laquelle Y représente un atome de soufre, le groupe méthylène ou le groupe $CH_3$-CH<, $CH_3O$-CH< ou $(CH_3)_2C$<.

**16.** Composition selon la revendication 14 ou 15, dans laquelle $R^1$ représente le groupe éthyle, 2-fluoroéthyle, 2-(aminométhylèneamino)éthyle, $N^1$,$N^1$,$N^2$-triméthylamidinométhyle, 3-pyrrolidinyle, 1-formimidoyl-3-pyrrolidinyle, 1-acétimidoyl-3-pyrrolidinyle, 1-propionimidoyl-3-pyrrolidinyle, 2-méthyl-1,4,5,6-tétrahydro-5-pyrimidinyle, 2-méthoxyméthyl-1,4,5,6-tétrahydro-5-pyrimidinyle, 3-azétidinyle, 1-acétimidoyl-3-azétidinyle, $N^1$-méthyl-$N^1$-(2-propynyl)amidinométhyle, $N^1$-(2-fluoroéthyl)-$N^1$-méthylamidinométhyle, $N^1$-(3-fluoropropyl)-$N^1$-méthylamidinométhyle, $N^1$-méthyl-$N^1$-(2,2,2-trifluoroéthyl)-amidinométhyle, 1-(3-azétidinyl)éthyle, 1-(1-acétimidoyl)-3-azétidinyl)éthyle, 1,4,5,6-tétrahydro-2-pyrimidinylméthyle, 1-(4,5-dihydro-2-thiazolyl)éthyle, 5-carbamoyl-3-pyrrolidinyle, 1-acétimidoyl-5-carbamoyl-3-pyrrolidinyle, 2-chlorométhyl-1,4,5,6-tétrahydro-5-pyrimidinyle, 1-butyrimidoyl-3-pyrrolidinyle, 1-nicotinimidoyl-3-pyrrolidinyle, $N^1$,$N^1$-diallylamidinométhyle, $N^1$-méthyl-$N^1$-(2-propynyl)amidino, $N^1$-(2-fluoroéthyl)-$N^1$-méthylamidino, $N^1$-(3-fluoropropyl)-$N^1$-méthylamidino, $N^1$-méthyl-$N^1$-(2,2,2-trifluoroéthyl)amidino, $N^1$-allyl-$N^1$-méthylamidinométhyle, cyanométhyle, 2-cyanoéthyle, 1-cyanoéthyle, 2-cyano-1-méthyléthyle, 2-aminoéthyle, 1-carbamoyléthyle, 2-(1-aminoéthylidèneamino)éthyle, 1-amidino-3-pyrrolidinyle, 2-méthyl-1,3-diazabicyclo[3.3.O]oct-2-ène-7-yle, 2-méthoxyméthyl-1,3-diazabicyclo[3.3.O]oct-2-ène-7-yle, 5-imino-2-pyrrolidinyle, 2-imino-5-pipéridinyle, 1-acétimidoyl-5-méthylcarbamoyl-3-pyrrolidinyle, 1-acétimidoyl-5-méthoxycarbamoyl-3-pyrrolidinyle, 2-imino-2-(S-oxothiomorpholino)éthyle, 2-imino-2-(1,1-dioxo-1,3-thiazolidine-3-yl)éthyle, 2-imino-2-(S,S-dioxothiomorpholino)éthyle, 2-imino-2-(3,5-dioxo-1-pipérazinyl)éthyle, 2-imino-2-(4-méthyl-3,5-dioxo-1-pipérazinyl)éthyle, 2-imino-2-(3-oxo-1-pipérazinyl)éthyle, 2-imino-2-(4-méthyl-3-oxo-1-pipérazinyl)éthyle, 2-imino-2-(4-acétyl-3-oxo-1-pipérazinyl)-éthyle, 2-imino-2-(4-méthanesulfonyl-3-oxo-1-pipérazinyl)éthyle, $N^1$-(2-carbamoyloxyéthyl)-$N^1$-méthyla-

midinométhyle, 2-(3-hydroximino-1-pyrrolidinyl)-2-iminoéthyle, 2-imino-2-(3-méthoximino-1-pyrrolidinyl)-éthyle, 2-(4-hydroximino-pipéridino)-2-iminoéthyle, 2-imino-2-(4-méthoximino- pipéridino)éthyle, 2-(3-carbamoyloxy-1-pyrrolidinyl)-2-iminoéthyle, 2-imino-2-(3-oxo-1-pipérazinyl)éthyle, 2-(3-carbamoylpipéridino)-2-iminoéthyle, 2-(3-carbamoyloxypipéridino)-2-iminoéthyle, 2-(2-carbamoyloxy-1-pyrrolidinyl)2-iminoéthyle, 2-(2-carbamoyloxyméthyl-1-pyrrolidinyl)-2iminoéthyle, 2-(4-carbamoyloxypipéridino)-2-iminoéthyle, 2-(4-formyl-1-pipérazinyl)-2-iminoéthyle, 2-(4-acétyl-1-pipérazinyl)-2-iminoéthyle, 1-formyl-3-azétidinyle, 1-imino-méthyl-3-azétidinyle, 1-méthyl-4-pipéridyle, 1-acétimidoyl-4pipéridyle ou 1-acétyl-3-pyrrolidinyle.

**17.** Composition selon la revendication 14, dans laquelle ledit antibiotique est:
l'acide (5R,6S)-2-{2-[(aminométhylène)amino]éthylthio}-6-[1(R)-hydroxyéthyl]-2-carbapénème-3-carboxylique
l'acide (5R,6S)-2-[(3S)-1-acétimidoylpyrrolidine-3-ylthio]-6-[1(R)-hydroxyéthyl]-2-carbapénème-3-carboxylique
l'acide (5R,6S)-2-[(3R)-1-acétimidoylpyrrolidine-3-ylthio]-6-[1(R)-hydroxyéthyl]-2-carbapénème-3-carboxylique
l'acide (5R,6S)-2-[(3R)-1-acétimidoylpyrrolidine-3-ylthio]-6-[1(R)-hydroxyéthyl]-1(S)-méthyl-2-carbapénème-3-carboxylique
l'acide (5R,6S)-2-[(3S)-1-acétimidoylpyrrolidine-3-ylthio]-6-[1(R)-hydroxyéthyl]-1(R)-méthyl-2-carbapénème-3-carboxylique
l'acide (5R,6S)-2-[(35)-1-acétimidoylpyrrolidine-3-ylthio]-6-[1(R)-hydroxyéthyl]-1(S)-méthyl-2-carbapénème-3-carboxylique
ou
l'acide (5R,6S)-2-[(3S)-1-acétimidoyl-5(S)-carbamoylpyrrolidine-3-ylthio]-6-[1(R)-hydroxyéthyl]-2-carbapénème-3-carboxylique.

**18.** Composition selon l'une quelconque des revendications précédentes, dans laquelle le rapport pondéral dudit aminoacide N-acylé audit antibiotique va de 0,1:1 à 4:1.

**19.** Composition pharmaceutique conditionnée comprenant:
(a) d'une part, un antibiotique pénème ou carbapénème; et
(b) d'autre part, un dérivé N-acylé pharmaceutiquement acceptable d'un aminoacide dans lequel le groupe amino et le groupe carboxy sont liés à un atome de carbone saturé ou à une chaîne carbonée aliphatique saturée, ou un sel de celui-ci, étant entendu que l'aminoacide n'est pas l'ornithine, la lysine, la phénylalanine ni la phénylglycine seule.

**20.** Composition selon la revendication 19, dans laquelle ledit aminoacide N-acylé est tel que défini dans l'une quelconque des revendications 2 à 13.

**21.** Composition selon la revendication 19 ou 20, dans laquelle ledit antibiotique est tel que défini dans l'une quelconque des revendications 14 à 17.

**22.** Utilisation, pour la fabrication d'un médicament pour le traitement d'infections bactériennes, de
(a) un antibiotique pénème ou carbapénème,
en association avec
(b) un dérivé N-acylé pharmaceutiquement acceptable d'un aminoacide dans lequel le groupe amino et le groupe carboxy sont liés à un atome de carbone saturé ou à une chaîne carbonée aliphatique saturée, ou un sel de celui-ci, étant entendu que l'aminoacide n'est pas l'ornithine, la lysine, la phénylalanine ni la phénylglycine seule.

**23.** Utilisation selon la revendication 22, dans laquelle ledit aminoacide N-acylé est tel que défini dans l'une quelconque des revendications 2 à 13.

**24.** Utilisation selon la revendication 22 ou 23, dans laquelle ledit antibiotique est tel que défini dans l'une quelconque des revendications 14 à 17.

**Revendications pour les Etats contractants suivants : AT, ES**

1. Procédé de préparation d'une composition pharmaceutique par mélange: d'un antibiotique pénème ou carbapénème, et d'un dérivé N-acylé pharmaceutiquement acceptable d'un aminoacide dans lequel le groupe amino et le groupe carboxy sont liés à un atome de carbone saturé ou à une chaîne carbonée aliphatique saturée, ou d'un sel de celui-ci, étant entendu que lorsque la composition ne comprend qu'un seul dérivé d'aminoacide, l'aminoacide n'est pas l'ornithine, la lysine, la phénylalanine ni la phénylglycine, la quantité du dérivé d'aminoacide étant suffisante pour atténuer toute toxicité rénale de l'antibiotique, de préférence en un rapport pondéral dudit aminoacide N-acylé audit antibiotique allant de 0,1:1 à 4:1.

2. Procédé selon la revendication 1, dans lequel ledit aminoacide est un composé de formule générale II:

$H_2N$-X-COOH (II)

dans laquelle X représente un groupe alkylène en $C_1$-$C_{10}$ ou un groupe alkylène en $C_1$-$C_{10}$ comportant au moins un substituant choisi parmi des groupes hydroxy, alcoxy en $C_1$-$C_4$, aryloxy en $C_6$-$C_{14}$, aryloxy en $C_6$-$C_{14}$ substitués, aralkyloxy en $C_7$-$C_9$, aralkyloxy en $C_7$-$C_9$ substitués, mercapto, alkylthio en $C_1$-$C_4$, arylthio en $C_6$-$C_{14}$, arylthio en $C_6$-$C_{14}$ substitués, aralkylthio en $C_7$-$C_9$, aralkylthio en $C_7$-$C_9$ substitués, carboxyalkylthio en $C_2$-$C_5$, le groupe amino, des groupes amino comportant un ou deux substituants choisis parmi

des radicaux alkyle en $C_1$-$C_4$, aryle en $C_6$-$C_{14}$, aryle en $C_6$-$C_{14}$ substitués, aralkyle en $C_7$-$C_9$, aralkyle en $C_7$-$C_9$ substitués et carboxy,

des groupes aryle en $C_6$-$C_{14}$, aryle en $C_6$-$C_{14}$ substitués, car-boxy, amidino, sulfo, alkyl($C_1$-$C_4$)-sulfinyle, alkyl($C_1$-$C_4$)-sulfonyle et des groupes hétérocycliques ayant de 5 à 14 atomes formant le cycle, dont de 1 à 5 sont des hétéroatomes d'azote et/ou d'oxygène et/ou de soufre, lesdits groupes aryloxy, aralkyloxy, arylthio, aralkylthio, aryle et aralkyle substitués comportant au moins un substituant choisi parmi des groupes alkyle en $C_1$-$C_4$, hydroxy, amino et alcoxy en $C_1$-$C_4$,
ou un sel pharmaceutiquement acceptable de celui-ci.

3. Procédé selon la revendication 2, dans lequel X représente un groupe alkylène en $C_1$-$C_5$ qui n'est pas substitué ou comporte un ou deux substituants choisi(s) parmi des groupes hydroxy, alcoxy en $C_1$-$C_4$, des groupes aryloxy dans lequel le cycle aryle possède de 6 à 14 atomes de carbone formant le cycle et n'est pas substitué ou comporte de un à trois substituants choisi(s) parmi des radicaux alkyle en $C_1$-$C_4$, hydroxy, amino et alcoxy en $C_1$-$C_4$; des groupes aralkyloxy en $C_7$-$C_9$ dans lesquels le fragment aryle n'est pas substitué ou comporte de un à trois substituants choisi(s) parmi des radicaux alkyle en $C_1$-$C_4$, hydroxy, amino et alcoxy en $C_1$-$C_4$; le groupe mercapto, des groupes alkylthio en $C_1$-$C_4$, des groupes arylthio dans lesquels le cycle aryle a de 6 à 14 atomes de carbone formant le cycle et n'est pas substitué ou comporte de un à trois substituants choisi(s) parmi des radicaux alkyle en $C_1$-$C_4$, hydroxy, amino et alcoxy en $C_1$-$C_4$; des groupes aralkylthio en $C_7$-$C_9$ dans lesquels le fragment aryle n'est pas substitué ou comporte de un à trois substituants choisi(s) parmi des radicaux alkyle en $C_1$-$C_4$, hydroxy, amino et alcoxy en $C_1$-$C_4$; des groupes carboxyalkylthio dans lesquels le fragment alkyle a de 1 à 4 atomes de carbone; le groupe amino; des groupes amino comportant un ou deux substituants alkyle en $C_1$-$C_4$; des groupes amino comportant un ou deux substituants aryle dans lesquels le noyau aryle a de 6 à 14 atomes de carbone formant le cycle et n'est pas substitué ou comporte de un à trois substituants choisi(s) parmi des radicaux alkyle en $C_1$-$C_4$, hydroxy, amino et alcoxy en $C_1$-$C_4$; des groupes amino comportant un ou deux substituants aralkyle en $C_7$-$C_9$ dans lesquels le fragment aryle n'est pas substitué ou comporte de un à trois substituants choisi(s) parmi des radicaux alkyle en $C_1$-$C_4$, hydroxy, amino et alcoxy en $C_1$-$C_4$; des groupes amino comportant un ou deux substituants acyle carboxyliques; des groupes aryle ayant de 6 à 14 atomes de carbone formant le cycle et n'étant pas substitués ou comportant de un à trois substituants choisi(s) parmi des radicaux alkyle en $C_1$-$C_4$, hydroxy, amino et alcoxy en $C_1$-$C_4$; le groupe carboxy et des groupes hétérocycliques ayant de 5 à 9 atomes formant le cycle, dont de 1 à 3 sont des hétéroatomes d'azote et/ou d'oxygène et/ou de soufre.

4. Procédé selon la revendication 2, dans lequel X représente un groupe alkylène en $C_1$-$C_5$ qui n'est pas substitué ou comporte un ou deux susbtituants choisi(s) parmi: des groupes hydroxy; alcoxy en $C_1$-$C_4$; mercapto; alkylthio en $C_1$-$C_4$; amino; des groupes amino comportant un ou deux substituants alkyle en $C_1$-$C_4$; des groupes amino comportant un ou deux substituants acyle carboxylique; des groupes aryle ayant de 6 à 14 atomes de carbone, dans lesquels le noyau aryle n'est pas substitué ou comporte de 1 à 3 substituants choisi(s) parmi des radicaux alkyle en $C_1$-$C_4$, hydroxy, amino et alcoxy en $C_1$-$C_4$; le

groupe carboxy et des groupes hétérocycliques ayant de 5 à 9 atomes formant le cycle, dont de 1 à 3 sont des hétéroatomes d'azote et/ou d'oxygène.

5. Procédé selon l'une quelconque des revendications 1 à 4, dans lequel le groupe N-acyle est: un groupe alcanoyle en $C_1$-$C_{18}$, alcénoyle en $C_3$-$C_8$, alcynoyle en $C_3$-$C_8$,un groupe acyle aromatique dans lequel le fragment aryle est un reste aryle carbocyclique en $C_6$-$C_{14}$ et n'est pas substitué ou comporte de 1 à 5 substituants choisi(s) parmi des radicaux alkyle en $C_1$-$C_4$, hydroxy, alcoxy en $C_1$-$C_4$, amino, sulfo et des atomes d'halogène; un groupe cycloalcanecarbonyle dans lequel le fragment cycloalcane est en $C_3$-$C_8$ et n'est pas substitué ou comporte au moins un substituant choisi parmi des radicaux alkyle en $C_1$-$C_4$ et phényle; un groupe acyle araliphatique dans lequel le noyau aryle est un noyau carbocyclique ayant de 6 à 14 atomes de carbone qui n'est pas substitué ou comporte de 1 à 5 substituants choisi(s) parmi des groupes alkyle en $C_1$ -$C_4$, hydroxy, alcoxy en $C_1$ -$C_4$, amino, sulfo et des atomes d'halogène, et dans lequel le fragment alkyle a de 1 à 4 atomes de carbone; un groupe acyle hétérocyclique qui comporte un système cyclique saturé ou insaturé, les cycles ayant 5 ou 6 atomes formant le cycle, dont de 1 à 3 sont des hétéroatomes d'azote et/ou de soufre et/ou d'oxygène et le cycle n'étant pas substitué ou ayant de 1 à 3 substituants choisi(s) parmi des groupes alkyle en $C_1$-$C_4$ et hydroxy; un groupe alcoxy($C_2$-$C_7$)-carbonyle; un groupe aralkyloxycarbonyle dans lequel le fragment aralkyle a de 7 à 9 atomes de carbone et n'est pas substitué ou comporte de 1 à 5 substituants choisi(s) parmi des radicaux amino, alkyle en $C_1$-$C_4$, alcoxy en $C_1$-$C_4$ et hydroxy; ou un groupe acyle dérivé d'un aminoacide par enlèvement d'OH hors du groupe carboxy et N-acylation du groupe amino par au moins un des groupes acyle mentionnés plus haut.

6. Procédé selon l'une quelconque des revendications 1 à 4, dans lequel le groupe N-acyle est: un groupe acyle aliphatique saturé ayant de 1 à 8 atomes de carbone; un groupe acyle aromatique dans lequel le fragment aryle a de 6 à 10 atomes de carbone formant le cycle et n'est pas substitué ou comporte de 1 à 3 substituants choisi(s) parmi des radicaux alkyle en $C_1$-$C_4$ et alcoxy en $C_1$-$C_4$; un groupe acyle alicyclique dans lequel le cycle cycloalcane a de 3 à 6 atomes de carbone; un groupe acyle araliphatique dans lequel le cycle aryle a de 6 à 10 atomes de carbone formant le cycle et le groupe alkyle a de 1 à 4 atomes de carbone, le cycle aryle n'étant pas susbtitué ou comportant de 1 à 3 substituants choisi(s) parmi des radicaux alkyle en $C_1$-$C_4$ et alcoxy en $C_1$-$C_4$; un groupe acyle hétérocyclique dans lequel le noyau hétérocyclique est saturé ou insaturé et a 5 ou 6 atomes formant le cycle, dont un est un hétéroatome d'azote, soufre ou oxygène; un groupe alcoxycarbonyle ayant au total de 2 à 7 atomes de carbone; un groupe aralkyloxycarbonyle dans lequel le fragment aralkyle a de 7 à 9 atomes de carbone et le cycle aryle n'est pas substitué ou comporte de 1 à 3 substituants choisi(s) parmi des radicaux alkyle en $C_1$-$C_4$ et alcoxy en $C_1$-$C_4$; ou un groupe acyle dérivé d'un aminoacide par enlèvement d'OH hors du groupe carboxy et N-acylation du groupe amino par au moins un des groupes acyle mentionnés cidessus.

7. Procédé selon l'une quelconque des revendications 1 à 4, dans lequel le groupe N-acyle est: un groupe acyle aromatique dans lequel le fragment aryle a de 6 à 10 atomes formant le cycle et n'est pas substitué ou comporte un seul substituant choisi parmi des radicaux alkyle en $C_1$-$C_4$, alcoxy en $C_1$-$C_4$, hydroxy et amino; un groupe acyle alicyclique dans lequel le fragment cycloalcane a de 3 à 6 atomes de carbone; un groupe acyle phénylaliphatique dans lequel le fragment phényle n'est pas substitué ou comporte un seul substituant alkyle en $C_1$-$C_4$ et dans lequel le fragment alkyle a de 1 à 4 atomes de carbone; un groupe alcoxycarbonyle ayant au total de 4 à 6 atomes de carbone; un groupe aralkyloxycarbonyle dans lequel le fragment aralkyle a de 7 à 9 atomes de carbone et comporte 0 ou 1 substituant choisi parmi des groupes alkyle en $C_1$-$C_4$ et alcoxy en $C_1$-$C_4$; ou un groupe acyle dérivé d'un aminoacide par enlèvement d'OH hors du groupe carboxy et N-acylation du groupe amino par au moins un des groupes acyle mentionnés ci-dessus.

8. Procédé selon l'une quelconque des revendications 1 à 4, dans lequel le groupe N-acyle est le groupe acétyle, benzoyle, cyclohexanecarbonyle, cyclopropanecarbonyle, hexanoyle, isobutyryle, crotonoyle, éthoxycarbonyle, 4-hydroxybenzoyle, anisoyle, 4-aminobenzoyle, naphtoyle, toluyle, benzyloxycarbonyle ou 4-méthoxybenzyloxycarbonyle.

9. Procédé selon l'une quelconque des revendications 1 et 5 à 8, dans lequel ledit aminoacide est la glycine, la $\beta$-alanine, l'acide 4-aminobutyrique, l'acide 5-aminovalérique, l'acide 6-aminohexanoïque, l'acide 8-aminooctanoïque, l'alanine, l'acide 2-aminobutyrique, la norvaline, la valine, la leucine, l'isoleucine, la norleucine, la tyrosine, l'O-méthyltyrosine, l'acide aspartique, l'acide glutamique, l'acide 4-carboxyglutamique, l'acide 3-méthylaspartique, l'acide 2-aminoadipique, l'acide 2-aminopimélique, l'acide 2-aminosubérique, l'acide 3-hydroxyaspartique, l'acide 3-hydroxyglutamique, l'acide 2,3-diaminopropionique, l'acide 2,4-diaminobutyrique, la 5-hydroxylysine, l'arginine, la $N^{\delta}$,$N^{\delta}$ -diméthylornithine, la $N^{\delta}$ -méthyllysine, la cystéine, la méthionine, l'éthionine, la S-carboxyméthylcystéine, la S-benzylcystéine, le S-oxyde de méthionine, le S-oxyde d'éthionine, le S,5-dioxyde de méthionine, l'acide cystéique, la sérine, l'O-méthylsérine, la thréonine, l'O-méthylthréonine, l'homothréonine, l'éthoxinine, la 3-méthoxyvaline, la 3-phénylsérine, la 3-méthyl-3-phénylalanine, l'histidine, le tryptophane, la 2-méthylalanine, la 2-méthylsérine, la 2-hydroxy-isoleucine, la 2-méthylméthionine, la 2-éthyl-2-phénylglycine, l'acide 3-aminobutyrique, l'acide 3-amino-4-méthylvalérique, l'acide 3-amino-3-phénylpropionique, l'acide 3-amino-2-hydroxypropionique ou l'acide 4-amino-3-hydroxybutyrique.

**10.** Procédé selon l'une quelconque des revendications 1 et 5 à 8, dans lequel ledit aminoacide est la glycine, la $\beta$-alanine, l'acide 4-aminobutyrique, l'acide 5-aminovalérique, l'acide 6-aminohexanoïque, l'acide 8-aminooctanoïque, l'alanine, la norvaline, la valine, la leucine, l'isoleucine, la norleucine, la $N^{\delta}$ ,$N^{\delta}$ -diméthylornithine, la méthionine, l'éthionine, l'O-méthylsérine, l'O-méthylthréonine, l'éthoxinine, la 3-méthoxyvaline, la 3-phénylsérine, la 3-méthyl-3-phénylalanine, l'histidine, la 2-méthylalanine, la 2-méthylsérine, la 2-hydroxy-isoleucine, la 2-éthylphénylglycine, l'acide 3-aminobutyrique, l'acide 3-amino-4-méthylvalérique ou l'acide 3-amino-3-phénylpropionique.

**11.** Procédé selon l'une quelconque des revendications 1 et 5 à 8, dans lequel ledit aminoacide est la $\beta$-alanine, l'acide 4-aminobutyrique, l'acide 5-aminovalérique l'acide 6-aminohexanoïque, l'alanine, la valine, la leucine, la norleucine, la méthionine, l'histidine ou la glycine.

**12.** Procédé selon l'une quelconque des revendications 1 et 5 à 8, dans lequel l'aminoacide est la leucylglycine, la glycyl-$\beta$-alanine, la glcylalanine, la valylalanine, la leucylalanine, la glycylvaline, l'alanylvaline, la leucylvaline, la valylleucine, la phénylalanylleucine, l'histidylleucine, la glycylphénylalanine, l'alanylphénylalanine, la leucylphénylalanine, la glycylméthionine, la valylméthionine, la glycylhistidine, l'alanylvalylglycine, la glycylalanylvaline, la glycylphénylalanylleucine ou la glycylglycylhistidine.

**13.** Procédé selon la revendication 1, dans lequel ledit aminoacide N-acylé est:
la N-(p-toluyl)-$\beta$-alanine
la N-(4-méthoxybenzoyl)-$\beta$-alanine
la N-(3-hydroxy-2-naphtoyl)-$\beta$-alanine
la N-benzoylglycyl-$\beta$-alanine
la N-benzoyl-$\beta$-alanine
l'acide N-benzoyl-5-aminovalérique
l'acide N-benzoyl-6-aminohexanoïque
l'acide N-cyclohexanecarbonyl-6-aminohexanoïque
l'acide N-(N-méthylnicotinoyl)-6-aminohexanoïque
l'acide N-benzoyl-8-amino-octanoïque
la N-benzoylalanine
la N-(1-naphtoyl)alanine
la N-benzoylvalylalanine
l'acide N-benzoyl-2-aminobutyrique
la N-benzoylnorvaline
la N-valérylvaline
la N-benzoylalanylvaline
la N-benzoylvaline
la N-benzoylleucine
la N-benzoylglycylphénylalanylleucine
la N-benzoylnorleucine
la N-benzoylglycylphénylalanine
la N-benzoylalanylphénylalanine
la N-cyclohexanecarbonylleucylphénylalanine
la N-benzoyl-O-méthyltyrosine
la N-benzoylméthionine
la N-phénylacétylméthionine

la N-benzoylvalylméthionine
la N-benzoyléthionine
la N-(4-méthoxybenzyloxycarbonyl)éthionine
la N-benzoylthréonine
la N-benzoylhistidine
la N-(p-toluyl)histidine
la N-(4-méthoxybenzoyl)histidine
l'acide N-(4-méthoxybenzoyl)-3-aminobutyrique ou
l'acide N-butyryl-3-amino-3-phénylpropionique.

**14.** Procédé selon l'une quelconque des revendications précédentes, dans lequel ledit antibiotique est un composé de formule générale I:

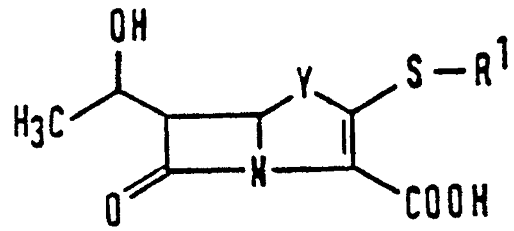

(I)

dans laquelle

Y représente un atome de soufre, le groupe méthylène ou un groupe méthylène comportant un ou deux substituants méthyle et/ou méthoxy; et

$R^1$ représente un groupe alkyle en $C_1$-$C_6$, un groupe alkyle en $C_1$-$C_6$ comportant au moins un des substituants (I) ou un groupe hétérocyclique ayant de 4 à 14 atomes formant le cycle, dont de 1 à 5 sont des hétéroatomes d'azote et/ou d'oxygène et/ou de soufre, ledit groupe hétérocyclique n'étant pas substitué ou comportant au moins un des substituants (II):

substituants (I):

des atomes d'halogène, le groupe amino, des groupes amino comportant au moins un des substituants (III), des groupes alkylidène($C_1$-$C_4$)-amino, amino-alkylidène-($C_1$-$C_4$)-amino, le groupe amidino, des groupes amidino comportant de 1 à 3 des substituants (III), des groupes hétérocycliques ayant de 4 à 14 atomes formant le cycle, dont de 1 à 5 sont des hétéroatomes d'azote, et/ou d'oxygène et/ou de soufre, ledit groupe hétérocyclique n'étant pas substitué ou comportant au moins un des substituants (II), le groupe imino, cyano, carbamoyle et des groupes carbamoyle comportant au moins un substituant choisi parmi des radicaux alkyle en $C_1$-$C_4$ et alcoxy en $C_1$-$C_4$;

substituants (II):

des groupes alcane($C_1$-$C_6$)-imidoyle, alkyle en $C_1$-$C_6$, des groupes alcoxyalkyle dans lesquels les fragments alkyle et alcoxy sont chacun en $C_1$-$C_4$, le groupe carbamoyle, des groupes carbamoyle comportant au moins un substituant choisi parmi des radicaux alkyle en $C_1$-$C_4$ et alcoxy en $C_1$-$C_4$, des groupes halogénoalkyle en $C_1$-$C_4$, des groupes acylimidoyle hétérocycliques dans lesquels le fragment hétérocyclique a de 5 à 9 atomes formant le cycle, dont de 1 à 3 sont des hétéroatomes d'azote et/ou d'oxygène et/ou de soufre, le groupe amidino, des groupes amidino comportant de 1 à 3 substituants (III), le groupe imino, des atomes d'oxygène, des groupes alcanoyle en $C_1$-$C_6$, alcane-($C_1$-$C_6$)-sulfonyle, alcane($C_1$-$C_6$)-sulfinyle, hydroximino, alcoximino en $C_1$-$c_4$, carbamoyloxy, des groupes carbamoyloxy comportant au moins un substituant choisi parmi des radicaux alkyle en $C_1$-$C_4$ et alcoxy en $C_1$-$C_4$, des groupes carbamoyloxyalkyle dans lesquels le fragment alkyle est en $C_1$-$C_4$ et le fragment carbamoyle n'est pas substitué ou comporte au moins un substituant choisi parmi des radicaux alkyle en $C_1$-$C_4$ et alcoxy en $C_1$-$C_4$ et des groupes iminoalkyle en $C_1$-$C_4$;

substituants (III):

des groupes alkyle en $C_1$-$C_6$, alcényle en $C_2$-$C_6$, alcynyle en $C_2$-$C_6$, lesdits groupes alkyle, alcényle et alcynyle comportant au moins un substituant choisi parmi des atomes d'halogène, des radicaux carbamoyloxy et des radicaux carbamoyloxy comportant au moins un substituant choisi parmi des restes alkyle en $C_1$-$C_4$ et alcoxy en $C_1$-$C_4$;

ou les sels pharmaceutiquement acceptables de celui-ci.

**15.** Procédé selon la revendication 14, dans lequel Y représente un atome de soufre, le groupe méthylène

ou le groupe $CH_3$-CH<, $CH_3$O-CH< ou $(CH_3)_2$C<.

**16.** Procédé selon la revendication 14 ou 15, dans lequel $R^1$ représente le groupe éthyle, 2-fluoroéthyle, 2-(aminométhylèneamino)éthyle, $N^1$,$N^1$,$N^2$-triméthylamidinométhyle, 3-pyrolidinyle, 1-formimidoyl-3-pyrrolidinyle, 1-acétimidoyl-3-pyrrolidinyle, 1-propionimidoyl-3-pyrrolidinyle, 2-méthyl-1,4,5,6-tétrahydro-5-pyrimidinyle, 2-méthoxyméthyl-1,4,5,6-tétrahydro-5-pyrimidinyle, 3-azétidinyle, 1-acétimidoyl-3-azétidinyle, $N^1$-méthyl-$N^1$-(2-propynyl)amidinométhyle, $N^1$-(2-fluoroéthyl)-$N^1$ méthylamidinométhyle, $N^1$-(3-fluoropropyl)-$N^1$-méthylamidinométhyle, $N^1$-méthyl-$N^1$-(2,2,2-trifluoroéthyl)amidinométhyle, 1-(3-azétidinyl)éthyle, 1-(1-acétimidoyl)-3-azétidinyl)-éthyle, 1,4,5,6-tétrahydro-2-pyrimidinylméthyle, 1-(4,5-dihydro-2-thiazolyl)éthyle, 5-carbamoyl-3-pyrrolidinyle, 1-acétimidoyl-5-carbamoyl-3-pyrrolidinyle, 2-chlorométhyl-1,4,5,6-tétrahydro-5-pyrimidinyle, 1-butyrimidoyl-3-pyrrolidinyle, 1-nicotinimidoyl-3-pyrrolidinyle, $N^1$,$N^1$-diallylamidinométhyle, $N^1$-méthyl-$N^1$-(2-propynyl)amidino, $N^1$-(2-fluoroéthyl)-$N^1$-méthylamidino, $N^1$-(3-fluoropropyl)-$N^1$-méthylamidino, $N^1$-méthyl-$N^1$-(2,2,2-trifluoroéthyl)amidino, $N^1$-allyl-$N^1$-méthylamidinométhyle, cyanométhyle, 2-cyanoéthyle, 1-cyanoéthyle, 2-cyano-1-méthyléthyle, 2-aminoéthyle, 1-carbamoyléthyle, 2-(1-aminoéthylidèneamino)éthyle, 1-amidino-3-pyrrolidinyle, 2-méthyl-1,3-diazabicyclo[3.3.0]oct-2-ène-7-yle, 2-méthoxyméthyl-1,3-diazabicyclo[3.3.0]oct-2-ène-7-yle, 5-imino-2-pyrrolidinyle, 2-imino-5-pipéridinyle, 1-acétimidoyl-5-méthylcarbamoyl-3-pyrrolidinyle, 1-acétimidoyl-5-méthoxycarbamoyl-3-pyrrolidinyle, 2-imino-2-(S-oxothiomorpholino)éthyle, 2-imino-2-(1,1-dioxo-1,3-thiazolidine-3-yl)éthyle, 2-imino-2-(S,S-dioxothiomorpholino)éthyle, 2-imino-2-(3,5-dioxo-1-pipérazinyl)-éthyle, 2-imino-2-(4-méthyl-3,5-dioxo-1-pipérazinyl)éthyle, 2-imino-2-(3-oxo-1-pipérazinyl)éthyle, 2-imino-2-(4-méthyl-3-oxo-1-pipérazinyl)éthyle, 2-imino-2-(4-acétyl-3-oxo-1-pipérazinyl)éthyle, 2-imino-2-(4-méthanesulfonyl-3-oxo-1-pipérazinyl)éthyle, $N^1$-(2-carbamoyloxyéthyl)-$N^1$-méthylamidinométhyle, 2-(3-hydroximino-1-pyrrolidinyl)-2-iminoéthyle, 2-imino-2-(3-méthoximino-1-pyrrolidinyl)éthyle, 2-(4-hydroximino-pipéridino)-2-iminoéthyle, 2-imino-2-(4-méthoximino- pipéridino)éthyle, 2-(3-carbamoyloxy-1-pyrrolidinyl)-2-iminoéthyle, 2-imino-2-(3-oxo-1-pipérazinyl)éthyle, 2-(3-carbamoylpipéridino)-2-iminoéthyle, 2-(3-carbamoyloxypipéridino)-2-iminoéthyle, 2-(2-carbamoyloxy-1-pyrrolidinyl)2-iminoéthyle, 2-(2-carbamoyloxyméthyl-1-pyrrolidinyl)-2iminoéthyle, 2-(4-carbamoyloxypipéridino)-2-iminoéthyle, 2-(4-formyl-1-pipérazinyl)-2-iminoéthyle, 2-(4-acétyl-1-pipérazinyl)-2-iminoéthyle, 1-formyl-3-azétidinyle, 1-imino-méthyl-3-azétidinyle, 1-méthyl-4-pipéridyle, 1-acétimidoyl-4pipéridyle ou 1-acétyl-3-pyrrolidinyle.

**17.** Procédé selon la revendication 14, dans lequel ledit antibiotique est:
l'acide (5R,6S)-2- 2-[(aminométhylène)amino]éthylthio -6-[1(R)-hydroxyéthyl]-2-carbapénème-3-carboxylique
l'acide (5R,6S)-2-[(3S)-1-acétimidoylpyrrolidine-3-ylthio]-6-[1(R)-hydroxyéthyl]-2-carbapénème-3-carboxylique
l'acide (5R,6S)-2-[(3R)-1-acétimidoylpyrrolidine-3-ylthio]-6-[1(R)-hydroxyéthyl]-2-carbapénème-3-carboxylique
l'acide (5R,6S)-2-[(3R)-1-acétimidoylpyrrolidine-3-ylthio]-6-[1(R)-hydroxyéthyl]-1(S)-méthyl-2-carbapénème-3-carboxylique
l'acide (5R,6S)-2-[(3S)-1-acétimidoylpyrrolidine-3-ylthio]-6-[1(R)-hydroxyéthyl]-1(R)-méthyl-2-carbapénème-3-carboxylique
l'acide (5R,6S)-2-[(3S)-1-acétimidoylpyrrolidine-3-ylthio]-6-[1(R)-hydroxyéthyl]-1(S)-méthyl-2-carbapénème-3-carboxylique
ou
l'acide (5R,6S)-2-[(3S)-1-acétimidoyl-5(S)-carbamoylpyrrolidine-3-ylthio]-6-[1(R)-hydroxyéthyl]-2-carbapénème-3-carboxylique.

**18.** Procédétion selon l'une quelconque des revendications précédentes, dans lequel le rapport pondéral dudit aminoacide N-acylé audit antibiotique va de 0,1:1 à 4:1.

**19.** Procédé selon l'une quelconque des revendications précédentes, qui comprend: la dissolution dudit aminoacide N-acylé dans de l'eau, l'addition et la dissolution dudit antibiotique dans la solution résultante, et éventuellement la lyophilisation de la solution pour l'obtention d'un mélange pulvérulent.

**20.** Procédé selon la revendication 19, dans lequel on dissout l'aminoacide N-acylé en le dispersant dans de l'eau et en ajoutant une quantité suffisante d'une base pour ajuster le pH à une valeur allant de 5,5 à 9.

**21.** Utilisation, pour la fabrication d'un médicament pour le traitement d'infections bactériennes, de
(a) un antibiotique pénème ou carbapénème,
en association avec
(b) un dérivé N-acylé pharmaceutiquement acceptable d'un aminoacide dans lequel le groupe amino et le groupe carboxy sont liés à un atome de carbone saturé ou à une chaîne carbonée aliphatique saturée, ou un sel de celui-ci, étant entendu que l'aminoacide n'est pas l'ornithine, la lysine, la phénylalanine ni la phénylglycine seule.

**22.** Utilisation selon la revendication 22, dans laquelle ledit aminoacide N-acylé est:
la N-(p-toluyl)-$\beta$-alanine
la N-(4-méthoxybenzoyl)-$\beta$-alanine
la N-(3-hydroxy-2-naphtoyl)-$\beta$-alanine
la N-benzoylglycyl-$\beta$-alanine
la N-benzoyl-$\beta$-alanine
l'acide N-benzoyl-5-aminovalérique
l'acide N-benzoyl-6-aminohexanoïque
l'acide N-cyclohexanecarbonyl-6-aminohexanoïque
l'acide N-(N-méthylnicotinoyl)-6-aminohexanoïque
l'acide N-benzoyl-8-amino-octanoïque
la N-benzoylalanine
la N-(1-naphtoyl)alanine
la N-benzoylvalylalanine
l'acide N-benzoyl-2-aminobutyrique
la N-benzoylnorvaline
la N-valérylvaline
la N-benzoylalanylvaline
la N-benzoylvaline
la N-benzoylleucine
la N-benzoylglycylphénylalanylleucine
la N-benzoylnorleucine
la N-benzoylglycylphénylalanine
la N-benzoylalanylphénylalanine
la N-cyclohexanecarbonylleucylphénylalanine
la N-benzoyl-0-méthyltyrosine
la N-benzoylméthionine
la N-phénylacétylméthionine
la N-benzoylvalylméthionine
la N-benzoyléthionine
la N-(4-méthoxybenzyloxycarbonyl)éthionine
la N-benzoylthréonine
la N-benzoylhistidine
la N-(p-toluyl)histidine
la N-(4-méthoxybenzoyl)histidine
l'acide N-(4-méthoxybenzoyl)-3-aminobutyrique ou
l'acide N-butyryl-3-amino-3-phénylpropionique.

**23.** Utilisation selon la revendication 21 ou 22, dans laquelle ledit antibiotique est:
l'acide (5R,6S)-2- 2-[(aminométhylène)amino]éthylthio -6-[1(R)-hydroxyéthyl]-2-carbapénème-3-carboxylique
l'acide (5R,6S)-2-[(3S)-1-acétimidoylpyrrolidine-3-ylthiol-6-[1(R)-hydroxyéthyl]-2-carbapénème-3-carboxylique
l'acide (5R,6S)-2-[(3R)-1-acétimidoylpyrrolidine-3-ylthio]-6-[1(R)-hydroxyéthyl]-2-carbapénème-3-carboxylique
l'acide (5R,6S)-2-[(3R)-1-acétimidoylpyrrolidine-3-ylthio]-6-[1(R)-hydroxyéthyl]-1(S)-méthyl-2-carbapénème-3-carboxylique
l'acide (5R,6S)-2-[(3S)-1-acétimidoylpyrrolidine-3-ylthio]-6-[1(R)-hydroxyéthyl]-1(R)-méthyl-2-carbapénème-3-carboxylique
l'acide (5R,6S)-2-[(3S)-1-acétimidoylpyrrolidine-3-ylthio]-6-[1(R)-hydroxyéthyl]-1(S)-méthyl-2-carbapénème-3-carboxylique
ou
l'acide (5R,6S)-2-[(3S)-1-acétimidoyl-5(S)-carbamoylpyrrolidine-3-ylthio]-6-[1(R)-hydroxyéthyl]-2-carbapénème-3-carboxylique.

**Patentansprüche**
**Patentansprüche für folgende Vertragsstaaten : GB, DE, FR, IT, CH, BE, NL, SE, LU**

1. Mischung, welche

ein Penem- oder Carbapenemantibiotikum und

ein pharmazeutisch annehmbares N-acyliertes Derivat einer Aminosäure mit an eine gesättigte aliphatische Kohlenstoffkette oder an ein Kohlenstoffatom gebundener Aminogruppe und Carboxylgruppe oder ein Salz dieser Aminosäure mit der Maßgabe enthält,
daß, falls die Mischung nur eine einzige Aminosäure enthält, die Aminosäure nicht Ornithin, Lysin, Phenylalanin oder Phenylglycin ist,
wobei die Menge an Aminosäurederivat ausreicht eine allfällige renale Toxizität des Antibiotikums abzuschwächen und vorzugsweise das Gewichtsverhältnis der N-acylierten Aminosäure zum Antibiotikum 0,1:1 bis 4:1 beträgt.

2. Mischung nach Anspruch 1, worin die Aminosäure eine Verbindung der allgemeinen Formel II

$H_2N$-X-COOH (II)

ist, worin X eine $C_1$-$C_{10}$-Alkylengruppe oder eine $C_1$-$C_{10}$-Alkylengruppe mit zumindest einem aus Hydroxygruppen, $C_1$-$C_4$-Alkoxygruppen, $C_6$-$C_{14}$-Aryloxygruppen, substituierten $C_6$-$C_{14}$-Aryloxygruppen, $C_7$-$C_9$-Aralkyloxygruppen, substituierten $C_7$-$C_9$-Aralkyloxygruppen, Mercaptogruppen, $C_1$-$C_4$-Alkylthiogruppen, $C_6$-$C_{14}$-Arylthiogruppen, substituierten $c_6$-$c_{14}$-Arylthiogruppen, $C_7$-$C_9$-Aralkylthiogruppen, substituierten $C_7$-$C_9$-Aralkylthiogruppen, $C_2$-$C_5$-Carboxyalkylthiogruppen, Aminogruppen, Aminogruppen mit einem oder zwei aus

$C_1$-$C_4$-Alkylgruppen, $C_6$-$C_{14}$-Arylgruppen, substituierten $C_6$-$C_{14}$-Arylgruppen, $C_7$-$C_9$-Aralkylgruppen, substituierten $C_7$-$C_9$-Aralkylgruppen und von Carbonsäuren abgeleiteten Acylgruppen ausgewählten Substituenten,

$C_6$-$C_{14}$-Arylgruppen, substituierten $C_6$-$C_{14}$-Arylgruppen, Carboxygruppen, Amidinogruppen, Sulfogruppen, $C_1$-$C_4$-Alkylsulfinylgruppen, $C_1$-$C_4$-Alkylsulfonylgruppen und heterocyclischen Gruppen mit 5 bis 14 Ringatomen, von welchen 1 bis 5 von Stickstoff und/oder Sauerstoff und/oder Schwefel gebildete Heteroatome sind, ausgewählten Substituenten, wobei die substituierten Aryloxy-, Aralkyloxy-, Arylthio-, Aralkylthio-, Aryl- und Aralkylgruppen zumindest einen aus $C_1$-$C_4$-Alkylgruppen, Hydroxygruppen, Aminogruppen und $C_1$-$C_4$-Alkoxygruppen ausgewählten Substituenten aufweisen,

oder ein pharmazeutisch annehmbares Salz hievon ist.

3. Mischung nach Anspruch 2, worin X eine $C_1$-$C_5$-Alkylengruppe darstellt, welche unsubstituiert ist oder einen oder zwei Substituenten aufweist, die aus Hydroxygruppen, $C_1$-$C_4$-Alkoxygruppen, Aryloxygruppen mit 6 bis 14 Ringkohlenstoffatomen im unsubstituierten oder 1 bis 3 aus $C_1$-$C_4$-Alkylgruppen, Hydroxygruppen, Aminogruppen und $C_1$-$C_4$-Alkoxygruppen ausgewählte Substituenten besitzenden Arylring, $C_7$-$C_9$-Aralkyloxygruppen mit unsubstituiertem oder 1 bis 3 aus $C_1$-$C_4$-Alkylgruppen Hydroxygruppen, Aminogruppen und $C_1$-$C_4$-Alkoxygruppen ausgewählte Substituenten besitzendem Arylrest, Mercaptogruppen, $C_1$-$C_4$-Alkylthiogruppen, Arylthiogruppen mit 6 bis 14 Ringkohlenstoffatomen im unsubstituierten oder 1 bis 3 aus $C_1$-$C_4$-Alkylgruppen, Hydroxygruppen, Aminogruppen und $C_1$-$C_4$-Alkoxygruppen ausgewählte Substituenten besitzenden Arylring, $C_7$-$C_9$-Aralkylthiogruppen mit unsubstituiertem oder 1 bis 3 aus $C_1$-$C_4$-Alkylgruppen, Hydroxygruppen, Aminogruppen und $C_1$-$C_4$-Alkoxygruppen ausgewählte Substituenten aufweisenden Arylring, Carboxyalkylthiogruppen mit 1 bis 4 Kohlenstoffatomen im Alkylteil, Aminogruppen, Aminogruppen mit einem oder mit zwei $C_1$-$C_4$-Alkylsubstituenten, Aminogruppen mit einem oder zwei Arylsubstituenten, worin der Arylring 6 bis 14 Ringkohlenstoffatome besitzt und unsubstituiert ist oder 1 bis 3 aus $C_1$-$C_4$-Alkylgruppen, Hydroxygruppen, Aminogruppen und $C_1$-$C_4$-Alkoxygruppen ausgewählte Substituenten aufweist, Aminogruppen mit einem oder zwei $C_7$-$C_9$-Aralkylsubstituenten mit unsubstituiertem oder 1 bis 3 aus $C_1$-$C_4$-Alkylgruppen, Hydroxygruppen, Aminogruppen und $C_1$-$C_4$-Alkoxygruppen ausgewählten Substituenten im Arylteil, Aminogruppen mit einem oder zwei von Carbonsäuren abgeleiteten Acylgruppen als Substituenten, Arylgruppen mit 6 bis 14 Ringkohlenstoffatomen, welche unsubstituiert sind oder 1 bis 3 aus $C_1$-$C_4$-Alkylgruppen, Hydroxygruppen, Aminogruppen und $C_1$-$C_4$-Alkoxygruppen ausgewählte Substituenten aufweisen, Carboxygruppen und heterocyclische Gruppen mit 5 bis 9 Ringatomen, von welchen 1 bis 3 von Stickstoff und/oder Sauerstoff und/oder Schwefel gebildete Heteroatome sind, ausgewählt sind.

4. Mischung nach Anspruch 2, worin X eine $C_1$-$C_5$-Alkylengruppe darstellt, die unsubstituiert ist oder 1 oder 2 Substituenten aufweist, die aus Hydroxygruppen, $C_1$-$C_4$-Alkoxygruppen, Mercaptogruppen, $C_1$-$C_4$-Alkylthiogruppen, Aminogruppen, Aminogruppen mit einem oder zwei $C_1$-$C_4$-Alkylsubstituenten, Aminogruppen mit einem oder zwei von Carbonsäuren abgeleiteten Acylsubstituenten, Arylgruppen mit 6 bis 14 Kohlenstoffatomen und unsubstituiertem oder 1 bis 3 aus $C_1$-$C_4$-Alkylgruppen, Hydroxygruppen, Aminogruppen und $C_1$-$C_4$-Alkoxygruppen ausgewählte Substituenten aufweisendem Arylring, Carboxygruppen und heterocyclischen Gruppen mit 5 bis 9 Ringatomen, von welchen 1 bis 3 von Stickstoff und/oder Sauerstoff gebildete Heteroatome sind, ausgewählt sind.

5. Mischung nach irgendeinem der Ansprüche 1 bis 4, worin die N-Acylgruppe eine $C_1$-$C_{18}$-Alkanoylgruppe, eine $C_3$-$C_8$-Alkenoylgruppe, eine $C_3$-$C_8$-Alkinoylgruppe, eine aromatische Acylgruppe mit carbocyclischem $C_6$-$C_{14}$-Arylteil, welcher unsubstituiert ist oder 1 bis 5 aus $C_1$-$C_4$-Alkylgruppen, Hydroxygruppen, $C_1$-$C_4$-Alkoxygruppen, Aminogruppen, Sulfogruppen und Halogenatomen ausgewählte Substituenten aufweist, eine Cycloalkancarbonylgruppe mit unsubstituiertem oder zumindest einen aus $C_1$-$C_4$-Alkylgruppen und Phenylgruppen ausgewählte Substituenten aufweisendem $C_3$-$C_8$-Cycloalkanteil, eine araliphatische Acylgruppe mit einem 6 bis 14 Kohlenstoffatome aufweisenden carbocyclischen Ring als Arylring, welcher unsubstituiert ist oder 1 bis 5 aus $C_1$-$C_4$-Alkylgruppen, Hydroxygruppen, $C_1$-$C_4$-Alkoxygruppen, Aminogruppen, Sulfogruppen und Halogenatomen ausgewählte Substituenten aufweist, und mit 1 bis 4 Kohlenstoffatomen im Alkylteil, eine heterocyclische Acylgruppe mit gesättigtem oder ungesättigtem Ringsystem und mit 5 oder 6 Ringatomen,von welchen 1 bis 3 von Stickstoff und/oder Schwefel und/oder Sauerstoff gebildete Heteroatome sind, wobei der Ring unsubstituiert ist oder 1 bis 3 aus $C_1$-$C_4$-Alkylgruppen und Hydroxygruppen ausgewählte Substituenten aufweist, eine $C_2$-$C_7$-Alkoxycarbonylgruppe, eine Aralkyloxycarbonylgruppe mit 7 bis 9 Kohlenstoffatome aufweisendem Aralkylteil, welcher unsubstituiert ist oder 1 bis 5 aus Aminogruppen, $C_1$-$C_4$-Alkylgruppen, $C_1$-$C_4$-Alkoxygruppen und Hydroxygruppen ausgewählte Substituenten aufweist oder eine Acylgruppe ist, die von einer Aminosäure durch Abspalten der OH-Gruppe von der Carboxylgruppe und N-Acylieren der Aminogruppe mit zumindest einer der oben genannten Acylgruppen abgeleitet worden ist.

6. Mischung nach irgendeinem der Ansprüche 1 bis 4, worin die N-Acylgruppe eine gesättigte aliphatische Acylgruppe mit 1 bis 8 Kohlenstoffatomen, eine aromatische Acylgruppe mit 6 bis 10 Ringkohlenstoffatomen im unsubstituierten oder 1 bis 3 aus $C_1$-$C_4$-Alkylgruppen und $C_1$-$C_4$-Alkoxygruppen ausgewählte Substituenten aufweisenden Arylrest, eine alicyclische Acylgruppe mit 3 bis 6 Kohlenstoffatomen im Cycloalkanring, eine araliphatische Acylgruppe mit 6 bis 10 Ringkohlenstoffatomen im Arylring und mit 1 bis 4 Kohlenstoffatomen in der Alkylgruppe und mit unsubstituiertem oder 1 bis 3 aus $C_1$-$C_4$-Alkylgruppen und $C_1$-$C_4$-Alkoxygruppen ausgewählte Substituenten aufweisendem Arylring, eine heterocyclische Acylgruppe mit gesättigtem oder ungesättigtem und 5 oder 6 Ringatome, von welchen eines ein von Stickstoff, Schwefel oder Sauerstoff gebildetes Heteroatom ist, aufweisendem heterocyclischem Ring, eine Alkoxycarbonylgruppe mit insgesamt 2 bis 7 Kohlenstoffatomen, eine Aralkyloxycarbonylgruppe mit einem 7 bis 9 Kohlenstoffatome aufweisenden Aralkylrest und mit unsubstituiertem oder 1 bis 3 aus $C_1$-$C_4$-Alkylgruppen und $C_1$-$C_4$-Alkoxygruppen ausgewählte Substituenten aufweisendem Arylring oder eine Acylgruppe ist, die von einer Aminosäure durch Abspalten der OH-Gruppe von der Carboxylgruppe und durch N-Acylieren der Aminogruppe mit zumindest einer der oben erwähnten Acylgruppen abgeleitet worden ist.

7. Mischung nach irgendeinem der Ansprüche 1 bis 4, worin die N-Acylgruppe eine aromatische Acylgruppe mit einem 6 bis 10 Ringatome besitzenden und unsubstituierten oder einen einzigen aus $C_1$-$C_4$-Alkylgruppen, $C_1$-$C_4$-Alkoxygruppen, Hydroxygruppen und Aminogruppen ausgewählte Substituenten aufweisenden Arylring, eine alicyclische Acylgruppe mit einem 3 bis 6 Kohlenstoffatome

aufweisenden Cycloalkanrest, eine phenylaliphatische Acylgruppe mit unsubstituierter oder einen einzigen $C_1$-$C_4$-Alkylsubstituenten aufweisender Phenylgruppe und mit einem 1 bis 4 Kohlenstoffatome aufweisenden Alkylteil, eine Alkoxycarbonylgruppe mit insgesamt 4 bis 6 Kohlenstoffatomen, eine Aralkyloxycarbonylgruppe mit einem 7 bis 9 Kohlenstoffatome aufweisenden und 0 oder 1 aus $C_1$-$C_4$-Alkylgruppen und $C_1$-$C_4$-Alkoxygruppen ausgewählte Substituenten aufweisenden Aralkylteil oder eine Acylgruppe ist, die von einer Aminosäure durch Abspalten der OH-Gruppe aus der Carboxylgruppe und N-Acylieren der Aminogruppe mit zumindest einer der oben erwähnten Acylgruppen erhalten worden ist.

8. Mischung nach irgendeinem der Ansprüche 1 bis 4, worin die N-Acylgruppe eine Acetyl-, Benzoyl-, Cyclohexancarbonyl-, Cyclopropancarbonyl-, Hexanoyl-, Isobutyryl-, Crotonoyl-, Äthoxycarbonyl-, 4-Hydroxybenzoyl-, Anisoyl-, 4-Aminobenzoyl-, Naphthoyl-, Toluoyl-, Benzyloxycarbonyl-oder 4-Methoxybenzyloxycarbonylgruppe ist.

9. Mischung nach irgendeinem der Ansprüche 1 und 5 bis 8, worin die Aminosäure Glycin, ß-Alanin, 4-Aminobuttersäure, 5-Aminovaleriansäure, 6-Aminohexansäure, 8-Aminooctansäure, Alanin, 2-Aminobuttersäure, Norvalin, Valin, Leucin, Isoleucin, Norleucin, Tyrosin, O-Methyltyrosin, Asparaginsäure, Glutaminsäure, 4-Carboxyglutaminsäure, 3-Methylasparaginsäure, 2-Aminoadipinsäure, 2-Aminopimelinsäure, 2-Aminosuberinsäure, 3-Hydroxyasparaginsäure, 3-Hydroxyglutaminsäure, 2,3-Diaminopropionsäure, 2,4-Diaminobuttersäure, 5-Hydroxylysin, Arginin, $N^{\delta}$ ,$N^{\epsilon}$ -Dimethylornithin, $N^{\epsilon}$-Methyllysin, Cystein, Methionin, Äthionin, S-Carboxymethylcystein, S-Benzylcystein, Methionin-S-oxid, Äthionin-S-oxid, Methionin-S,S-dioxid, Cysteinsäure, Serin, O-Methylserin, Threonin, O-Methylthreonin, Homothreonin, Äthoxinin, 3-Methoxyvalin, 3-Phenylserin, 3-Methyl-3-phenylalanin, Histidin, Tryptophan, 2-Methylalanin, 2-Methylserin, 2-Hydroxyisoleucin, 2-Methylmethionin, 2-Äthyl-2-phenylglycin, 3-Aminobuttersäure, 3-Amino-4-methyl-valeriansäure, 3-Amino-3-phenylpropionsäure, 3-Amino-2-hydroxypropionsäure oder 4-Amino-3-hydroxybuttersäure ist.

10. Mischung nach irgendeinem der Ansprüche 1 und 5 bis 8, worin die Aminosäure Glycin, ß-Alanin, 4-Aminobuttersäure, 5-Aminovaleriansäure, 6-Aminohexansäure, 8-Aminooctansäure, Alanin, Norvalin, Valin, Leucin, Isoleucin, Norleucin, $N^{\delta}$, $N^{\epsilon}$ -dimethylornithin, Methionin, Äthionin, O-Methylserin, O-Methylthreonin, Äthoxinin, 3-Methoxyvalin, 3-Phenylserin, 3-Methyl-3-phenylalanin, Histidin, 2-Methylalanin, 2-Methylserin, 2-Hydroxyisoleucin, 2-Äthylphenylglycin, 3-Aminobuttersäure, 3-Amino-4-methylvaleriansäure, oder 3-Amino-3--phenylpropionsäure ist.

11. Mischung nach irgendeinem der Ansprüche 1 und 5 bis 8, worin die Aminosäure ß-Alanin, 4-Aminobuttersäure, 5-Aminovaleriansäure, 6-Aminohexansäure, Alanin, Valin, Leucin, Norleucin, Methionin, Histidin oder Glycin ist.

12. Mischung nach irgendeinem der Ansprüche 1 und 5 bis 8, worin die Amininosäure Leucylglycin, Glycyl-ß-alanin, Glycylalanin, Valylalanin, Leucylalanin, Glycylvalin, Alanylvalin, Leucylvalin, Valylleucin, Phenylalanylleucin, Histidylleucin, Glycylphenylalanin, Alanylphenylalanin, Leucylphenylalanin, Glycylmethionin, Valylmethionin, Glycylhistidin, Alanylvalylglycin, Glycylalanylvalin, Glycylphenylalanylleucin oder Glycylglycylhistidin ist.

13. Mischung nach Anspruch 1, worin die N-acylierte Aminosäure
N-(p-Toluoyl)-ß-alanin
N-(4-Methoxybenzoyl)-ß-alanin
N-(3-Hydroxy-2-naphthoyl)-ß-alanin
N-Benzoylglycyl -ß-alanin
N-Benzoyl-ß-alanin
N-Benzoyl-5-aminovaleriansäure
N-Benzoyl-6-aminohexansäure
N-Cyclohexancarbonyl-6-aminohexansäure
N-(N-Methylnicotinoyl)-6-aminohexansäure
N-Benzoyl-8-aminooctansäure
N-Benzoylalanin
N-(1-Naphthoyl)alanin
N-Benzoylvalylalanin

N-Benzoyl-2-aminobuttersäure
N-Benzoylnorvalin
N-Valerylvalin
N-Benzoylalanylvalin
N-Benzoylvalin
N-Benzoylleucin
N-Benzoylglycylphenylalanylleucin
N-Benzoylnorleucin
N-Benzoylglycylphenylalanin
N-Benzoylalanylphenylalanin
N-Cyclohexancarbonylleucylphenylalanin
N-Benzoyl -O-methyltyrosin
N-Benzoylmethionin
N-Phenylacetylmethionin
N-Benzoylvalylmethionin
N-Benzoyläthionin
N-(4-Methoxybenzyloxycarbonyl)äthionin
N-Benzoylthreonin
N-Benzoylhistidin
N-(p-Toluoyl)histidin
N-(4-Methoxybenzoyl)histidin
N-(4-Methoxybenzoyl)-3-aminobuttersäure
oder
N-Butyryl-3-amino-3-phenylpropionsäure
ist.

**14.** Mischung nach irgendeinem der vorhergehenden Ansprüche, worin das Antibiotikum eine Verbindung der allgemeinen Formel I

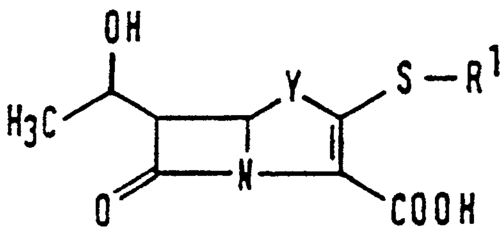

(I)

ist, in welcher

Y ein Schwefelatom, eine Methylengruppe oder eine Methylengruppe mit 1 oder 2 Methylgruppen und/oder Methoxygruppen als Substituenten bedeutet und

$R^1$ eine $C_1$-$C_6$-Alkylgruppe, eine $C_1$-$C_6$-Alkylgruppe mit zumindest einem der Substituenten (i) oder eine heterocyclische Gruppe mit 4 bis 14 Ringatomen, von welchen 1 bis 5 von Stickstoff und/oder Sauerstoff und/oder Schwefel gebildete Heteroatome sind, darstellt, wobei die heterocyclische Gruppe unsubstituiert ist oder zumindest einen der Substituenten (ii) aufweist,

wobei folgendes gilt:

Substituenten (i):
Halogenatome, Aminogruppen, Aminogruppen mit zumindest einem der Substituenten (iii), $C_1$-$C_4$-Alkylidenaminogruppen, $C_1$-$C_4$-Aminoalkylidenaminogruppen, Amidinogruppen, Amidinogruppen mit 1 bis 3 der Substituenten (iii), heterocyclische Gruppen mit 4 bis 14 Ringatomen, von welchen 1 bis 5 von Stickstoff und/oder Sauerstoff und/oder Schwefel gebildete Heteroatome sind, und hiebei die heterocyclische Gruppe unsubstituiert ist oder zumindest einen der Substituenten (ii) aufweist,

Iminogruppen, Cyanogruppen, Carbamoylgruppen und Carbamoylgruppen mit zumindest einem aus $C_1$-$C_4$-Alkylgruppen und $C_1$-$C_4$-Alkoxygruppen ausgewählten Substituenten
Substituenten (ii):
$C_1$-$C_6$-Alkanimidoylgruppen, $C_1$-$C_6$-Alkylgruppen, Alkoxyalkylgruppen mit $C_1$-$C_4$-Alkoxyteil und $C_1$-$C_4$-Alkylteil, Carbamoylgruppen, Carbamoylgruppen mit zumindest einem aus $C_1$-$C_4$-Alkylgruppen und $C_1$-$C_4$-Alkoxygruppen ausgewählten Substituenten, $C_1$-$C_4$-Halogenalkylgruppen, Heterocyclylacylimidoylgruppen mit 5 bis 9 Ringatomen, von welchen 1 bis 3 von Stickstoff und/oder Sauerstoff und/oder Schwefel gebildete Heteroatome sind, Amidinogruppen, Amidinogruppen mit 1 bis 3 der Substituenten (iii), Iminogruppen, Sauerstoffatome, $C_1$-$C_6$-Alkanoylgruppen, $C_1$-$C_6$-Alkansulfonylgruppen, $C_1$-$C_6$-Alkansulfinylgruppen, Hydroximinogruppen, $C_1$-$C_4$-Alkoximinogruppen, Carbamoyloxygruppen, Carbamoyloxygruppen mit zumindest einem aus $C_1$-$C_4$-Alkylgruppen und $C_1$-$C_4$-Alkoxygruppen ausgewählten Substituenten, Carbamoyloxyalkylgruppen mit einer $C_1$ -$C_4$-Alkylgruppe als Alkylteil und unsubstituiertem oder durch zumindest einen aus $C_1$-$C_4$-Alkylgruppen und $C_1$-$C_4$-Alkoxygruppen ausgewählten Substituenten substituiertem Carbamoylteil und $C_1$-$C_4$-Iminoalkylgruppen,
Substituenten (iii):
$C_1$-$C_6$-Alkylgruppen, $C_2$-$C_6$-Alkenylgruppen, $C_2$-$C_6$--Alkinylgruppen und - diese Alkyl-, Alkenyl- und Alkinylgruppen mit zumindest einem Substituenten, welcher aus Halogenatomen, Carbamoyloxygruppen und Carbamoyloxygruppen mit zumindest einem aus $C_1$-$C_4$-Alkylgruppen und $C_1$-$C_4$-Alkoxygruppen ausgewählten Substituenten ausgewählt ist,

und ein pharmazeutisch annehmbares Salz hievon ist.

15. Mischung nach Anspruch 14, worin Y ein Schwefelatom, eine Methylengruppe oder die Gruppe $CH_3$-CH< , $CH_3O$-CH< oder $(CH_3)_2$C< bedeutet.

16. Mischung nach Anspruch 14 oder Anspruch 15, worin $R^1$ eine Äthyl-, 2-Fluoräthyl-, 2-(Aminomethylenamino)äthyl-, $N^1$,$N^1$-Dimetbylamidinomethyl-, $N^1$,$N^1$,$N^2$-Trimethylamidinomethyl, 3-Pyrrolidinyl-, 1-Formimidoyl-3-pyrrolidinyl-, 1-Acetimidoyl-3-pyrrolidinyl-, 1-Propionimidoyl-3-pyrrolidinyl-, 2-Methyl-1,4,5,6,-tetrahydro-5-pyrimidinyl-, 2-Methoxymethyl-1,4,5,6-tetrahydro-5-pyrimidinyl-, 3-Acetidinyl-, 1-Acetimidoyl-3-acetidinyl-, $N^1$-Methyl-$N^1$-(2-propinyl)amidinomethyl-, $N^1$-(2-Fluoräthyl)-$N^1$-methylamidinomethyl-, $N^1$-(3-Fluorpropyl)-$N^1$-methylamidinomethyl-, $N^1$-Methyl-$N^1$ -(2,2,2-trifluoräthyl)amidinomethyl-, 1-(3-Acetidinyl)äthyl-, 1-(1-Acetimidoyl-3-acetidinyl)äthyl-, 1,4,5,6-Tetrahydro-2-pyrimidinylmethyl-, 1-(4,5-Dihydro-2--thiazolyl)äthyl-, 5-Carbamoyl-3-pyrrolidinyl-, 1-Acetimidoyl-5-carbamoyl-3-pyrrolidinyl-, 2-Chlormethyl-1,4,5,6-tetrahydro-5-pyrimidinyl-, 1-Butyrimidoyl-3-pyrrolidinyl-, 1-Nicotinimidoyl-3-pyrrolidinyl-, $N^1$,$N^1$-Diallylamidinomethyl-, $N^1$-Methyl-$N^1$-(2-propinyl)-amidino-, $N^1$-(2-Fluoräthyl)-$N^1$-methylamidino-, $N^1$-(3-Fluorpropyl)-$N^1$-methylamidino-, $N^1$-Methyl-$N^1$ -(2,2,2-trifluoräthyl)amidino-, $N^1$-Allyl-$N^1$-methylamidinomethyl-, Cyanomethyl-, 2-Cyanoäthyl-, 1-Cyanoäthyl-, 2-Cyano-1-methyläthyl-, 2-Aminoäthyl-, 1-Carbamoyläthyl-, 2-(1-Aminoäthylidenamino)-äthyl-, 1-Amidino-3-pyrrolidinyl-, 2-Methyl-1,3-diazabicyclo [3.3.0]-oct-2-en-7-yl-, 2-Methoxymethyl-1,3-diazabicyclo[3.3.0]oct-2-en-7-yl-, 5-Imino-2-pyrrolidinyl-, 2-Imino-5-piperidinyl-, 1-Acetimidoyl-5-methylcarbamoyl-3-pyrrolidinyl-, 1-Acetimidoyl-5-methoxycarbamoyl-3-pyrrolidinyl-, 2-Imino-2-(S-oxothiomorpholino)äthyl-, 2-Imino-2-(1,1-dioxo-1,3-thiazolidin-3-yl)äthyl-, 2-Imino-2-(S,S-dioxothiomorpholino)-äthyl-, 2-Imino-2-(3,5-dioxo-1-piperazinyl)äthyl-, 2-Imino-2-(4-methyl-3,5-dioxo-1-piperazinyl)äthyl-, 2-Imino-2-(3-oxo-1-piperazinyl)äthyl-, 2-Imino-2-(4-methyl-3-oxo-1-piperazinyl)äthyl-, 2-Imino-2-(4-acetyl-3-oxo-1-piperazinyl)äthyl-, 2-Imino-2-(4-methansulfonyl-3-oxo1-piperazinyl)äthyl-, $N^1$-(2-Carbamoyloxyäthyl)-$N^1$-methylamidinomethyl-, 2-(3-Hydroximino-1-pyrrolidinyl)-2-iminoäthyl-, 2-Imino-2-(3-methoximino-1-pyrrolidinyl)äthyl-, 2-(4-Hydroximinopiperidino)-2-iminoäthyl-, 2-Imino-2-(4-methoximinopiperidino)äthyl-, 2-(3-Carbamoyloxy-1-pyrrolidinyl)-2-iminoäthyl-, 2-Imino-2-(3-oxo-1-piperazinyl)-äthyl-, 2-(3-Carbamoylpiperidino)-2-iminoäthyl, 2-(3-Carbamoyloxypiperidino)-2-iminoäthyl-, 2-(2-Carbamoyloxy-1-pyrrolidinyl)--2-iminoäthyl-, 2-(2-Carbamoyloxymethyl-1-pyrrolidinyl)-2--iminoäthyl-, 2-(4-Carbamoyloxypiperidino)-2-iminoäthyl-, 2-(4-Formyl-1-piperazinyl)-2-iminoäthyl-, 2-(4-Acetyl-1--piperazinyl)-2-iminoäthyl-, 1-Formyl-3-acetidinyl-, 1-Iminomethyl-3-acetidinyl-; 1-Methyl-4-piperidyl-, 1-Acetimidoyl-4-piperidyl- oder 1-Acetyl-3-pyrrolidinylgruppe darstellt.

17. Mischung nach Anspruch 14, worin das Antibiotikum

(5R,6S)-2-{2-[(Aminomethylen)amino] äthyithio} -6--[1(R)-hydroxyäthyl]-2-carbapenem-3-carbonsäure

(5R,6S)-2-[(3S)-1-Acetimidoylpyrrolidin-3-ylthio]-6--[1(R)-hydroxyäthyl]-2-carbapenem-3-carbonsäure

(5R,6S)-2-[(3R)-1-Acetimidoylpyrrolidin-3-ylthio]-6--[1(R)-hydroxyäthyl]-2-carbapenem-3-carbonsäure

(5R,6S)-2-[(3R)-1-Acetimidoylpyrrolidin-3-ylthio]-6--[1(R)-hydroxyäthyl]-1-(S)-methyl-2-carbapenem-3-carbonsäure

(5R,6S)-2-[(3S)-1-Acetimidoylpyrrolidin-3-ylthio]-6--[1(R)-hydroxyäthyl]-1(R)-methyl-2-carbapenem-3-carbonsäure

(5R,6S)-2-[(3S)-1-Acetimidoylpyrrolidin-3-ylthio]-6--[1(R)-hydroxyäthyl]-1(S)-methyl-2-carbapenem-3-carbonsäure

oder

(5R,6S)-2-[(3S)-1-Acetimidoyl-5(S)-carbamoylpyrrolidin-3--ylthio]-6-[1(R)-hydroxyäthyl]-2-carbapenem-3-carbonsäure

ist.

**18.** Mischung nach irgendeinem der vorhergehenden Ansprüche, worin das Gewichtsverhältnis der N-acylierten Aminosäure zum Antibiotikum 0,1:1 bis 4:1 beträgt.

**19.** Verpacktes pharmazeutisches Präparat, welches
(a) in einem Teil ein Penem- oder Carbapenemantibiotikum und
(b) in einem anderen Teil ein pharmazeutisch annehmbares N-acyliertes Derivat einer Aminosäure mit an eine gesättigte aliphatische Kohlenstoffkette oder an ein Kohlenstoffatom gebundener Aminogruppe und Carboxygruppe oder ein Salz hievon mit der Maßgabe enthält, daß die Aminosäure nicht allein Ornithin, Lysin, Phenylalanin oder Phenylglycin ist.

**20.** Präparat nach Anspruch 19, worin die N-acylierte Aminosäure so wie in irgendeinem der Ansprüche 2 bis 13 definiert ist.

**21.** Präparat nach Anspruch 19 oder Anspruch 20, worin das Antibiotikum so wie in irgendeinem der Ansprüche 14 bis 17 definiert ist.

**22.** Verwendung
(a) eines Penem- oder Carbapenemantibiotikums
zusammen mit
(b) einem pharmazeutisch annehmbaren N-acylierten Derivat einer Aminosäure mit an eine gesättigte Kohlenwasserstoffkette oder an ein Kohlenstoffatom gebundener Aminogruppe und Carboxygruppe oder einem Salz hievon zur Herstellung eines Heilmittels zum Behandeln von bakteriellen Infektionen mit der Maßgabe, daß die Aminosäure nicht allein Ornithin, Lysin, Phenylalanin oder Phenylglycin ist.

**23.** Verwendung nach Anspruch 22, worin die N-acylierte Aminosäure so wie in irgendeinem der Ansprüche 2 bis 13 definiert ist.

**24.** Verwendung nach Anspruch 22 oder Anspruch 23, worin das Antibiotikum so wie in irgendeinem der Ansprüche 14 bis 17 definiert ist.

**Patentansprüche für folgende Vertragsstaaten : AT, ES**

**1.** Verfahren zum Herstellen einer pharmazeutischen Mischung, bei welchem

ein Penem- oder Carbapenemantibiotikum und

ein pharmazeutisch annehmbares N-acyliertes Derivat einer Aminosäure mit an eine gesättigte aliphatische Kohlenstoffkette oder an ein Kohlenstoffatom gebundener Aminogruppe und Carboxylgruppe oder ein Salz dieser Aminosäure mit der Maßgabe vermischt wird,

daß, falls die Mischung nur eine einzige Aminosäure enthält, die Aminosäure nicht Ornithin, Lysin, Phenylalanin oder Phenylglycin ist,
wobei die Menge an Aminosäurederivat ausreicht eine allfällige renale Toxizität des Antibiotikums abzuschwächen und vorzugsweise das Gewichtsverhältnis der N-acylierten Aminosäure zum Antibiotikum 0,1:1 bis 4:1 beträgt.

2. Verfahren nach Anspruch 1, worin die Aminosäure eine Verbindung der allgemeinen Formel II

$H_2N$-X-COOH (II)

ist, worin X eine $C_1$-$C_{10}$-Alkylengruppe oder eine $C_1$-$C_{10}$ --Alkylengruppe mit zumindest einem aus Hydroxygruppen, $C_1$-$C_4$-Alkoxygruppen, $C_6$-$C_{14}$-Aryloxygruppen, substituierten $C_6$-$C_{14}$-Aryloxygruppen, $C_7$-$C_9$-Aralkyloxygruppen, substituierten $C_7$-$C_9$-Aralkyloxygruppen, Mercaptogruppen, $C_1$-$C_4$-Alkylthiogruppen, $C_6$-$C_{14}$-Arylthiogruppen, substituierten $c_6$-$c_{14}$--Arylthiogruppen, $C_7$-$C_9$-Aralkylthiogruppen, substituierten $C_7$-$C_9$-Aralkylthiogruppen, $C_2$-$C_5$-Carboxyalkylthiogruppen, Aminogruppen, Aminogruppen mit einem oder zwei aus

$C_1$-$C_4$-Alkylgruppen, $C_6$-$C_{14}$-Arylgruppen, substituierten $C_6$-$C_{14}$-Arylgruppen, $C_7$-$C_9$-Aralkylgruppen, substituierten $C_7$-$C_9$-Aralkylgruppen und von Carbonsäuren abgeleiteten Acylgruppen ausgewählten Substituenten,

$C_6$-$C_{14}$-Arylgruppen, substituierten $C_6$-$C_{14}$-Arylgruppen, Carboxygruppen, Amidinogruppen, Sulfogruppen, $C_1$-$C_4$-Alkylsulfinylgruppen, $C_1$-$C_4$-Alkylsulfonylgruppen und heterocyclischen Gruppen mit 5 bis 14 Ringatomen, von welchen 1 bis 5 von Stickstoff und/oder Sauerstoff und/oder Schwefel gebildete Heteroatome sind, ausgewählten Substituenten, wobei die substituierten Aryloxy-, Aralkyloxy-, Arylthio-, Aralkylthio-, Aryl- und Aralkylgruppen zumindest einen aus $C_1$-$C_4$--Alkylgruppen, Hydroxygruppen, Aminogruppen und $C_1$-$C_4$-Alkoxygruppen ausgewählten Substituenten aufweisen,

oder ein pharmazeutisch annehmbares Salz hievon ist.

3. Verfahren nach Anspruch 2, worin X eine $C_1$ -$C_5$-Alkylengruppe darstellt, welche unsubstituiert ist oder einen oder zwei Substituenten aufweist, die aus Hydroxygruppen, $C_1$-$C_4$--Alkoxygruppen, Aryloxygruppen mit 6 bis 14 Ringkohlenstoffatomen im unsubstituierten oder 1 bis 3 aus $C_1$-$C_4$-Alkylgruppen, Hydroxygruppen, Aminogruppen und $C_1$-$C_4$-Alkoxygruppen ausgewählte Substituenten besitzenden Arylring, $C_7$-$C_9$-Aralkyloxygruppen mit unsubstituiertem oder 1 bis 3 aus $C_1$-$C_4$-Alkylgruppen Hydroxygruppen, Aminogruppen und $C_1$-$C_4$-Alkoxygruppen ausgewählte Substituenten besitzendem Arylrest, Mercaptogruppen, $C_1$-$C_4$-Alkylthiogruppen, Arylthiogruppen mit 6 bis 14 Ringkohlenstoffatomen im unsubstituierten oder 1 bis 3 aus $C_1$-$C_4$-Alkylgruppen, Hydroxygruppen, Aminogruppen und $C_1$-$C_4$-Alkoxygruppen ausgewählte Substituenten besitzenden Arylring, $C_7$-$C_9$-Aralkylthiogruppen mit unsubstituiertem oder 1 bis 3 aus $C_1$-$C_4$--Alkylgruppen, Hydroxygruppen, Aminogruppen und $C_1$-$C_4$--Alkoxygruppen ausgewählte Substituenten aufweisenden Arylring, Carboxyalkylthiogruppen mit 1 bis 4 Kohlenstoffatomen im Alkylteil, Aminogruppen, Aminogruppen mit einem oder mit Zwei $C_1$-$C_4$-Alkylsubstituenten, Aminogruppen mit einem oder zwei Arylsubstituenten, worin der Arylring 6 bis 14 Ringkohlenstoffatome besitzt und unsubstituiert ist oder 1 bis 3 aus $C_1$-$C_4$-Alkylgruppen, Hydroxygruppen, Aminogruppen und $C_1$-$C_4$-Alkoxygruppen ausgewählte Substituenten aufweist, Aminogruppen mit einem oder zwei $C_7$-$C_9$-Aralkylsubstituenten mit unsubstituiertem oder 1 bis 3 aus $C_1$-$C_4$--Alkylgruppen, Hydroxygruppen, Aminogruppen und $C_1$-$C_4$-Alkoxygruppen ausgewählten Substituenten im Arylteil, Aminogruppen mit einem oder zwei von Carbonsäuren abgeleiteten Acylgruppen als Substituenten, Arylgruppen mit 6 bis 14 Ringkohlenstoffatomen, welche unsubstituiert sind oder 1 bis 3 aus $C_1$-$C_4$--Alkylgruppen, Hydroxygruppen, Aminogruppen und $C_1$-$C_4$-Alkoxygruppen ausgewählte Substituenten aufweisen, Carboxygruppen und heterocyclische Gruppen mit 5 bis 9 Ringatomen, von welchen 1 bis 3 von Stickstoff und/oder Sauerstoff und/oder Schwefel gebildete Heteroatome sind, ausgewählt sind.

4. Verfahren nach Anspruch 2, worin X eine $C_1$-$C_5$-Alkylengruppe darstellt, die unsubstituiert ist oder 1 oder 2 Substituenten aufweist, die aus Hydroxygruppen, $C_1$-$C_4$-Alkoxygruppen, Mercaptogruppen, $C_1$-$C_4$-Alkylthiogruppen, Aminogruppen, Aminogruppen mit einem oder zwei $C_1$-$C_4$-Alkylsubstituenten, Aminogruppen mit einem oder zwei von Carbonsäuren abgeleiteten Acylsubstituenten, Arylgruppen mit 6 bis 14 Kohlenstoffatomen und unsubstituiertem oder 1 bis 3 aus $C_1$-$C_4$-Alkylgruppen, Hydroxygruppen, Aminogruppen und $C_1$-$C_4$--Alkoxygruppen ausgewählte Substituenten aufweisendem Arylring, Carboxygruppen und heterocyclischen Gruppen mit 5 bis 9 Ringatomen, von welchen 1 bis 3 von Stickstoff und/ /oder Sauerstoff gebildete Heteroatome sind, ausgewählt sind.

5. Verfahren nach irgendeinem der Ansprüche 1 bis 4, worin die N-Acylgruppe eine $C_1$-$C_{18}$-Alkanoylgruppe, eine $C_3$-$C_8$-Alkenoylgruppe, eine $C_3$-$C_8$-Alkinoylgruppe, eine aromatische Acylgruppe mit carbocyclischem $C_6$-$C_{14}$-Arylteil, welcher unsubstituiert ist oder 1 bis 5 aus $C_1$-$C_4$-Alkylgruppen, Hydroxygruppen, $C_1$-$C_4$-Alkoxygruppen, Aminogruppen, Sulfogruppen und Halogenatomen ausgewählte Substituenten aufweist, eine Cycloalkancarbonylgruppe mit unsubstituiertem oder zumindest einen aus $C_1$-$C_4$-Alkylgruppen und Phenylgruppen ausgewählte Substituenten aufweisendem $C_3$-$C_8$-Cycloalkanteil, eine araliphatische Acylgruppe mit einem 6 bis 14 Kohlenstoffatome aufweisenden carbocyclischen Ring als Arylring, welcher unsubstituiert ist oder 1 bis 5 aus $C_1$-$C_4$-Alkylgruppen, Hydroxygruppen, $C_1$-$C_4$-Alkoxygruppen, Aminogruppen, Sulfogruppen und Halogenatomen ausgewählte Substituenten aufweist, und mit 1 bis 4 Kohlenstoffatomen im Alkylteil, eine heterocyclische Acylgruppe mit gesättigtem oder ungesättigtem Ringsystem und mit 5 oder 6 Ringatomen, von welchen 1 bis 3 von Stickstoff und/oder Schwefel und/oder Sauerstoff gebildete Heteroatome sind, wobei der Ring unsubstituiert ist oder 1 bis 3 aus $C_1$-$C_4$-Alkylgruppen und Hydroxygruppen ausgewählte Substituenten aufweist, eine $C_2$-$C_7$-Alkoxycarbonylgruppe, eine Aralkyloxycarbonylgruppe mit 7 bis 9 Kohlenstoffatome aufweisendem Aralkylteil, welcher unsubstituiert ist oder 1 bis 5 aus Aminogruppen, $C_1$-$C_4$-Alkylgruppen, $C_1$-$C_4$-Alkoxygruppen und Hydroxygruppen ausgewählte Substituenten aufweist oder eine Acylgruppe ist, die von einer Aminosäure durch Abspalten der OH-Gruppe von der Carboxylgruppe und N-Acylieren der Aminogruppe mit zumindest einer der oben genannten Acylgruppen abgeleitet worden ist.

6. Verfahren nach irgendeinem der Ansprüche 1 bis 4, worin die N-Acylgruppe eine gesättigte aliphatische Acylgruppe mit 1 bis 8 Kohlenstoffatomen, eine aromatische Acylgruppe mit 6 bis 10 Ringkohlenstoffatomen im unsubstituierten oder 1 bis 3 aus $C_1$-$C_4$-Alkylgruppen und $C_1$-$C_4$-Alkoxygruppen ausgewählte Substituenten aufweisenden Arylrest, eine alicyclische Acylgruppe mit 3 bis 6 Kohlenstoffatomen im Cycloalkanring, eine araliphatische Acylgruppe mit 6 bis 10 Ringkohlenstoffatomen im Arylring und mit 1 bis 4 Kohlenstoffatomen in der Alkylgruppe und mit unsubstituiertem oder 1 bis 3 aus $C_1$-$C_4$-Alkylgruppen und $C_1$-$C_4$-Alkoxygruppen ausgewählte Substituenten aufweisendem Arylring, eine heterocyclische Acylgruppe mit gesättigtem oder ungesättigtem und 5 oder 6 Ringatome, von welchen eines ein von Stickstoff, Schwefel oder Sauerstoff gebildetes Heteroatom ist, aufweisendem heterocyclischem Ring, eine Alkoxycarbonylgruppe mit insgesamt 2 bis 7 Kohlenstoffatomen, eine Aralkyloxycarbonylgruppe mit einem 7 bis 9 Kohlenstoffatome aufweisenden Aralkylrest und mit unsubstituiertem oder 1 bis 3 aus $C_1$-$C_4$-Alkylgruppen und $C_1$-$C_4$-Alkoxygruppen ausgewählte Substituenten aufweisendem Arylring oder eine Acylgruppe ist, die von einer Aminosäure durch Abspalten der OH-Gruppe von der Carboxylgruppe und durch N-Acylieren der Aminogruppe mit zumindest einer der oben erwähnten Acylgruppen abgeleitet worden ist.

7. Verfahren nach irgendeinem der Ansprüche 1 bis 4, worin die N-Acylgruppe eine aromatische Acylgruppe mit einem 6 bis 10 Ringatome besitzenden und unsubstituierten oder einen einzigen aus $C_1$-$C_4$-Alkylgruppen, $C_1$-$C_4$-Alkoxygruppen, Hydroxygruppen und Aminogruppen ausgewählten Substituenten aufweisenden Arylring, eine alicyclische Acylgruppe mit einem 3 bis 6 Kohlenstoffatome aufweisenden Cycloalkanrest, eine phenylaliphatische Acylgruppe mit unsubstituierter oder einen einzigen $C_1$-$C_4$-Alkylsubstituenten aufweisender Phenylgruppe und mit einem 1 bis 4 Kohlenstoffatome aufweisenden Alkylteil, eine Alkoxycarbonylgruppe mit insgesamt 4 bis 6 Kohlenstoffatomen, eine Aralkyloxycarbonylgruppe mit einem 7 bis 9 Kohlenstoffatome aufweisenden und 0 oder 1 aus $C_1$-$C_4$-Alkylgruppen und $C_1$-$C_4$-Alkoxygruppen ausgewählte Substituenten aufweisenden Aralkylteil oder eine Acylgruppe ist, die von einer Aminosäure durch Abspalten der OH-Gruppe aus der Carboxylgruppe und N-Acylieren der Aminogruppe mit zumindest einer der oben erwähnten Acylgruppen erhalten worden ist.

8. Verfahren nach irgendeinem der Ansprüche 1 bis 4, worin die N-Acylgruppe eine Acetyl-, Benzoyl-,

Cyclohexancarbonyl-, Cyclopropancarbonyl-, Hexanoyl-, Isobutyryl-, Crotonoyl-, Äthoxycarbonyl-, 4-Hydroxybenzoyl-, Anisoyl-, 4-Aminobenzoyl-, Naphthoyl-, Toluoyl-, Benzyloxycarbonyl-oder 4-Methoxybenzyloxycarbonylgruppe ist.

**9.** Verfahren nach irgendeinem der Ansprüche 1 und 5 bis 8, worin die Aminosäure Glycin, ß-Alanin, 4-Aminobuttersäure, 5-Aminovaleriansäure, 6-Aminohexansäure, 8-Aminooctansäure, Alanin, 2-Aminobuttersäure, Norvalin, Valin, Leucin, Isoleucin, Norleucin, Tyrosin, O-Methyltyrosin, Asparaginsäure, Glutaminsäure, 4-Carboxyglutaminsäure, 3-Methylasparaginsäure, 2-Aminoadipinsäure, 2-Aminopimelinsäure, 2-Aminosuberinsäure, 3-Hydroxyasparaginsäure, 3-Hydroxyglutaminsäure, 2,3-Diaminopropionsäure, 2,4-Diaminobuttersäure, 5-Hydroxylysin, Arginin, $N^{\delta}$,$N^{\epsilon}$ -Dimethylornithin, $N^{\epsilon}$-Methyllysin, Cystein, Methionin, Äthionin, S-Carboxymethylcystein, S-Benzylcystein, Methionin-S-oxid, Äthionin-S-oxid, Methionin-S,S-dioxid, Cysteinsäure, Serin, O-Methylserin, Threonin, O-Methylthreonin, Homothreonin, Äthoxinin, 3-Methoxyvalin, 3-Phenylserin, 3-Methyl-3-phenylalanin, Histidin, Tryptophan, 2-Methylalanin, 2-Methylserin, 2-Hydroxyisoleucin, 2-Methylmethionin, 2-Äthyl-2-phenylglycin, 3-Aminobuttersäure, 3-Amino-4-methylvaleriansäure, 3-Amino-3-phenylpropionsäure, 3-Amino-2-hydroxypropionsäure oder 4-Amino-3-hydroxybuttersäure ist.

**10.** Verfahren nach irgendeinem der Ansprüche 1 und 5 bis 8, worin die Aminosäure Glycin, ß-Alanin, 4-Aminobuttersäure, 5-Aminovaleriansäure, 6-Aminohexansäure, 8-Aminooctansäure, Alanin, Norvalin, Valin, Leucin, Isoleucin, Norleucin, $N^{\delta}$,$N^{\delta}$-dimethylornithin, Methionin, Äthionin, O-Methylserin, O-Methylthreonin, Äthoxinin, 3-Methoxyvalin, 3-Phenylserin, 3-Methyl-3-phenylalanin, Histidin, 2-Methylalanin, 2-Methylserin, 2-Hydroxyisoleucin, 2-Äthylphenylglycin, 3-Aminobuttersäure, 3-Amino-4-methylvaleriansäure, oder 3-Amino-3-phenylpropionsäure ist.

**11.** Verfahren nach irgendeinem der Ansprüche 1 und 5 bis 8, worin die Aminosäure ß-Alanin, 4-Aminobuttersäure, 5-Aminovaleriansäure, 6-Aminohexansäure, Alanin, Valin, Leucin, Norleucin, Methionin, Histidin oder Glycin ist.

**12.** Verfahren nach irgendeinem der Ansprüche 1 und 5 bis 8, worin die Amininosäure Leucylglycin, Glycyl-ß-alanin, Glycylalanin, Valylalanin, Leucylalanin, Glycylvalin, Alanylvalin, Leucylvalin, Valylleucin, Phenylalanylleucin, Histidylleucin, Glycylphenylalanin, Alanylphenylalanin, Leucylphenylalanin, Glycylmethionin, Valylmethionin, Glycylhistidin, Alanylvalylglycin, Glycylalanylvalin, Glycylphenylalanylleucin oder Glycylglycylhistidin ist.

**13.** Verfahren nach Anspruch 1, worin die N-acylierte Aminosäure
N-(p-Toluoyl)-ß-alanin
N-(4-Methoxybenzoyl)-ß-alanin
N-(3-Hydroxy-2-naphthoyl)-ß-alanin
N-Benzoylglycyl -ß-alanin
N-Benzoyl-ß-alanin
N-Benzoyl-5-aminovaleriansäure
N-Benzoyl-6-aminohexansäure
N-Cyclohexancarbonyl-6-aminohexansäure
N-(N-Methylnicotinoyl)-6-aminohexansäure
N-Benzoyl-8-aminooctansäure
N-Benzoylalanin
N-(1-Naphthoyl)alanin
N-Benzoylvalylalanin
N-Benzoyl-2-aminobuttersäure
N-Benzoylnorvalin
N-Valerylvalin
N-Benzoylalanylvalin
N-Benzoylvalin
N-Benzoylleucin
N-Benzoylglycylphenylalanylleucin
N-Benzoylnorleucin
N-Benzoylglycylphenylalanin
N-Benzoylalanylphenylalanin

N-Cyclohexancarbonylleucylphenylalanin
N-Benzoyl -O-methyltyrosin
N-Benzoylmethionin
N-Phenylacetylmethionin
N-Benzoylvalylmethionin
N-Benzoyläthionin
N-(4-Methoxybenzyloxycarbonyl)äthionin
N-Benzoylthreonin
N-Benzoylhistidin
N-(p-Toluoyl)histidin
N-(4-Methoxybenzoyl)histidin
N-(4-Methoxybenzoyl)-3-aminobuttersäure
oder
N-Butyryl-3-amino-3-phenylpropionsäure
ist.

**14.** Verfahren nach irgendeinem der vorhergehenden Ansprüche, worin das Antibiotikum eine Verbindung der allgemeinen Formel I

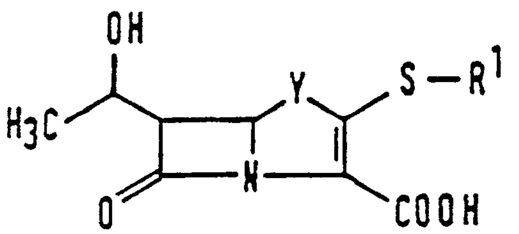

(I)

ist, in welcher

Y ein Schwefelatom, eine Methylengruppe oder eine Methylengruppe mit 1 oder 2 Methylgruppen und/oder Methoxygruppen als Substituenten bedeutet und

$R^1$ eine $C_1$-$C_6$-Alkylgruppe, eine $C_1$-$C_6$-Alkylgruppe mit zumindest einem der Substituenten (i) oder eine heterocyclische Gruppe mit 4 bis 14 Ringatomen, von welchen 1 bis 5 von Stickstoff und/oder Sauerstoff und/oder Schwefel gebildete Heteroatome sind, darstellt, wobei die heterocyclische Gruppe unsubstituiert ist oder zumindest einen der Substituenten (ii) aufweist,

wobei folgendes gilt:

Substituenten (i):
Halogenatome, Aminogruppen, Aminogruppen mit zumindest einem der Substituenten (iii), $C_1$-$C_4$-Alkylidenaminogruppen, $C_1$-$C_4$-Aminoalkylidenaminogruppen, Amidinogruppen, Amidinogruppen mit 1 bis 3 der Substituenten (iii), heterocyclische Gruppen mit 4 bis 14 Ringatomen, von welchen 1 bis 5 von Stickstoff und/oder Sauerstoff und/oder Schwefel gebildete Heteroatome sind, und hiebei die heterocyclische Gruppe unsubstituiert ist oder zumindest einen der Substituenten (ii) aufweist, Iminogruppen, Cyanogruppen, Carbamoylgruppen und Carbamoylgruppen mit zumindest einem aus $C_1$-$C_4$-Alkylgruppen und $C_1$-$C_4$-Alkoxygruppen ausgewählten Substituenten
Substituenten (ii):
$C_1$-$C_6$-Alkanimidoylgruppen, $C_1$-$C_6$-Alkylgruppen, Alkoxyalkylgruppen mit $C_1$-$C_4$-Alkoxyteil und $C_1$-$C_4$-Alkylteil, Carbamoylgruppen, Carbamoylgruppen mit zumindest einem aus $C_1$-$C_4$-Alkylgruppen und $C_1$-$C_4$-Alkoxygruppen ausgewählten Substituenten, $C_1$-$C_4$-Halogenalkylgruppen, Heterocyclylacylimidoylgruppen mit 5 bis 9 Ringatomen, von welchen 1 bis 3 von Stickstoff und/oder Sauerstoff und/oder Schwefel gebildete Heteroatome sind, Amidinogruppen, Amidinogruppen mit 1 bis 3 der Substituenten (iii), Iminogruppen, Sauerstoffatome, $C_1$-$C_6$-Alkanoylgruppen, $C_1$-$C_6$-Alkansulfonylgruppen, $C_1$-$C_6$-Alkansulfinylgruppen, Hydroximinogruppen, $C_1$-$C_4$-Alkoximinogruppen, Carbamoyloxygruppen, Carbamoyloxygruppen mit zumindest einem aus $C_1$-$C_4$-Alkylgruppen und $C_1$-$C_4$-Alkoxy-

gruppen ausgewählten Substituenten, Carbamoyloxyalkylgruppen mit einer $C_1$-$C_4$-Alkylgruppe als Alkylteil und unsubstituiertem oder durch zumindest einen aus $C_1$-$C_4$-Alkylgruppen und $C_1$-$C_4$-Alkoxygruppen ausgewählten Substituenten substituiertem Carbamoylteil und $C_1$-$C_4$-Iminoalkylgruppen,

Substituenten (iii):

$C_1$-$C_6$-Alkylgruppen, $C_2$-$C_6$-Alkenylgruppen, $C_2$-$C_6$-Alkinylgruppen und diese Alkyl-, Alkenyl- und Alkinylgruppen mit zumindest einem Substituenten, welcher aus Halogenatomen, Carbamoyloxygruppen und Carbamoyloxygruppen mit zumindest einem aus $C_1$-$C_4$-Alkylgruppen und $C_1$-$C_4$-Alkoxygruppen ausgewählten Substituenten ausgewählt ist,

und ein pharmazeutisch annehmbares Salz hievon ist.

**15.** Verfahren nach Anspruch 14, worin Y ein Schwefelatom, eine Methylengruppe oder die Gruppe $CH_3$-CH<, $CH_3O$-CH< oder $(CH_3)_2$C< bedeutet.

**16.** Verfahren nach Anspruch 14 oder Anspruch 15, worin $R^1$ eine Äthyl-, 2-Fluoräthyl-, 2-(Aminomethylenamino)äthyl-,$N^1$,$N^1$-Dimethylamidinomethyl-, $N^1$,$N^1$,$N^2$-Trimethylamidinomethyl, 3-Pyrrolidinyl-, 1-Formimidoyl-3-pyrrolidinyl-, 1-Acetimidoyl-3-pyrrolidinyl-, 1-Propionimidoyl-3-pyrrolidinyl-, 2-Methyl-1,4,5,6,-tetrahydro-5-pyrimidinyl-, 2-Methoxymethyl-1,4,5,6-tetrahydro-5-pyrimidinyl-, 3-Acetidinyl-, 1-Acetimidoyl-3-acetidinyl-, $N^1$-Methyl-$N^1$-(2-propinyl)amidinomethyl-, $N^1$-(2-Fluoräthyl)-$N^1$-methylamidinomethyl-, $N^1$-(3-Fluorpropyl)-$N^1$-methylamidinomethyl-, $N^1$-Methyl-$N^1$-(2,2,2-trifluoräthyl)amidinomethyl-, 1-(3-Acetidinyl)äthyl-, 1-(1-Acetimidoyl-3-acetidinyl)äthyl-, 1,4,5,6-Tetrahydro-2-pyrimidinylmethyl-, 1-(4,5-Dihydro-2-thiazolyl)äthyl-, 5-Carbamoyl-3-pyrrolidinyl-, 1-Acetimidoyl-5-carbamoyl-3-pyrrolidinyl-, 2-Chlormethyl-1,4,5,6-tetrahydro-5-pyrimidinyl-, 1-Butyrimidoyl-3-pyrrolidinyl-, 1-Nicotinimidoyl-3-pyrrolidinyl-, $N^1$,$N^1$-Diallylamidinomethyl-, $N^1$-Methyl-$N^1$-(2-propinyl)-amidino-, $N^1$-(2-Fluoräthyl)-$N^1$-methylamidino-, $N^1$-(3-Fluorpropyl)-$N^1$-methylamidino-, $N^1$-Methyl-$N^1$-(2,2,2-trifluoräthyl)amidino-, $N^1$-Allyl-$N^1$-methylamidinomethyl-, Cyanomethyl-, 2-Cyanoäthyl-, 1-Cyanoäthyl-, 2-Cyano-1-methyläthyl-, 2-Aminoäthyl-, 1-Carbamoyläthyl-, 2-(1-Aminoäthylidenamino)-äthyl-, 1-Amidino-3-pyrrolidinyl-, 2-Methyl-1,3-diazabicyclo[3.3.0]oct-2-en-7-yl-, 2-Methoxymethyl-1,3-diazabicyclo[3.3.0]oct--2-en-7-yl-, 5-Imino-2-pyrrolidinyl, 2-Imino-5-piperidinyl-, 1-Acetimidoyl-5-methylcarbamoyl-3-pyrrolidinyl-, 1-Acetimidoyl-5-methoxycarbamoyl-3-pyrrolidinyl-, 2-Imino-2-(S-oxothiomorpholino)äthyl-, 2-Imino-2-(1,1-dioxo-1,3-thiazolidin-3-yl)äthyl-, 2-Imino-2-(S,S-dioxothiomorpholino)äthyl-, 2-Imino-2-(3,5-dioxo-1-piperazinyl)äthyl-, 2-Imino-2-(4-methyl-3,5-dioxo-1-piperazinyl)äthyl-, 2-Imino-2-(3-oxo-1-Piperazinyl)äthyl-, 2-Imino-2-(4-methyl-3-oxo-1-piperazinyl)äthyl-, 2-Imino-2-(4-acetyl-3-oxo-1-piperazinyl)äthyl-, 2-Imino-2-(4-methansulfonyl-3-oxo-1-piperazinyl)äthyl-, $N^1$-(2-Carbamoyloxyäthyl)-$N^1$-methylamidinomethyl-, 2-(3-Hydroximino-1-pyrrolidinyl)-2-iminoäthyl-, 2-Imino-2-(3-methoximino-1-pyrrolidinyl)äthyl-, 2-(4-Hydroximinopiperidino)-2-iminoäthyl-, 2-Imino-2-(4-methoximinopiperidino)äthyl-, 2-(3-Carbamoyloxy-1-pyrrolidinyl)-2-iminoäthyl-, 2-Imino-2-(3-oxo-1-piperazinyl)-äthyl-, 2-(3-Carbamoylpiperidino)-2-iminoäthyl-, 2-(3-Carbamoyloxypiperidino)-2-iminoäthyl-, 2-(2-Carbamoyloxy-1-pyrrolidinyl)-2-iminoäthyl-, 2-(2-Carbamoyloxymethyl-1-pyrrolidinyl)-2-iminoäthyl-, 2-(4-Carbamoyloxypiperidino)-2-iminoäthyl-, 2-(4-Formyl-1-piperazinyl)-2-iminoäthyl-, 2-(4-Acetyl-1-piperazinyl)-2-iminoäthyl-, 1-Formyl-3-acetidinyl-, 1-Iminomethyl-3-acetidinyl-, 1-Methyl-4-piperidyl-, 1-Acetimidoyl-4-piperidyl- oder 1-Acetyl-3-pyrrolidinylgruppe darstellt.

**17.** Verfahren nach Anspruch 14, worin das Antibiotikum

(5R,6S)-2-{2-[(Aminomethylen)amino]äthylthio}-6-[1(R)-hydroxyäthyl]-2-carbapenem-3-carbonsäure

(5R,6S)-2-[(3S)-1-Acetimidoylpyrrolidin-3-ylthio]-6-[1(R)-hydroxyäthyl]-2-carbapenem-3-carbonsäure

(5R,6S)-2-[(3R)-1-Acetimidoylpyrrolidin-3-ylthio]-6-[1R)-hydroxyäthyl]-2-carbapenem-3-carbonsäure

(5R,6S)-2-[(3R)-1-Acetimidoylpyrrolidin-3-ylthio]-6-[1(R)-hydroxyäthyl]-1-(S)-methyl-2-carbapenem-3-carbonsäure

(5R,6S)-2-[(3S)-1-Acetimidoylpyrrolidin-3-ylthio]-6-[1(R)-hydroxyäthyl]-1(R)-methyl-2-carbapenem-3-carbonsäure

(5R,6S)-2-[(3S)-1-Acetimidoylpyrrolidin-3-ylthio]-6-[1(R)-hydroxyäthyl]-1(S)-methyl-2-carbapenem-3-carbonsäure

oder

(5R,6S)-2-[(3S)-1-Acetimidoyl-5(S)-carbamoylpyrrolidin-3-ylthio]-6-[1(R)-hydroxyäthyl]-2-carbapenem-3-carbonsäure

ist.

**18.** Verfahren nach irgendeinem der vorhergehenden Ansprüche, worin das Gewichtsverhältnis der N-acylierten Aminosäure zum Antibiotikum 0,1:1 bis 4:1 beträgt.

**19.** Verfahren nach irgendeinem der vorhergehenden Ansprüche, bei welchem die N-acylierte Aminosäure in Wasser solubilisiert wird, der erhaltenen Lösung das Antibiotikum zugesetzt und in der Lösung gelöst wird und gegebenenfalls die Lösung gefriergetrocknet wird, um ein pulveriges Gemisch zu erhalten.

**20.** Verfahren nach Anspruch 19, bei welchem die N-acylierte Aminosäure dadurch solubilisiert wird, daß sie in Wasser dispergiert und eine Base in einer Menge Zugesetzt wird, welche ausreicht den pH-Wert auf einen Wert von 5,5 bis 9 einzustellen.

**21.** Verwendung
(a) eines Penem- oder Carbapenemantibiotikum
zusammen mit
(b) einem pharmazeutisch annehmbaren N-acylierten Derivat einer Aminosäure mit an eine gesättigte Kohlenwasserstoffkette oder an ein Kohlenstoffatom gebundener Aminogruppe und Carboxygruppe oder einem Salz hievon zur Herstellung eines Heilmittels zum Behandeln von bakteriellen Infektionen mit der Maßgabe, daß die Aminosäure nicht allein Ornithin, Lysin, Phenylalanin oder Phenylglycin ist.

**22.** Verwendung nach Anspruch 21, worin die N-acylierte Aminosäure
N-(p-Toluoyl)-ß-alanin
N-(4-Methoxybenzoyl)-ß-alanin
N-(3-Hydroxy-2-naphthoyl)-ß-alanin
N-Benzoylglycyl-ß-alanin
N-Benzoyl-ß-alanin
N-Benzoyl-5-aminovaleriansäure
N-Benzoyl-6-aminohexansäure
N-Cyclohexancarbonyl-6-aminohexansäure
N-(N-Methylnicotinoyl)-6-aminohexansäure
N-Benzoyl-8-aminoctansäure
N-Benzoylalanin
N-(1-Naphthoyl)alanin
N-Benzoylvalylalanin
N-Benzoyl-2-aminobuttersäure
N-Benzoylnorvalin

N-Valerylvalin
N-Benzoylalanylvalin
N-Benzoylvalin
N-Benzoylleucin
N-Benzoylglycylphenylalanylleucin
N-Benzoylnorleucin
N-Benzoylglycylphenylalanin
N-Benzoylalanylphenylalanin
N-Cyclohexancarbonylleucylphenylalanin
N-Benzoyl-O-methyltyrosin

N-Benzoylmethionin
N-Phenylacetylmethionin
N-Benzoylvalylmethionin
N-Benzoyläthionin
N-(4-Methoxybenzyloxycarbonyl)äthionin
N-Benzoylthreonin
N-Benzylhistidin
N-(p-Toluoyl)histidin
N-(4-Methoxybenzoyl)histidin
N-(4-Methoxybenzoyl)-3-aminobuttersäure
oder
N-Butyryl-3-amino-3-phenylpropionsäure
ist.

**23.** Verwendung nach Anspruch 21 oder Anspruch 22, worin das Antibiotikum

(5R,6S)-2-{2-[(Aminomethylen)amino]äthylthio}-6-[1(R)-hydroxyäthyl]-2-carbapenem-3-carbonsäure,

(5R,6S)-2-[(3S)-1-Acetimidoylpyrrolidin-3-ylthio]-6-[1(R)-hydroxyäthyl]-2-carbapenem-3-carbonsäure,

(5R,6S)-2-[(3R)-1-Acetimidoylpyrrolidin-3-ylthio]-6-[1(R)-hydroxyäthyl]-2-carbapenem-3-carbonsäure,

(5R,6S)-2-[(3R)-1-Acetimidoylpyrrolidin-3-ylthio]-6-[1(R)-hydroxyäthyl]-1-(S)-methyl-2-carbapenem-3-carbonsäure,

(5R,6S)-2-[3S)-1-Acetimidoylpyrrolidin-3-ylthio]-6-[1(R)-hydroxyäthyl]-1(R)-methyl-2-carbapenem-3-carbonsäure,

(5R,6S)-2-[(3S)-1-Acetimidoylpyrrolidin-3-ylthio]-6-[1(R)-hydroxyäthyl]-1(S)-methyl-2-carbapenem-3-carbonsäure

oder

(5R,6S)-2-[(3S)-1-Acetimidoyl-5(S)-carbamoylpyrrolidin-3-ylthio]-6-[1(R)-hydroxyäthyl]-2-carbapenem-3-carbonsäure

ist.